# EUROPÄISCHE PATENTSCHRIFT

(11) **EP 2 616 428 B1**
(45) Veröffentlichungstag und Bekanntmachung des Hinweises auf die Patenterteilung: **24.12.2014**
(21) Anmeldenummer: 11757542.3
(22) Anmeldetag: 16.08.2011
(51) Int. Cl.: C07C 209/10, C07C 211/54, C07C 211/61, H01L 51/00

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 15.09.2010 DE 102010045405
(43) Veröffentlichungstag der Anmeldung: 24.07.2013
(73) Patentinhaber: Merck Patent GmbH, 64293 Darmstadt (DE)
(72) Erfinder: PFLUMM, Christof, 60316 Frankfurt am Main (DE); PARHAM, Amir, Hossain, 65929 Frankfurt am Main (DE); BROCKE, Constanze, 64521 Gross-Gerau (DE); MONTENEGRO, Elvira, 69469 Weinheim (DE); VOGES, Frank, 67098 Bad Duerkheim (DE); HEIL, Holger, 60389 Frankfurt am Main (DE); BUESING, Arne, 65929 Frankfurt am Main (DE)
(86) Internationale Anmeldenummer: PCT/EP2011/004106
(87) Internationale Veröffentlichungsnummer: WO 2012/034627

(56) Entgegenhaltungen:
- WO-A1-03/037844
- WO-A1-2007/043354
- WO-A1-2010/015306
- US-A1- 2008 038 587
- US-A1- 2009 167 161

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen enthaltend diese Materialien.

Der Aufbau organischer Elektrolumineszenzvorrichtungen (OLEDs), in denen organische Halbleiter als funktionelle Materialien eingesetzt werden, ist beispielsweise in US 4,539,507, US 5,151,629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6).

Gemäß dem Stand der Technik werden als Lochtransportmaterialien in der Lochtransportschicht bzw. in der Lochinjektionsschicht insbesondere Triarylaminderivate verwendet, welche entweder mindestens zwei Triarylaminogruppen oder mindestens eine Triarylaminogruppe und mindestens eine Carbazolgruppe aufweisen. Diese Verbindungen leiten sich häufig von Diarylamino-substituierten Triphenylaminen (TPA-Typ), von Diarylamino-substituierten Biphenyl-Derivaten (TAD-Typ) oder Kombinationen dieser Grundverbindungen ab. Weiterhin werden beispielsweise Spirobifluorenderivate eingesetzt, welche mit zwei oder vier Diarylaminogruppen substituiert sind (z. B. gemäß EP 676461 oder US 7,714,145). Bei diesen Verbindungen gibt es sowohl bei fluoreszierenden wie auch bei phosphoreszierenden OLEDs weiterhin Verbesserungsbedarf, insbesondere hinsichtlich Effizienz, Lebensdauer und Betriebsspannung bei Verwendung in einer organischen Elektrolumineszenzvorrichtung sowie hinsichtlich der thermischen Stabilität bei der Sublimation.

WO 2007/043354 offenbart Spirobifluorenderivate und deren Verwendung in organischen Elektrolumineszenzvorrichtungen.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Lochtransportmaterial in einer Lochtransportbzw. Exzitonenblockierschicht oder als Matrixmaterial in einer emittierenden Schicht.

Überraschend wurde gefunden, dass bestimmte, unten näher beschriebene Verbindungen diese Aufgabe lösen und zu deutlichen Verbesserungen der organischen Elektrolumineszenzvorrichtung führen, insbesondere hinsichtlich der Lebensdauer, der Effizienz und der Betriebsspannung. Dies gilt für phosphoreszierende und fluoreszierende Elektrolumineszenzvorrichtungen, vor allem bei Einsatz der erfindungsgemäßen Verbindungen als Lochtransportmaterial oder als Matrixmaterial. Die Materialien weisen im Allgemeinen eine hohe thermische Stabilität auf und lassen sich daher unzersetzt und rückstandsfrei sublimieren. Diese Materialien sowie elektronische Vorrichtungen, welche derartige Verbindungen enthalten, sind daher der Gegenstand der vorliegenden Erfindung. Insbesondere ist es ein überraschendes Ergebnis, dass mit einem aromatischen Monoamin sehr gute Ergebnisse erhalten werden, da in organischen Elektrolumineszenzvorrichtungen im Allgemeinen Lochtransportmaterialien mit mindestens zwei Stickstoffatomen eingesetzt werden.

Gegenstand der vorliegenden Erfindung ist daher eine Verbindung gemäß der folgenden Formel (1), 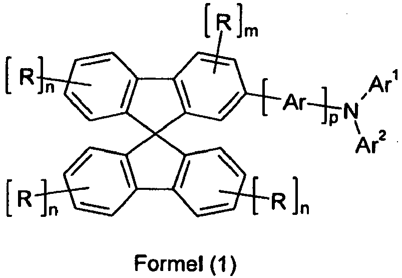 wobei für die verwendeten Symbole und Indizes gilt:
- Ar: ist gleich oder verschieden bei jedem Auftreten ein aromatisches Ringsystem, ausgewählt aus der Gruppe bestehend aus Benzol, Naphthalin, Phenanthren, Fluoren, Spirobifluoren, Dibenzofuran und Dibenzothiophen, welches jeweils mit einem oder mehreren Resten R¹ substituiert sein kann; dabei kann auch Ar mit Ar¹ und/oder mit Ar² durch eine Gruppe E verbunden sein;
- Ar¹, Ar²: ist gleich oder verschieden bei jedem Auftreten ein aromatisches oder heteroaromatisches Ringsystem mit 6 bis 60 C-Atomen, ausgewählt aus der Gruppe bestehend aus Benzol, Naphthalin, Phenanthren, Fluoren, Dibenzofuran, Dibenzothiophen, das jeweils auch durch einen oder mehrere Reste R¹ substituiert sein kann, oder unsubstituiertem Spirobifluoren oder einer Kombination aus zwei, drei, vier oder fünf dieser Gruppen, die jeweils gleich oder verschieden sein können; dabei können Ar¹ und Ar² miteinander und/oder Ar¹ mit Ar und/oder Ar² mit Ar durch eine Gruppe E verbunden sein;
- E: ist gleich oder verschieden bei jedem Auftreten ausgewählt aus der Gruppe bestehend aus C(R¹)₂, O, S und NR¹;
- R, R¹: ist bei jedem Auftreten gleich oder verschieden ausgewählt aus der Gruppe bestehend aus H, D, F, Cl, Br, I, CN, Si(R²)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils mit einem oder mehreren Resten R² substituiert sein kann, wobei jeweils eine oder mehrere nicht-benachbarte CH₂-Gruppen durch Si(R²)₂, C=NR², P(=O)(R²), SO, SO₂, NR², O, S oder CONR² ersetzt sein können und wobei ein oder mehrere H-Atome durch D, F, Cl, Br oder I ersetzt sein können, einem aromatischen oder heteroaromatischen Ringsystem mit 6 bis 60 C-Atomen, ausgewählt aus der Gruppe bestehend aus Benzol, Naphthalin, Phenanthren, Fluoren, Spirobifluoren, Dibenzofuran und Dibenzothiophen, das jeweils mit einem oder mehreren Resten R² substituiert sein kann, oder einer Kombination aus zwei, drei, vier oder fünf dieser Gruppen, die jeweils gleich oder verschieden sein können, einer Aryloxygruppe mit 5 bis 60 aromatischen Ringatomen, die mit einem oder mehreren Resten R² substituiert sein kann, oder einer Aralkylgruppe mit 5 bis 60 aromatischen Ringatomen, die jeweils mit einem oder mehreren Resten R² substituiert sein kann, wobei optional zwei oder mehr benachbarte Substituenten R bzw. zwei oder mehr benachbarte Substituenten R¹ ein mono- oder polycyclisches, aliphatisches Ringsystem bilden können, das mit einem oder mehreren Resten R² substituiert sein kann;
- R²: ist bei jedem Auftreten gleich oder verschieden ausgewählt aus der Gruppe bestehend aus H, D, F, Cl, Br, I, Si(R³)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils mit einem oder mehreren Resten R³ substituiert sein kann, wobei eine oder mehrere nicht-benachbarte CH₂-Gruppen durch Si(R³)₂, C=NR³, P(=O)(R³), SO, SO₂, NR³, O, S oder CONR³ ersetzt sein können und wobei ein oder mehrere H-Atome durch D, F, Cl, Br oder I ersetzt sein können, einem aromatischen Ringsystem mit 6 bis 60 C-Atomen, ausgewählt aus der Gruppe bestehend aus Benzol, Naphthalin, Phenanthren, Fluoren, Spirobifluoren oder einer Kombination aus zwei, drei, vier oder fünf dieser Gruppen, die jeweils gleich oder verschieden sein können, das jeweils mit einem oder mehreren Resten R³ substituiert sein kann, einer Aryloxygruppe mit 5 bis 60 aromatischen Ringatomen, die mit einem oder mehreren Resten R³ substituiert sein kann, oder einer Aralkylgruppe mit 5 bis 60 aromatischen Ringatomen, die mit einem oder mehreren Resten R³ substituiert sein kann, wobei optional zwei oder mehr benachbarte Substituenten R² ein mono- oder polycyclisches, aliphatisches Ringsystem bilden können, das mit einem oder mehreren Resten R³ substituiert sein kann;
- R³: ist ausgewählt aus der Gruppe bestehend aus H, D, F, einem aliphatischem Kohlenwasserstoffrest mit 1 bis 20 C-Atomen, einem aromatischem Ringsystem mit 6 bis 30 C-Atomen, in dem ein oder mehrere H-Atome durch D oder F ersetzt sein können, wobei zwei oder mehr benachbarte Substituenten R³ miteinander ein mono- oder polycyclisches, aliphatisches, Ringsystem bilden können;
- m: ist 0, 1, 2 oder 3;
- n: ist bei jedem Auftreten gleich oder verschieden 0, 1, 2, 3 oder 4;
- p: ist 0, 1 oder 2;
dabei sind die folgenden Verbindungen von der Erfindung ausgenommen:

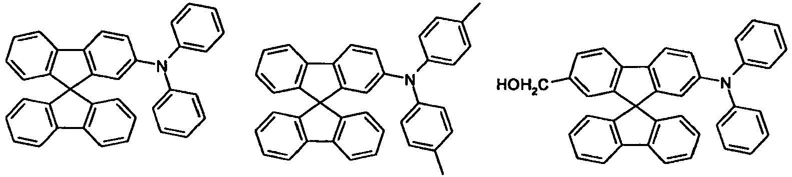

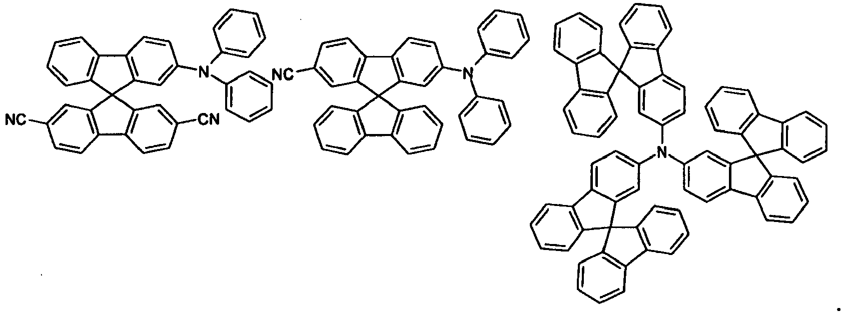

Wie aus den oben aufgeführten Definitionen hervorgeht, enthält die Verbindung gemäß Formel (1) keine heteroaromatischen Substituenten R am Spirobifluoren. Weiterhin enthält sie außer der in der Formel (1) aufgeführten Arylaminogruppe und gegebenenfalls der Gruppe E keine weiteren Aminogruppen bzw. keine Carbazolgruppen.

Unter einer Arylgruppe im Sinne dieser Erfindung wird entweder ein einfacher aromatischer Cyclus, also Benzol, oder eine kondensierte (anellierte) Arylgruppe, beispielsweise Naphthalin oder Phenanthren, verstanden. Miteinander durch Einfachbindung verknüpfte Aromaten, wie zum Beispiel Biphenyl oder Fluoren, werden dagegen nicht als Arylgruppe, sondern als aromatisches Ringsystem bezeichnet.

Ein aromatisches Ringsystem im Sinne dieser Erfindung enthält 6 bis 60 C-Atome im Ringsystem, wobei das aromatische Ringsystem aufgebaut ist aus Benzol, Naphthalin, Phenanthren, Fluoren und Spirobifluoren oder Kombinationen dieser Gruppen. Unter einem aromatischen Ringsystem im Sinne dieser Erfindung soll insbesondere auch ein System verstanden werden, in dem auch mehrere Arylgruppen direkt oder über ein Kohlenstoffatom miteinander verknüpft sind. So sollen beispielsweise insbesondere auch Systeme wie Biphenyl, Terphenyl, Quaterphenyl, Fluoren, 9,9'-Spirobifluoren, 9,9-Diarylfluoren, etc. als aromatische Ringsysteme im Sinne dieser Erfindung verstanden werden. Dabei enthält das aromatische Ringsystem definitionsgemäß keine Aminogruppen. Triarylaminogruppen sind somit von der Definition eines aromatischen Ringsystems nicht umfasst.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenyl-thio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

In einer bevorzugten Ausführungsform der Erfindung ist die Verbindung der Formel (1) ausgewählt aus den Verbindungen der folgenden Formel (2), 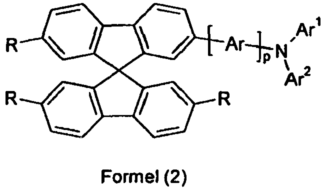 wobei die verwendeten Symbole und Indizes die oben genannten Bedeutungen aufweisen.

In einer besonders bevorzugten Ausführungsform der Erfindung ist die Verbindung der Formel (1) ausgewählt aus den Verbindungen der folgenden Formeln (3a) bzw. (3b), 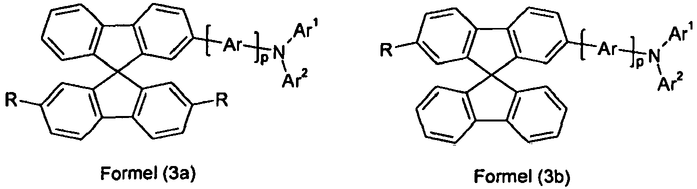 wobei die verwendeten Symbole und Indizes die oben genannten Bedeutungen aufweisen und die Reste R am Spirobifluoren bevorzugt für H stehen.

In einer ganz besonders bevorzugten Ausführungsform der Erfindung ist der Index p = 0, und die Verbindung der Formel (1) ist ausgewählt aus den Verbindungen der folgenden Formeln (4a) bzw. (4b), 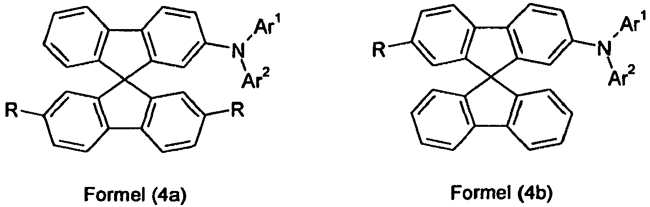 wobei die verwendeten Symbole die oben genannten Bedeutungen aufweisen.

Insbesondere bevorzugt stehen die beiden Reste R in Verbindungen der Formel (4a) bzw. (4b) für H. Insbesondere bevorzugt ist die Verbindung der Formel (1) daher ausgewählt aus den Verbindungen der folgenden Formel (4c), 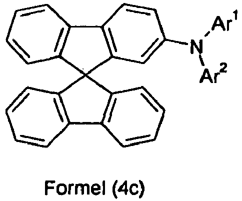 wobei die verwendeten Symbole die oben genannten Bedeutungen aufweisen.

In einer bevorzugten Ausführungsform der Erfindung sind die Gruppen Ar¹ und Ar² gleich oder verschieden bei jedem Auftreten ausgewählt aus den Gruppen der folgenden Formeln (5) bis (28), 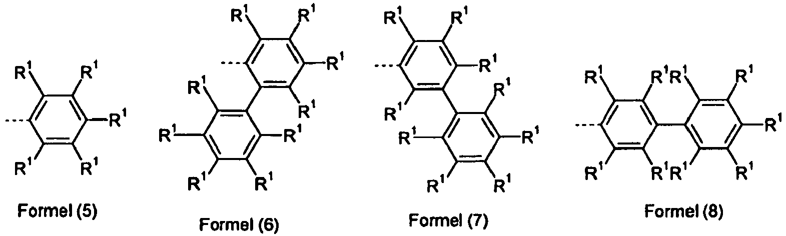 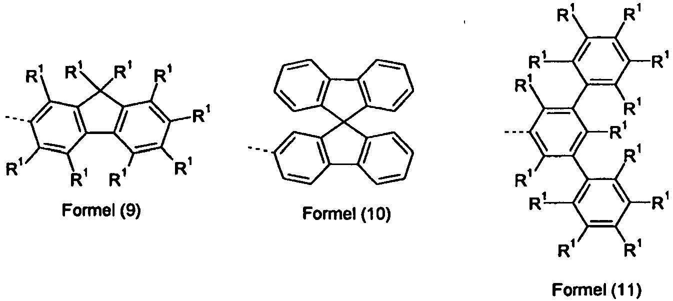 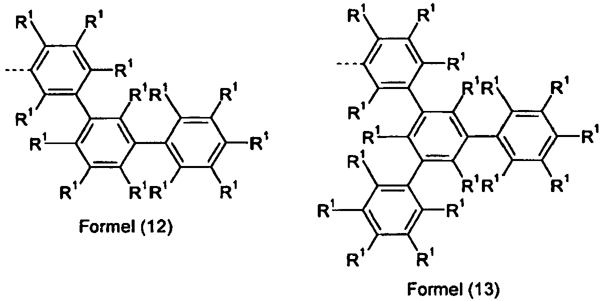 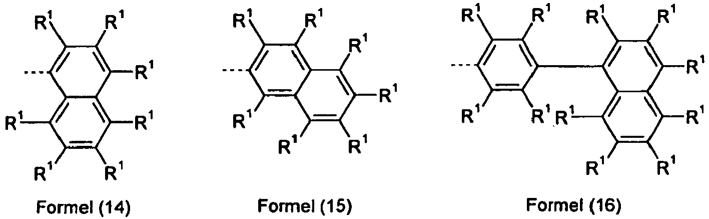 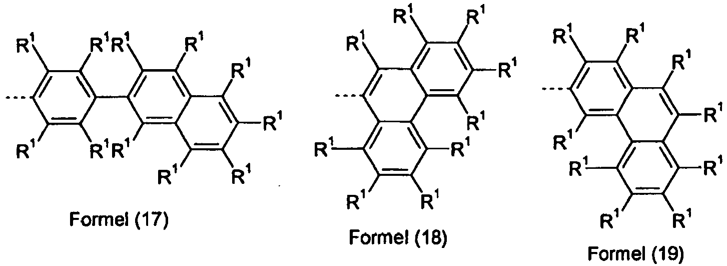 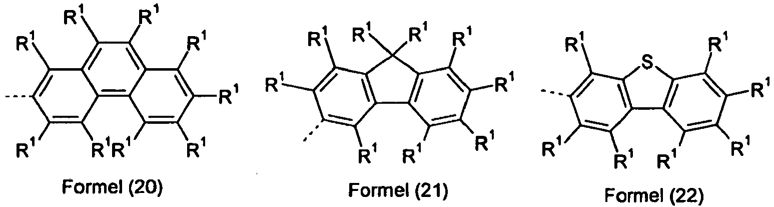 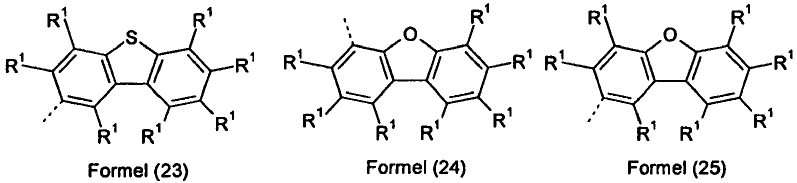 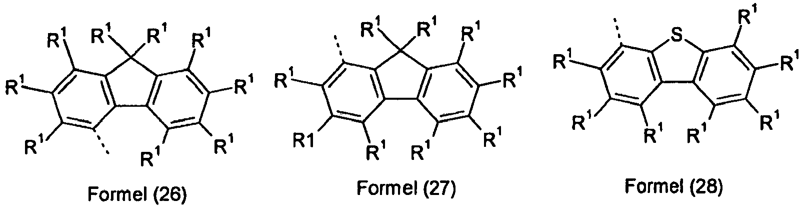 wobei die verwendeten Symbole die oben genannten Bedeutungen haben und die gestrichelte Bindung die Position der Bindung der Gruppe an den Stickstoff andeutet.

In einer besonders bevorzugten Ausführungsform der Erfindung sind die Gruppen Ar¹ und Ar² gleich oder verschieden bei jedem Auftreten ausgewählt aus den Gruppen der folgenden Formeln (5a) bis (28a), 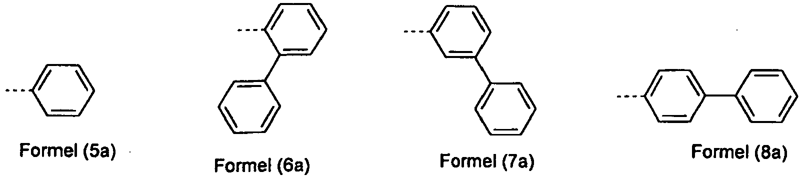 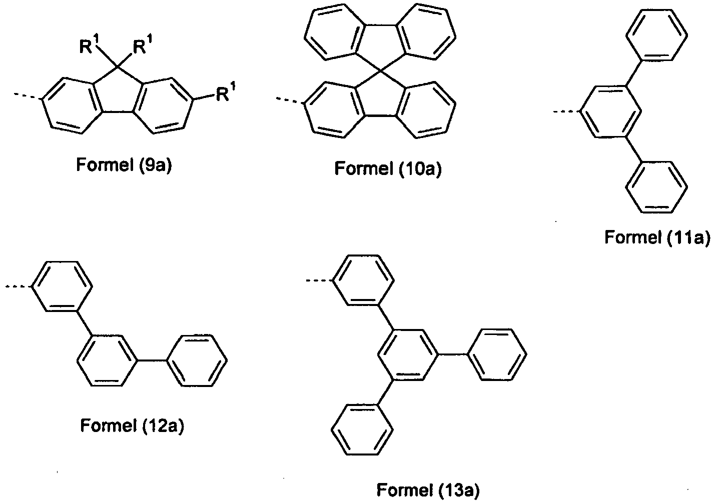 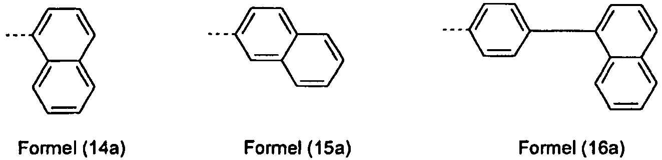 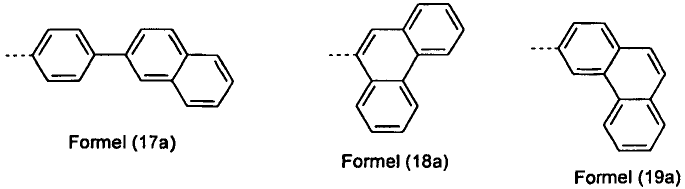 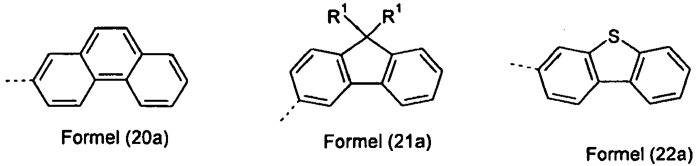 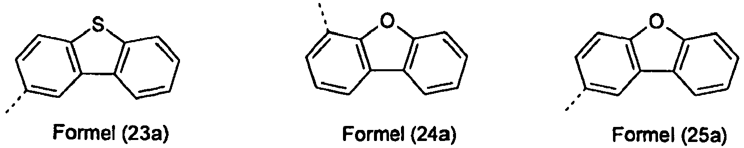 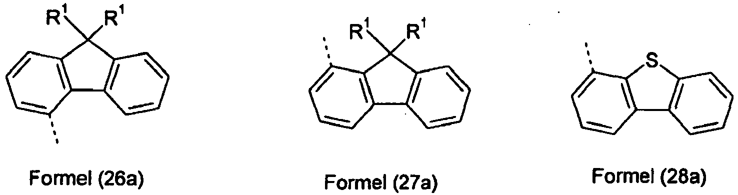 wobei die verwendeten Symbole die oben genannten Bedeutungen haben und die gestrichelte Bindung die Position der Bindung der Gruppe an den Stickstoff andeutet.

Dabei können die beiden Gruppen Ar¹ und Ar² der oben genannten Formeln (5) bis (28) bzw. (5a) bis (28a), die an den Stickstoff binden, beliebig miteinander kombiniert werden. Die Gruppen der Formeln (5), (6), (7), (8), (9), (10), (11), (12), (13), (21), (22), (23), (24) und (25) bzw. (5a), (6a), (7a), (8a), (9a), (10a), (11a), (12a), (13a), (21a), (22a), (23a), (24a) und (25a) sind dabei besonders bevorzugt.

Besonders bevorzugte Gruppen -NAr¹Ar² sind daher Gruppen, die die folgenden Kombinationen für Ar¹ und Ar² aufweisen:

| Ar¹ | Ar² |
|---|---|
| Formel (5) | Formel (5) |
| Formel (5) | Formel (6) |
| Formel (5) | Formel (7) |
| Formel (5) | Formel (8) |
| Formel (5) | Formel (9) |
| Formel (5) | Formel (10) |
| Formel (5) | Formel (11) |
| Formel (5) | Formel (12) |
| Formel (5) | Formel (13) |
| Formel (5) | Formel (21) |
| Formel (5) | Formel (22) |
| Formel (5) | Formel (23) |
| Formel (5) | Formel (24) |
| Formel (5) | Formel (25) |
| Formel (6) | Formel (6) |
| Formel (6) | Formel (7) |
| Formel (6) | Formel (8) |
| Formel (6) | Formel (9) |
| Formel (6) | Formel (10) |
| Formel (6) | Formel (11) |
| Formel (6) | Formel (12) |
| Formel (6) | Formel (13) |
| Formel (6) | Formel (21) |
| Formel (6) | Formel (22) |
| Formel (6) | Formel (23) |
| Formel (6) | Formel (24) |
| Formel (6) | Formel (25) |
| Formel (7) | Formel (7) |
| Formel (7) | Formel (8) |
| Formel (7) | Formel (9) |
| Formel (7) | Formel (10) |
| Formel (7) | Formel (11) |
| Formel (7) | Formel (12) |
| Formel (7) | Formel (13) |
| Formel (7) | Formel (21) |
| Formel (7) | Formel (22) |
| Formel (7) | Formel (23) |
| Formel (7) | Formel (24) |
| Formel (7) | Formel (25) |
| Formel (8) | Formel (8) |
| Formel (8) | Formel (9) |
| Formel (8) | Formel (10) |
| Formel (8) | Formel (11) |
| Formel (8) | Formel (12) |
| Formel (8) | Formel (13) |
| Formel (8) | Formel (21) |
| Formel (8) | Formel (22) |
| Formel (8) | Formel (23) |
| Formel (8) | Formel (24) |
| Formel (8) | Formel (25) |
| Formel (9) | Formel (9) |
| Formel (9) | Formel (10) |
| Formel (9) | Formel (11) |
| Formel (9) | Formel (12) |
| Formel (9) | Formel (13) |
| Formel (9) | Formel (21) |
| Formel (9) | Formel (22) |
| Formel (9) | Formel (23) |
| Formel (9) | Formel (24) |
| Formel (9) | Formel (25) |
| Formel (10) | Formel (10) |
| Formel (10) | Formel (11) |
| Formel (10) | Formel (12) |
| Formel (10) | Formel (13) |
| Formel (10) | Formel (21) |
| Formel (10) | Formel (22) |
| Formel (10) | Formel (23) |
| Formel (10) | Formel (24) |
| Formel (10) | Formel (25) |
| Formel (11) | Formel (11) |
| Formel (11) | Formel (12) |
| Formel (11) | Formel (13) |
| Formel (11) | Formel (21) |
| Formel (11) | Formel (22) |
| Formel (11) | Formel (23) |
| Formel (11) | Formel (24) |
| Formel (11) | Formel (25) |
| Formel (12) | Formel (12) |
| Formel (12) | Formel (13) |
| Formel (12) | Formel (21) |
| Formel (12) | Formel (22) |
| Formel (12) | Formel (23) |
| Formel (12) | Formel (24) |
| Formel (12) | Formel (25) |
| Formel (13) | Formel (13) |
| Formel (13) | Formel (21) |
| Formel (13) | Formel (22) |
| Formel (13) | Formel (23) |
| Formel (13) | Formel (24) |
| Formel (13) | Formel (25) |

Ganz besonders bevorzugte Gruppen -NAr¹Ar² sind Gruppen, die die Kombinationen Ar¹ und Ar² aus der oben genannten Tabelle aufweisen, wobei statt Formel (5) bis (28) jeweils Formel (5a) bis (28a) eingesetzt wird.

In einer bevorzugten Ausführungsform der Erfindung ist die Gruppe Ar¹ eine Gruppe der Formel (6), (7), (8), (9) oder (21) und insbesondere eine Gruppe der Formel (6a), (7a), (8a), (9a) oder (21a), insbesondere (6a) oder (9a). Insbesondere mit diesen Gruppen Ar¹ werden sehr gute Ergebnisse der organischen Elektrolumineszenzvorrichtung erzielt. Außer den in der obigen Tabelle aufgeführten Kombinationen sind daher noch die folgenden Kombinationen von Ar¹ und Ar² besonders bevorzugt:

| Ar¹ | Ar² |
|---|---|
| Formel (6) | Formel (14) |
| Formel (6) | Formel (15) |
| Formel (6) | Formel (16) |
| Formel (6) | Formel (17) |
| Formel (6) | Formel (18) |
| Formel (6) | Formel (19) |
| Formel (6) | Formel (20) |
| Formel (6) | Formel (26) |
| Formel (6) | Formel (27) |
| Formel (6) | Formel (28) |
| Formel (9) | Formel (14) |
| Formel (9) | Formel (15) |
| Formel (9) | Formel (16) |
| Formel (9) | Formel (17) |
| Formel (9) | Formel (18) |
| Formel (9) | Formel (19) |
| Formel (9) | Formel (20) |
| Formel (9) | Formel (26) |
| Formel (9) | Formel (27) |
| Formel (9) | Formel (28) |

Ganz besonders bevorzugte Gruppen -NAr¹Ar² sind daher weiterhin Gruppen, die die Kombinationen Ar¹ und Ar² aus der oben genannten Tabelle aufweisen, wobei statt Formel (6) Formel (6a) bzw. statt Formel (9) Formel (9a) und statt den Formeln (14) bis (20) bzw. (26) bis (28) entsprechend die Formeln (14a) bis (20a) bzw. (26a) bis (28a) eingesetzt werden.

In einer bevorzugten Ausführungsform der Erfindung sind die Gruppen Ar¹ und Ar² unterschiedlich voneinander.

In einer weiteren bevorzugten Ausführungsform der Erfindung ist mindestens eine der Gruppen Ar¹ und Ar² unverbrückt, enthält also weder ein Fluoren noch ein Spirobifluoren, also keine Gruppe der Formel (8), (9) oder (21) bzw. (8a), (9a) oder (21a). Wenn die Verbindung der Formel (1) bis (4) als Matrixmaterial für grün phosphoreszierende Emitter eingesetzt wird, sind bevorzugt beide Gruppen Ar¹ und Ar² unverbrückt, enthalten also weder ein Fluoren noch ein Spirobifluoren.

In einer weiteren bevorzugten Ausführungsform der Erfindung enthält die Verbindung der Formel (1), (2), (3a), (3b), (4a), (4b) oder (4c) als Gruppe Ar¹ und Ar² kein Spirobifluoren.

Wenn in den Verbindungen der Formel (1), (2), (3a), (3b), (4a), (4b) oder (4c) die Gruppen Ar¹ und Ar² durch eine Gruppe E miteinander verknüpft sind, dann hat die Gruppe -NAr¹Ar² bevorzugt die Struktur gemäß einer der folgenden Formeln (29), (30), (31) oder (32), 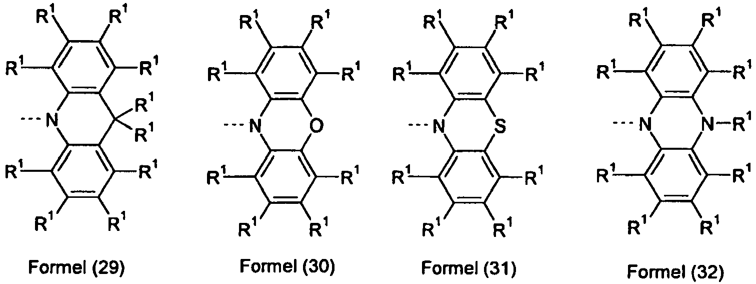 wobei die verwendeten Symbole die oben genannten Bedeutungen aufweisen und die gestrichelte Bindung die Bindung an das Spirobifluoren bzw. an Ar andeutet.

Bevorzugte Ausführungsformen der Formeln (29) bis (32) sind die folgenden Formeln (29a) bis (32a), 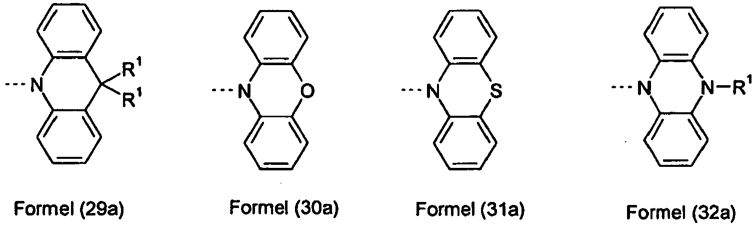 wobei die verwendeten Symbole die oben genannten Bedeutungen aufweisen und die gestrichelte Bindung die Bindung an das Spirobifluoren bzw. an Ar andeutet.

Wenn die Gruppe Ar mit Ar¹ durch eine Gruppe E miteinander verknüpft ist, dann hat die Gruppe -Ar-NAr¹Ar² bevorzugt die Struktur einer der folgenden Formeln (33) bis (36) und die gestrichelte Bindung deutet die Bindung an das Spirobifluoren an. Analoges gilt für eine Verknüpfung der Gruppe Ar mit Ar². 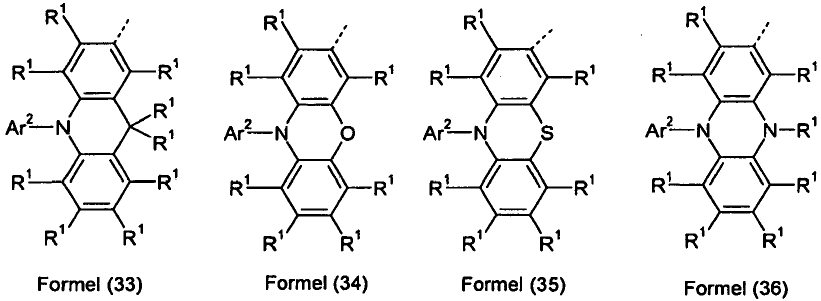 wobei die verwendeten Symbole die oben genannten Bedeutungen aufweisen und die gestrichelte Bindung die Bindung an das Spirobifluoren andeutet.

Bevorzugte Ausführungsformen der Formeln (33) bis (36) sind die folgenden Formeln (33a) bis (36a), 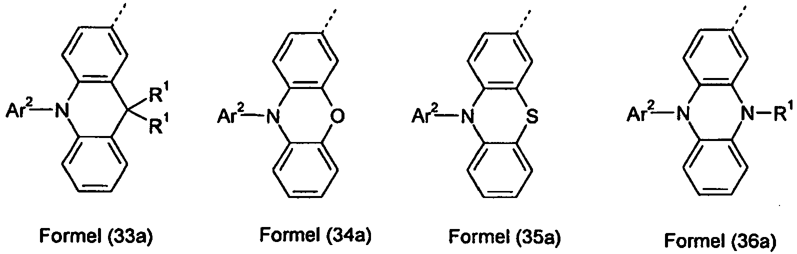 wobei die verwendeten Symbole die oben genannten Bedeutungen aufweisen und die gestrichelte Bindung die Bindung an das Spirobifluoren andeutet.

In einer weiteren bevorzugten Ausführungsform der Erfindung ist der Index p = 1 oder 2, und die Gruppe -(Ar)ₚ- steht für eine Gruppe gemäß einer der folgenden Formeln (37) bis (50), 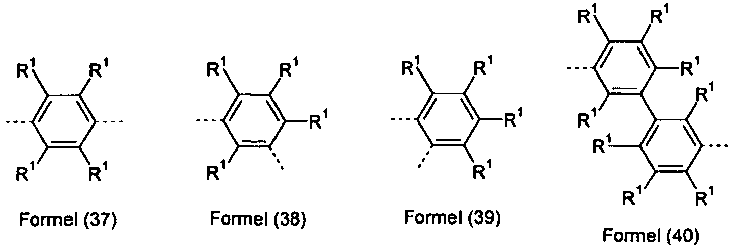 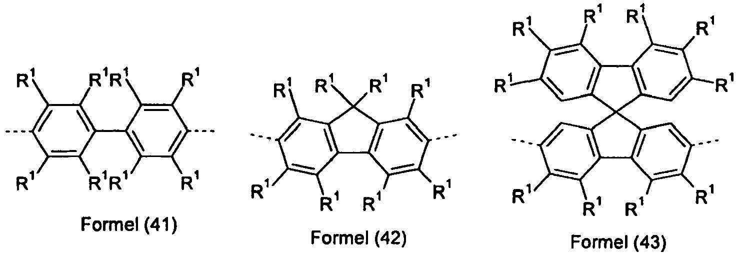 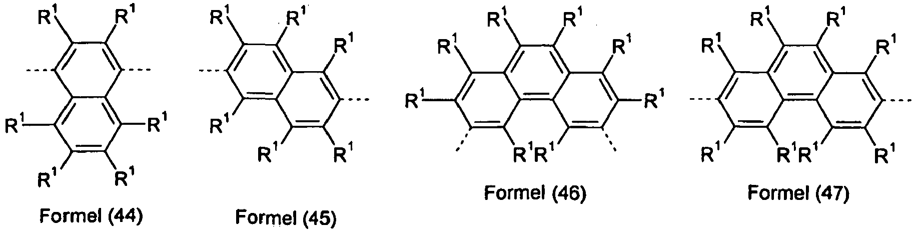 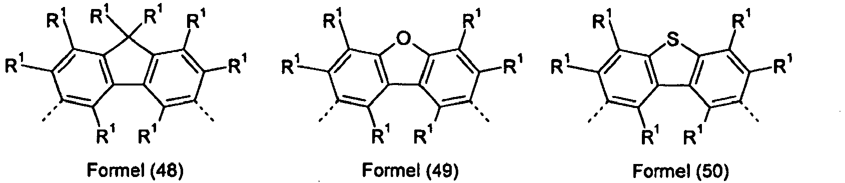 wobei die verwendeten Symbole die oben genannten Bedeutungen aufweisen und eine gestrichelte Bindung die Bindung an das Spirobifluoren andeutet und die andere gestrichelte Bindung die Bindung an das Stickstoffatom andeutet.

In einer besonders bevorzugten Ausführungsform der Erfindung ist der Index p = 1 oder 2, und die Gruppe -(Ar)ₚ- steht für eine Gruppe gemäß einer der folgenden Formeln (37a) bis (50a), 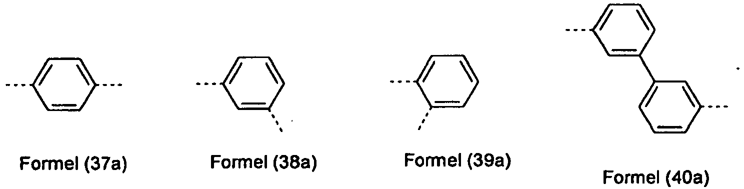 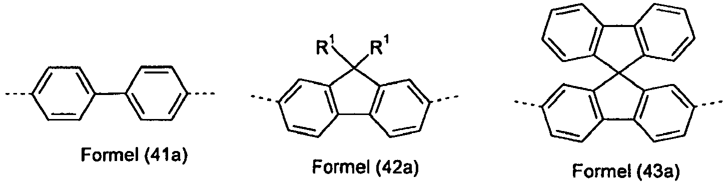 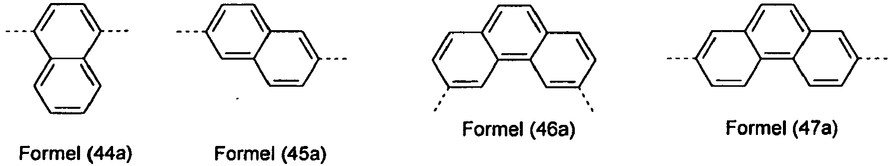 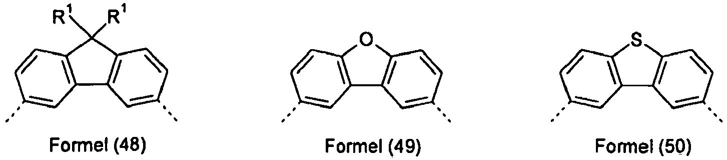 wobei die verwendeten Symbole die oben genannten Bedeutungen aufweisen und die eine gestrichelte Bindung die Bindung an das Spirobifluoren andeutet und die andere gestrichelte Bindung die Bindung an das Stickstoffatom andeutet.

In einer bevorzugten Ausführungsform der Erfindung ist R in den Verbindungen der Formel (1) bis (4) gleich oder verschieden bei jedem Auftreten ausgewählt aus der Gruppe bestehend aus H, D, F, Si(R²)₃, CN, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, einem aromatischen Ringsystem mit 6 bis 60 C-Atomen, ausgewählt aus der Gruppe bestehend aus Benzol, Naphthalin, Phenanthren, Fluoren, Spirobifluoren oder einer Kombination aus zwei, drei oder vier dieser Gruppen, die jeweils gleich oder verschieden sein können, das jeweils mit einem oder mehreren Resten R² substituiert sein kann, wobei optional zwei oder mehr benachbarte Substituenten R ein mono- oder polycyclisches, aliphatisches Ringsystem bilden können, das mit einem oder mehreren Resten R² substituiert sein kann.

In einer besonders bevorzugten Ausführungsform der Erfindung ist R in den Verbindungen der Formel (1) bis (4) gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R² substituiert sein kann, einem aromatischen Ringsystem mit 6 bis 60 C-Atomen, ausgewählt aus der Gruppe bestehend aus Benzol, Naphthalin, Phenanthren, Fluoren, Spirobifluoren oder einer Kombination aus zwei oder drei dieser Gruppen, die jeweils gleich oder verschieden sein können, das jeweils mit einem oder mehreren Resten R² substituiert sein kann, wobei optional zwei oder mehr benachbarte Substituenten R ein mono- oder polycyclisches, aliphatisches Ringsystem bilden können, das mit einem oder mehreren Resten R² substituiert sein kann.

In einer ganz besonders bevorzugten Ausführungsform der Erfindung ist R in den Verbindungen der Formel (1) bis (4) gleich H.

In einer weiteren bevorzugten Ausführungsform der Erfindung ist der Rest R¹, welcher an Ar¹ bzw. Ar² bzw. Ar bindet, gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen.

In einer weiteren bevorzugten Ausführungsform der Erfindung ist mindestens ein Rest R¹, welche in ortho-Position der Arylgruppe von Ar¹ bzw. Ar² bindet, welche direkt an den Stickstoff gebunden ist, ungleich Wasserstoff oder Deuterium. Dies gilt insbesondere dann, wenn an der Arylgruppe nicht bereits eine weitere Arylgruppe in ortho-Position gebunden ist, wie dies beispielsweise in Formel (6) der Fall ist.

Weiterhin kann es bevorzugt sein, wenn die beiden Substituenten R¹ in 9-Position eines Fluorens zusammen einen Cycloalkylring bilden, bevorzugt mit 3 bis 8 C-Atomen, besonders bevorzugt mit 5 oder 6 C-Atomen.

In einer weiteren bevorzugten Ausführungsform ist R¹, welcher in Formel (29) bzw. (33) an die Kohlenstoffbrücke bindet, gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das wie oben definiert ist und das mit einem oder mehreren Resten R² substituiert sein kann. Dabei können die beiden Gruppen R¹ auch miteinander ein Ringsystem bilden, welches aliphatisch oder zusätzlich zur oben gegebenen Definition von R¹ auch aromatisch sein kann. Durch Ringbildung wird ein Spiro-System aufgespannt.

In einer weiteren bevorzugten Ausführungsform ist R¹, welcher in Formel (32) bzw. (36) an die Stickstoffbrücke bindet, ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, insbesondere ein aromatisches Ringsystem mit 6 bis 24 C-Atomen, das wie oben definiert ist und das mit einem oder mehreren Resten R² substituiert sein kann.

Dabei haben für Verbindungen, die durch Vakuumverdampfung verarbeitet werden, die Alkylgruppen bevorzugt nicht mehr als vier C-Atome, besonders bevorzugt nicht mehr als 1 C-Atom. Für Verbindungen, die aus Lösung verarbeitet werden, eignen sich auch Verbindungen, die mit linearen, verzweigten oder cyclischen Alkylgruppen mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

In einer bevorzugten Ausführungsform der Erfindung ist R² gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 30 C-Atomen, das wie oben definiert ist und das jeweils mit einem oder mehreren Resten R³ substituiert sein kann.

In einer besonders bevorzugten Ausführungsform der Erfindung ist R² gleich oder verschieden bei jedem Auftreten ausgewählt aus der Gruppe bestehend aus H, D, einer geradkettigen Alkylgruppe mit 1 bis 5 C-Atomen oder einer verzweigten oder cyclischen Alkylgruppe mit 3 bis 5 C-Atomen, oder einem aromatischen Ringsystem mit 6 bis 18 C-Atomen, das wie oben definiert ist.

Besonders bevorzugt sind Verbindungen der Formel (1), (2), (3a), (3b), (4a), (4b) bzw. (4c), in denen die oben genannten bevorzugten Ausführungsformen gleichzeitig auftreten. Besonders bevorzugt sind daher Verbindungen, für die gilt:
- Ar: ist gleich oder verschieden bei jedem Auftreten ein aromatisches Ringsystem, wobei für p = 1 oder 2 -(Ar)p- ausgewählt ist aus den Gruppen der Formel (37) bis (50); dabei kann Ar auch mit Ar¹ und/oder Ar² durch eine Gruppe E verbunden sein;
- Ar¹, Ar²: ist gleich oder verschieden bei jedem Auftreten ein aromatisches Ringsystem, ausgewählt aus den Gruppen der Formel (5) bis (28);
oder -NAr¹Ar² steht für eine Gruppe gemäß einer der Formeln (29) bis (32);
oder -Ar-NAr¹Ar² steht für eine Gruppe gemäß einer der Formeln (33) bis (36);
- E: ist bei jedem Auftreten gleich oder verschieden C(R¹)₂, N(R¹), O oder S;
- R: ist bei jedem Auftreten gleich oder verschieden ausgewählt aus der Gruppe bestehend aus H, D, F, Si(R²)₃, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, einem aromatischen Ringsystem mit 6 bis 60 C-Atomen, ausgewählt aus der Gruppe bestehend aus Benzol, Naphthalin, Phenanthren, Fluoren, Spirobifluoren oder einer Kombination aus zwei oder drei dieser Gruppen, die jeweils gleich oder verschieden sein können, das jeweils mit einem oder mehreren Resten R² substituiert sein kann, wobei optional zwei oder mehr benachbarte Substituenten R ein mono- oder polycyclisches, aliphatisches Ringsystem bilden können, das mit einem oder mehreren Resten R² substituiert sein kann;
- R¹: ist, wenn der Rest R¹ and Ar¹ oder Ar² bindet, gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe
mit 3 bis 10 C-Atome;
oder R¹, welcher in Formel (29) bzw. (33) an die Kohlenstoffbrücke bindet, ist gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R² substituiert sein kann; dabei können die beiden Reste R¹ auch miteinander ein aliphatisches oder aromatisches Ringsystem bilden;
oder R¹, welcher in Formel (32) bzw. (36) an die Stickstoffbrücke bindet, ist ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R² substituiert sein kann;
- R²: ist bei jedem Auftreten gleich oder verschieden ausgewählt aus der Gruppe bestehend aus H, D, einer geradkettigen Alkylgruppe mit 1 bis 5 C-Atomen oder einer verzweigten oder cyclischen Alkylgruppe mit 3 bis 5 C-Atomen, oder einem aromatischen Ringsystem mit 6 bis 18 C-Atomen;
- R³: ist ausgewählt aus der Gruppe bestehend aus H, D, F, einem aliphatischem Kohlenwasserstoffrest mit 1 bis 10 C-Atomen, einem aromatischem Ringsystem mit 6 bis 24 C-Atomen, in dem ein oder mehrere H-Atome durch D oder F ersetzt sein können, wobei zwei oder mehr benachbarte Substituenten R³ miteinander ein mono- oder polycyclisches, aliphatisches, aromatisches oder heteroaromatisches Ringsystem bilden können;
- m: ist 0, 1 oder 2;
- n: ist bei jedem Auftreten gleich oder verschieden 0, 1 oder 2;
- p: ist 0, 1 oder 2.

Ganz besonders bevorzugt sind Verbindungen der Formel (1), (2), (3a), (3b), (4a), (4b) bzw. (4c), für die gilt:
- Ar: ist gleich oder verschieden bei jedem Auftreten ein aromatisches Ringsystem, wobei für p = 1 oder 2 -(Ar)p- ausgewählt ist aus den Gruppen der Formel (37a) bis (50a); dabei kann Ar auch mit Ar¹ oder Ar² durch eine Gruppe E verbunden sein;
- Ar¹, Ar²: ist gleich oder verschieden bei jedem Auftreten ein aromatisches Ringsystem, ausgewählt aus den Gruppen der Formel (5a) bis (28a), wobei mindestens eine Gruppe Ar¹ und/oder Ar² unverbrückte Gruppe darstellt;
oder -NAr¹Ar² steht für eine Gruppe gemäß einer der Formeln (29a) bis (32a);
oder Ar-NAr¹Ar² steht für eine Gruppe gemäß einer der Formeln (33a) bis (36a);
- E: ist bei jedem Auftreten gleich oder verschieden C(R¹)₂, NR¹, O oder S;
- R: ist bei jedem Auftreten gleich oder verschieden ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R² substituiert sein kann, einem aromatischen Ringsystem mit 6 bis 60 C-Atomen, ausgewählt aus der Gruppe bestehend aus Benzol, Naphthalin, Phenanthren, Fluoren, Spirobifluoren oder einer Kombination aus zwei dieser Gruppen, die jeweils gleich oder verschieden sein können, das jeweils mit einem oder mehreren Resten R² substituiert sein kann, wobei optional zwei oder mehr benachbarte Substituenten R ein mono- oder polycyclisches, aliphatisches Ringsystem bilden können, das mit einem oder mehreren Resten R² substituiert sein kann; bevorzugt ist R gleich H;
- R¹: ist, wenn der Rest R¹ and Ar¹ oder Ar² bindet, gleich oder verschieden bei jedem Auftreten ausgewählt aus der Gruppe bestehend aus H, D, einer geradkettigen Alkylgruppe mit 1 bis 5 C-Atomen oder einer verzweigten oder cyclischen Alkylgruppe mit 3 bis 5 C-Atome;
oder R¹, welcher in Formel (29) bzw. (33) an die Kohlenstoffbrücke bindet, ist gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R² substituiert sein kann; dabei können die beiden Reste R¹ auch miteinander ein aliphatisches oder aromatisches Ringsystem bilden;
oder R¹, welcher in Formel (32) bzw. (36) an die Stickstoffbrücke bindet, ist ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 24 C-Atomen, das mit einem oder mehreren Resten R² substituiert sein kann;
- R²: ist bei jedem Auftreten gleich oder verschieden ausgewählt aus der Gruppe bestehend aus H, D, einer geradkettigen Alkylgruppe mit 1 bis 5 C-Atomen oder einer verzweigten oder cyclischen Alkylgruppe mit 3 bis 5 C-Atomen, oder einem aromatischen Ringsystem mit 6 bis 18 C-Atomen;
- R³: ist ausgewählt aus der Gruppe bestehend aus H, D, F, einem aliphatischem Kohlenwasserstoffrest mit 1 bis 5 C-Atomen, einem aromatischem Ringsystem mit 6 bis 12 C-Atomen, in dem ein oder mehrere H-Atome durch D oder F ersetzt sein können, wobei zwei oder mehr benachbarte Substituenten R³ miteinander ein mono- oder polycyclisches, aliphatisches, aromatisches oder heteroaromatisches Ringsystem bilden können;
- m: ist 0;
- n: ist bei jedem Auftreten gleich oder verschieden 0 oder 1;
- p: ist 0, 1 oder 2.

Beispiele für geeignete erfindungsgemäße Verbindungen sind die in der folgenden Tabelle aufgeführten Verbindungen:

| | | |
|---|---|---|
| 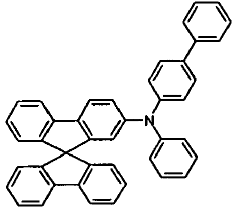 | 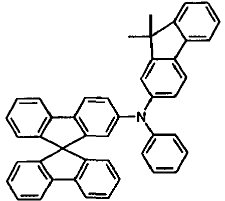 | 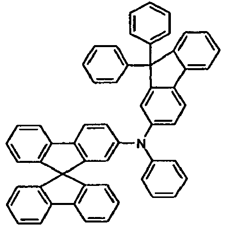 |
| (1) | (2) | (3) |
| 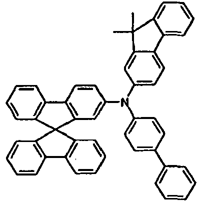 | 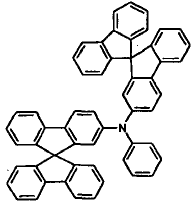 | 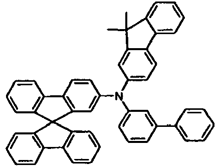 |
| (4) | (5) | (6) |
| 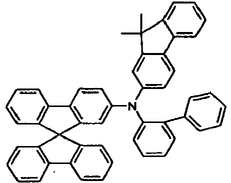 | 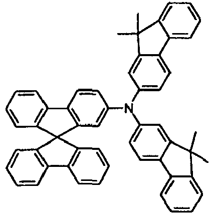 | 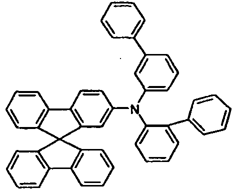 |
| (7) | (8) | (9) |
| 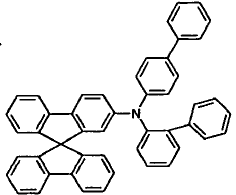 | 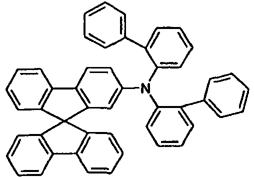 | 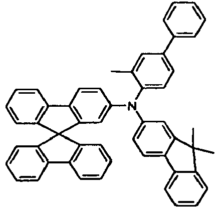 |
| (10) | (11) | (12) |
| 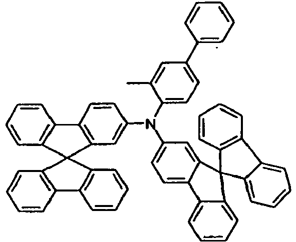 | 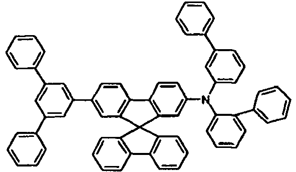 | 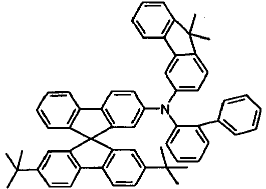 |
| (13) | (14) | (15) |
| 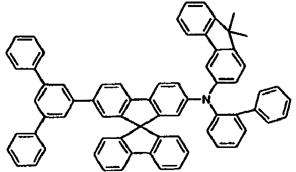 | 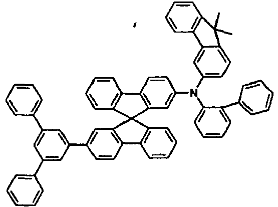 | 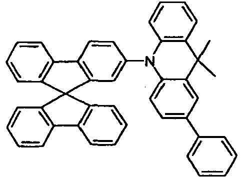 |
| (16) | (17) | (18) |
| 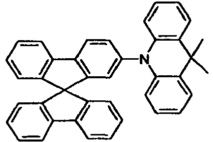 | 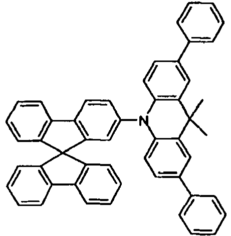 | 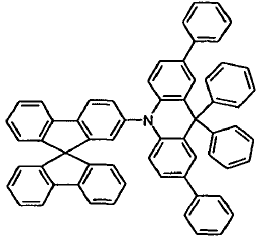 |
| (19) | (20) | (21) |
| 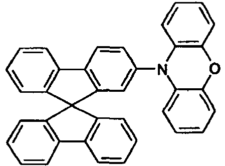 | 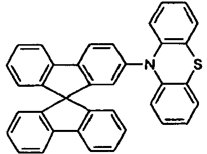 | 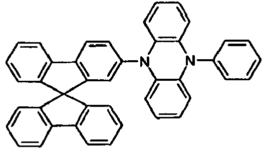 |
| (22) | (23) | (24) |
| 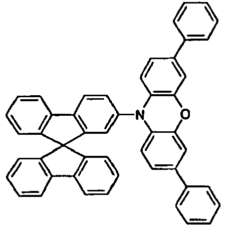 | 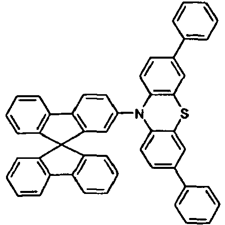 | 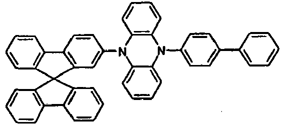 |
| (25) | (26) | (27) |
| 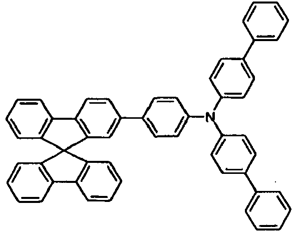 | 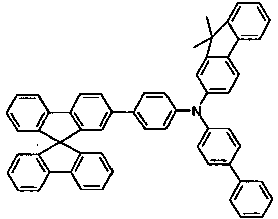 | 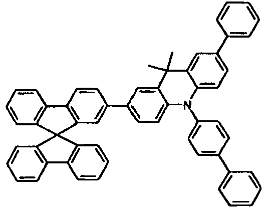 |
| (28) | (29) | (30) |
| 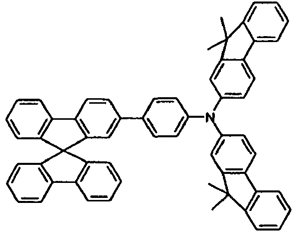 | 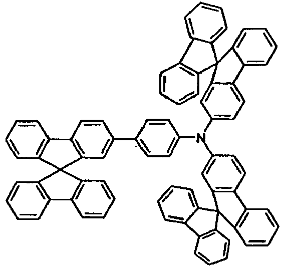 | 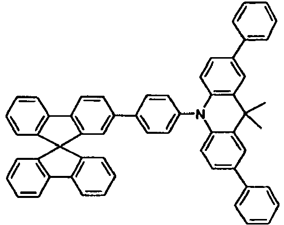 |
| (31) | (32) | (33) |
| 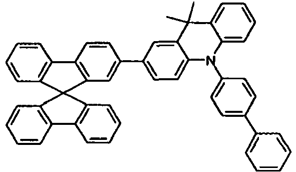 | 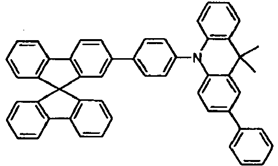 | 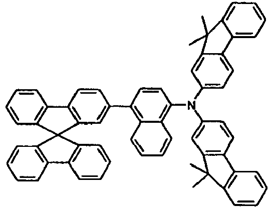 |
| (34) | (35) | (36) |
| 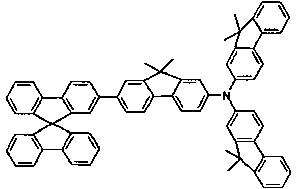 | 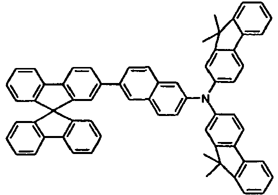 | 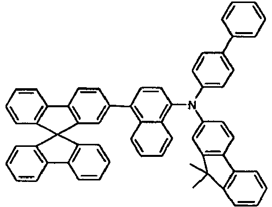 |
| (37) | (38) | (39) |
| 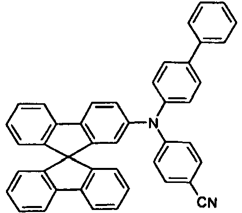 | 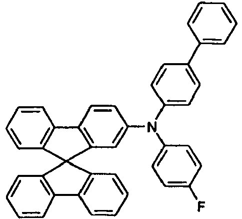 | 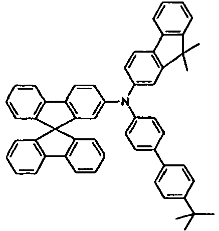 |
| (40) | (41) | (42) |
| 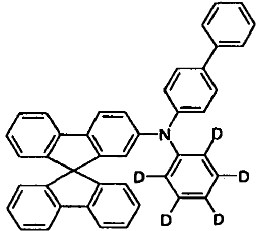 | 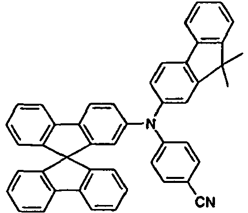 | 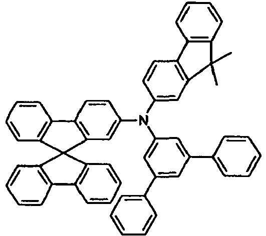 |
| (43) | (44) | (45) |
| 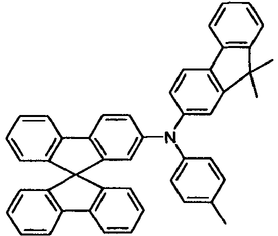 | 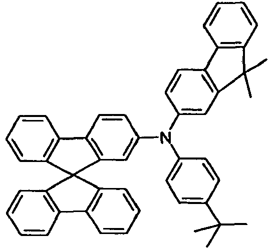 | 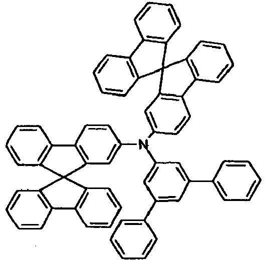 |
| (46) | (47) | (48) |
| 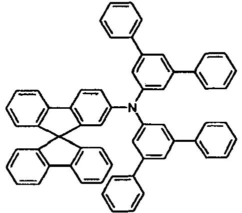 | 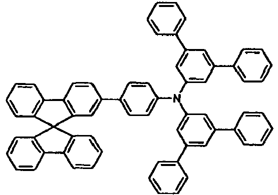 | 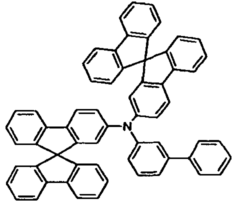 |
| (49) | (50) | (51) |
| 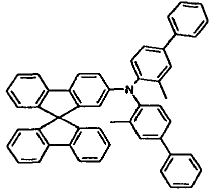 | 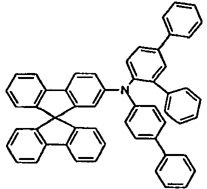 | 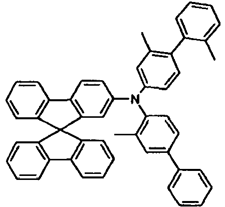 |
| (52) | (53) | (54) |
| 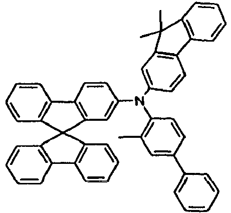 | 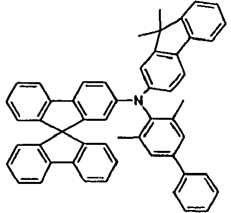 | 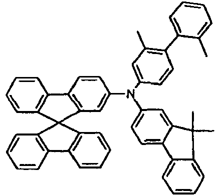 |
| (55) | (56) | (57) |
| 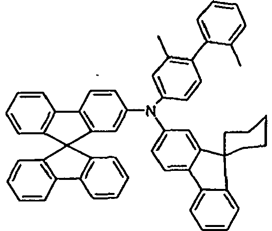 | 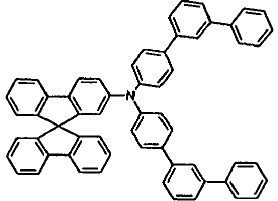 | 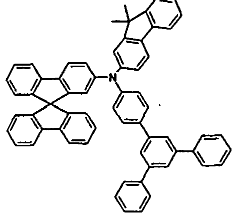 |
| (58) | (59) | (60) |
| 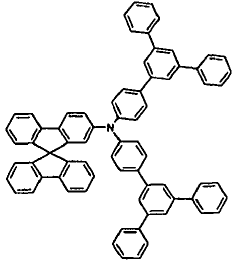 | 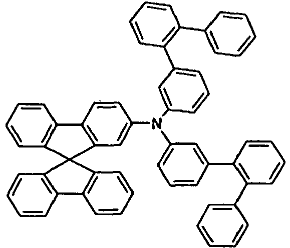 | 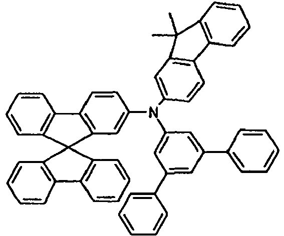 |
| (61) | (62) | (63) |
| 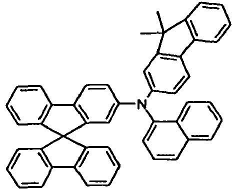 | 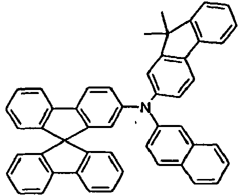 | 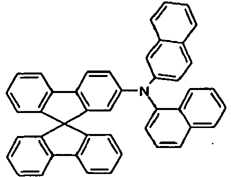 |
| (64) | (65) | (66) |
| 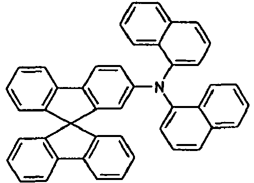 | 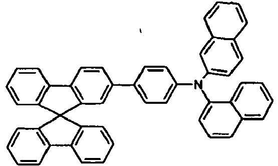 | 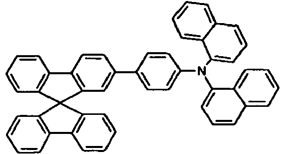 |
| (67) | (68) | (69) |
| 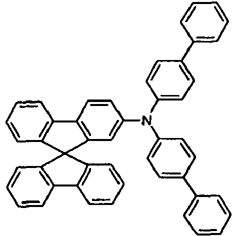 | 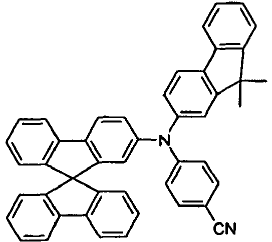 | 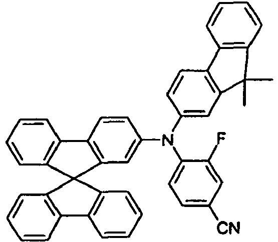 |
| (70) | (71) | (72) |
| 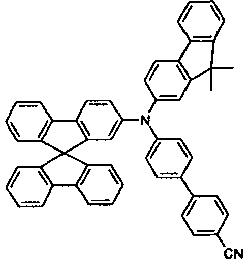 | 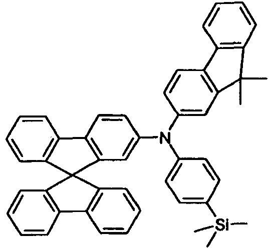 | 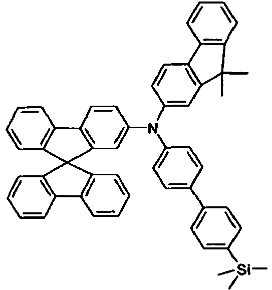 |
| (73) | (74) | (75) |
| 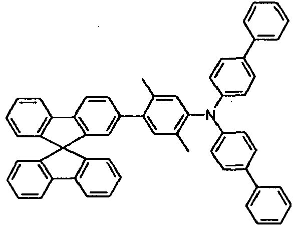 | 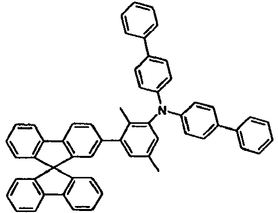 | 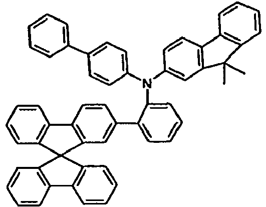 |
| (76) | (77) | (78) |
| 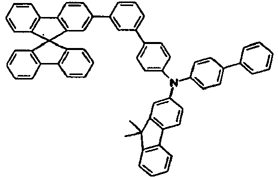 | 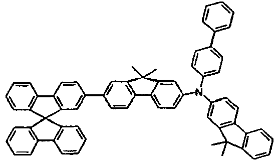 | 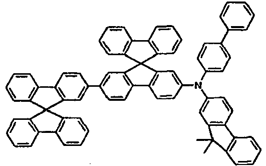 |
| (79) | (80) | (81) |
| 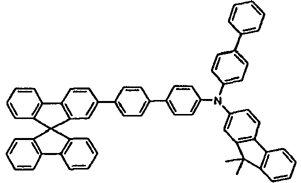 | 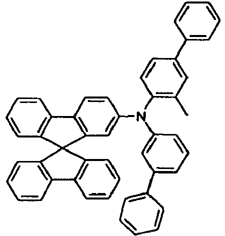 | 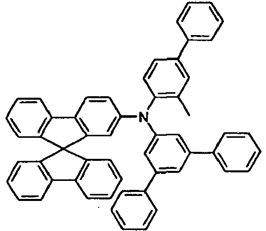 |
| (82) | (83) | (84) |
| 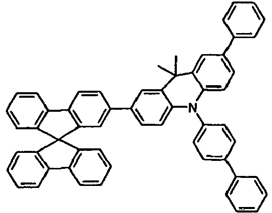 | 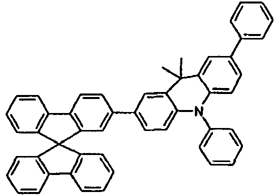 | 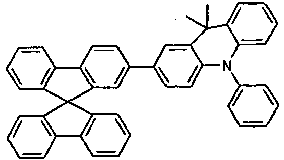 |
| (85) | (86) | (87) |
| 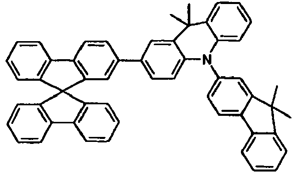 | 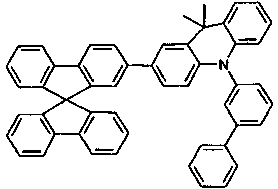 | 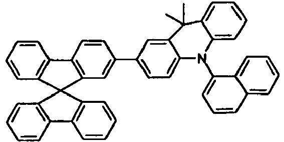 |
| (88) | (89) | (90) |
| 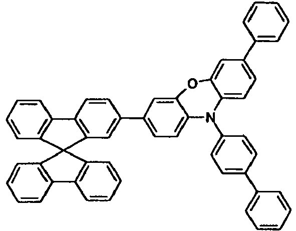 | 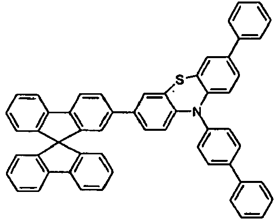 | 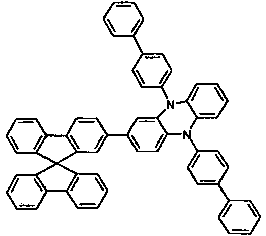 |
| (91) | (92) | (93) |
| 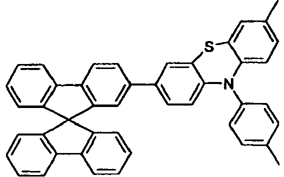 | 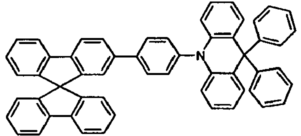 | 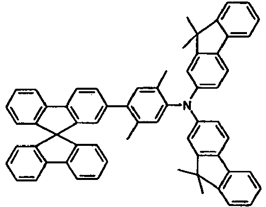 |
| (94) | (95) | (96) |
| 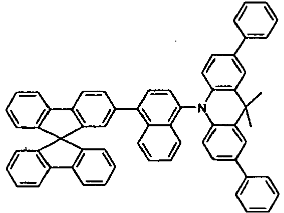 | 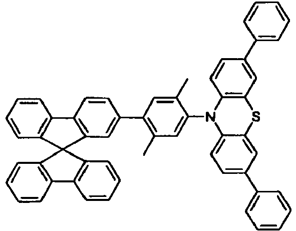 | 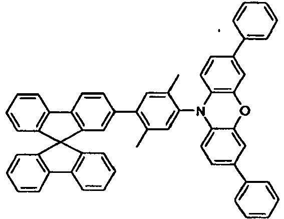 |
| (97) | (98) | (99) |
| 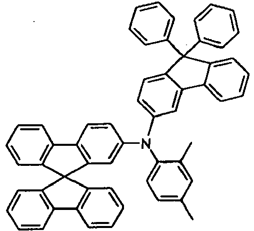 | 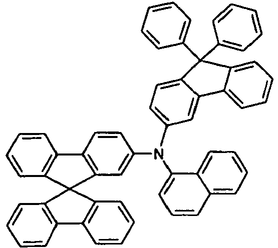 | 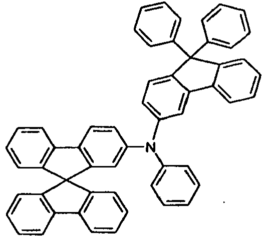 |
| (100) | (101) | (102) |
| 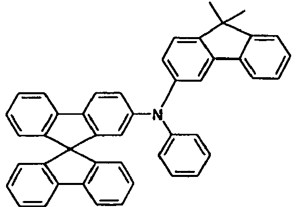 | 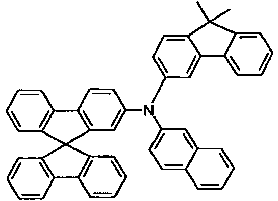 | 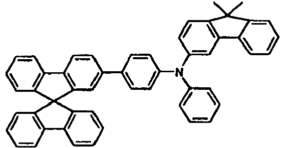 |
| (103) | (104) | (105) |
| 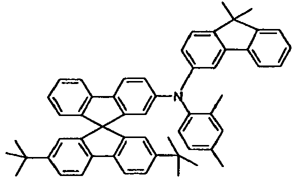 | 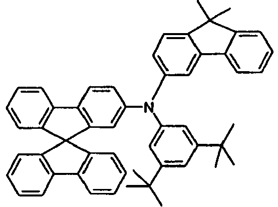 | 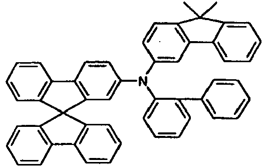 |
| (106) | (107) | (108) |
| 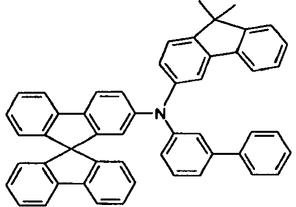 | 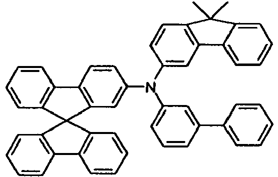 | 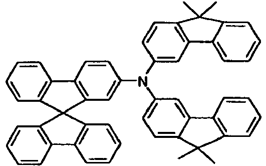 |
| (109) | (110) | (111) |
| 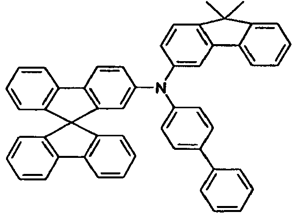 | 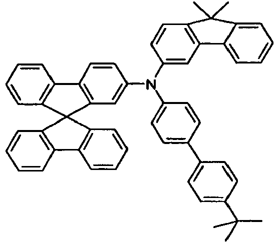 | 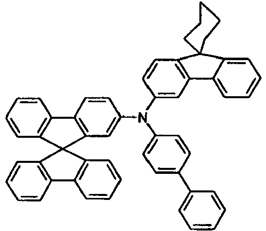 |
| (112) | (113) | (114) |
| 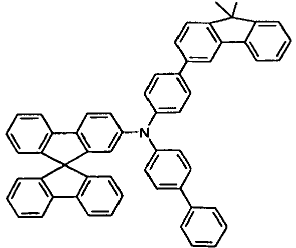 | 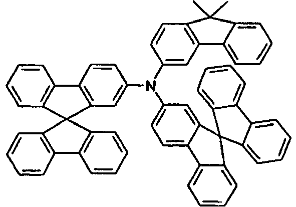 | 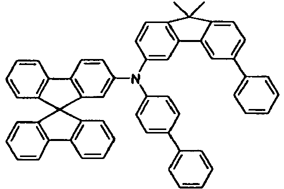 |
| (115) | (116) | (117) |
| 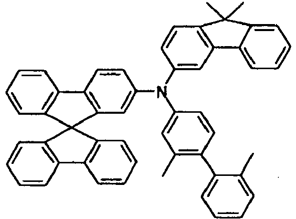 | 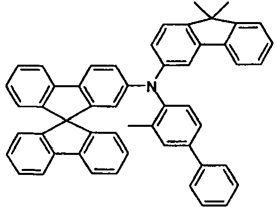 | 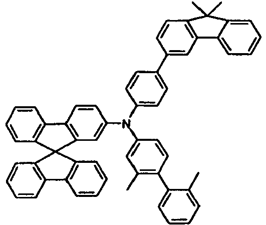 |
| (118) | (119) | (120) |
| 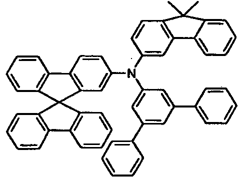 | 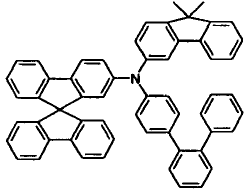 | 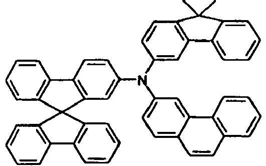 |
| (121) | (122) | (123) |
| 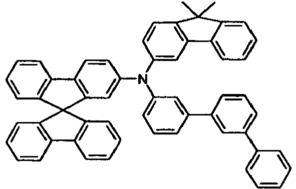 | 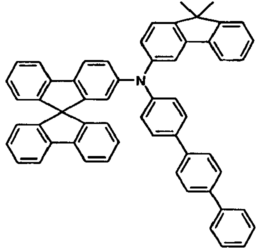 | 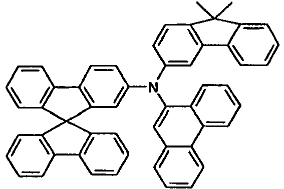 |
| (124) | (125) | (126) |
| 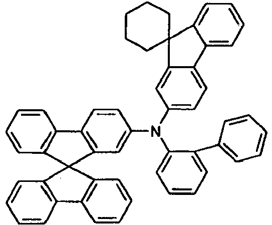 | 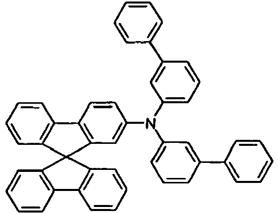 | 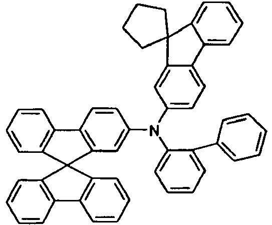 |
| (127) | (128) | (129) |
| 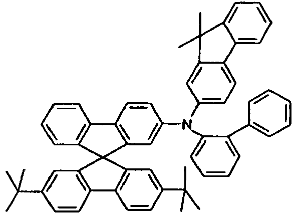 | 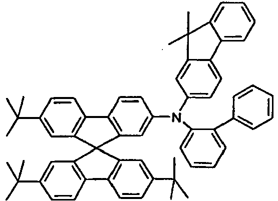 | 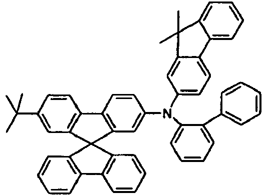 |
| (130) | (131) | (132) |
| 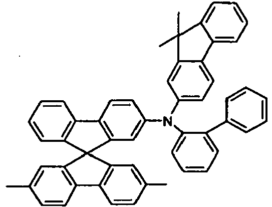 | 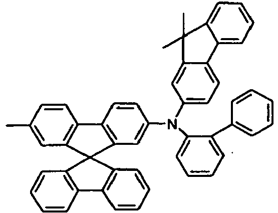 | 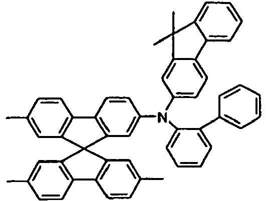 |
| (133) | (134) | (135) |
| 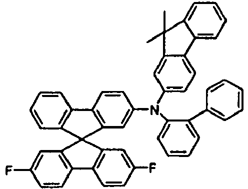 | 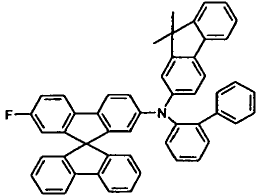 | 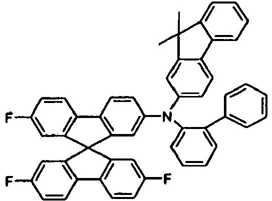 |
| (136) | (137) | (138) |
| 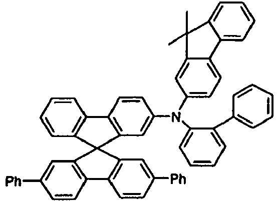 | 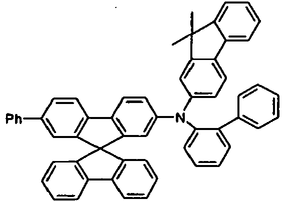 | 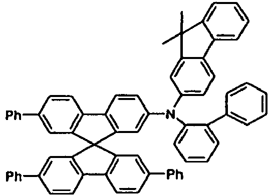 |
| (139) | (140) | (141) |
| 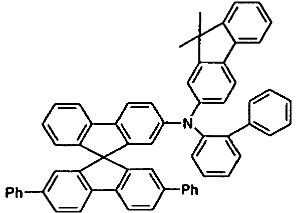 | 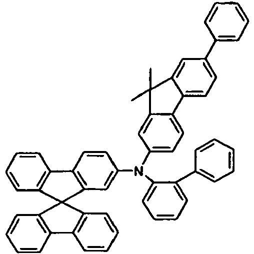 | 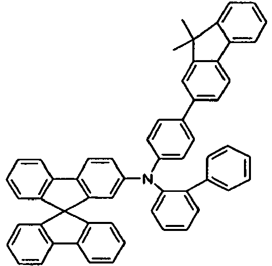 |
| (142) | (143) | (144) |
| 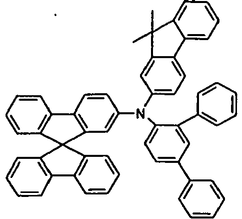 | 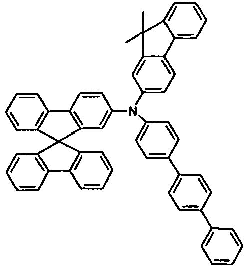 | 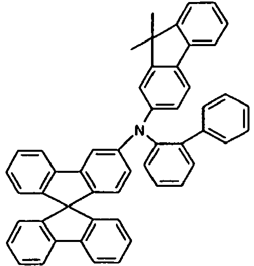 |
| (145) | (146) | (147) |
| 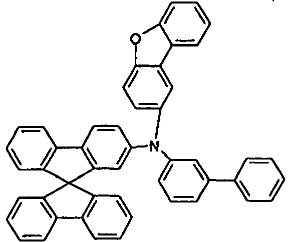 | 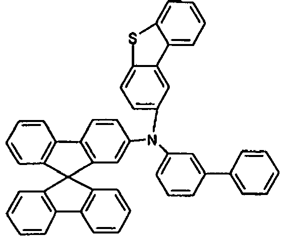 | 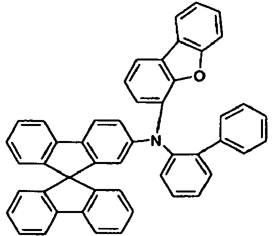 |
| (148) | (149) | (150) |
| 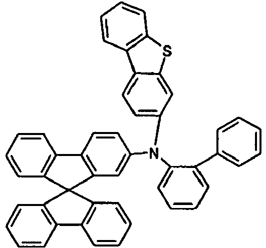 | 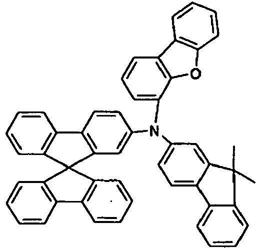 | 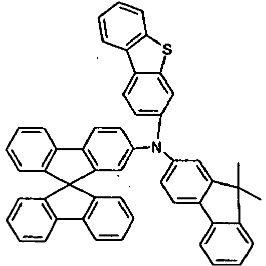 |
| (151) | (152) | (153) |
| 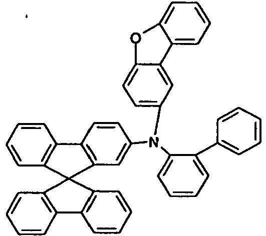 | 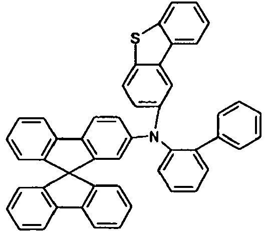 | 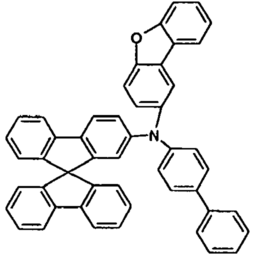 |
| (154) | (155) | (156) |
| 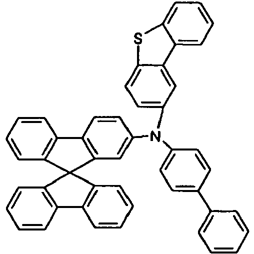 | 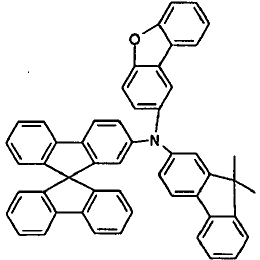 | 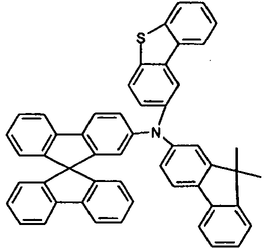 |
| (157) | (158) | (159) |
| 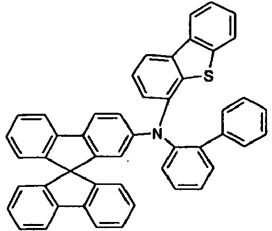 | 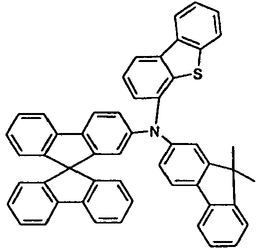 | 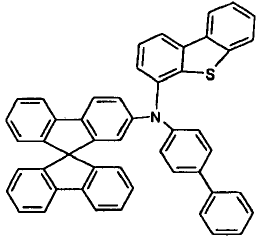 |
| (160) | (161) | (162) |
| 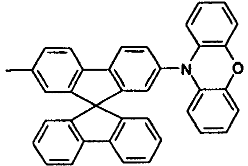 | 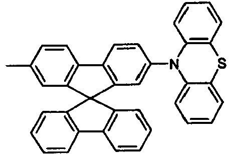 | 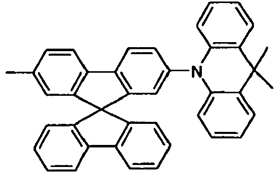 |
| (163) | (164) | (165) |
| 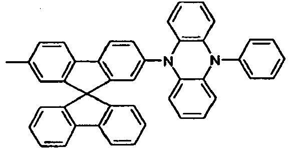 | 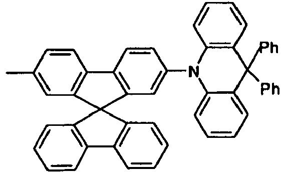 | 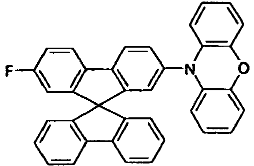 |
| (166) | (167) | (168) |
| 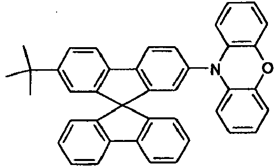 | 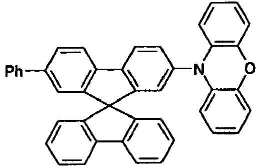 | 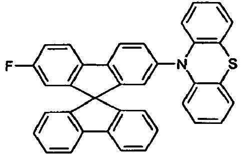 |
| (169) | (170) | (171) |
| 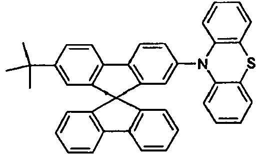 | 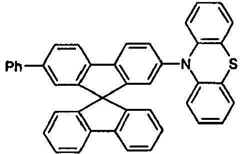 | 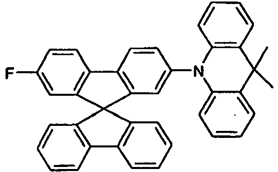 |
| (172) | (173) | (174) |
| 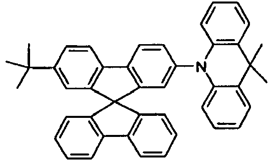 | 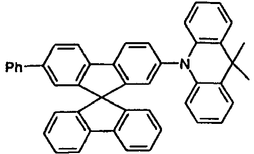 | 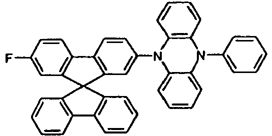 |
| (175) | (176) | (177) |
| 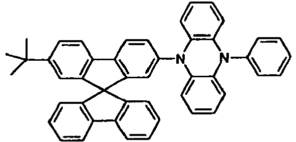 | 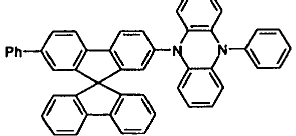 | 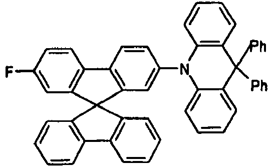 |
| (178) | (179) | (180) |
| 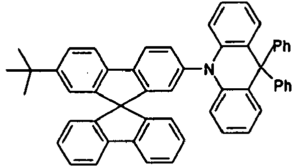 | 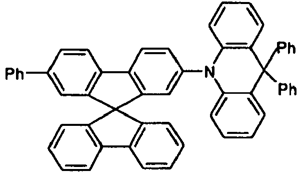 | 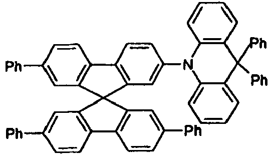 |
| (181) | (182) | (183) |
| 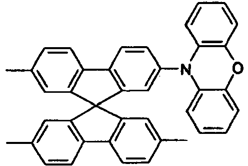 | 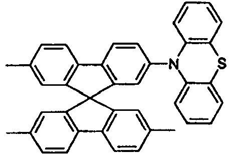 | 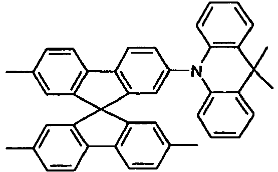 |
| (184) | (185) | (186) |
| 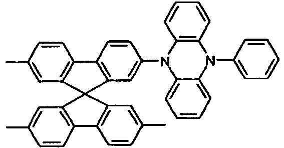 | 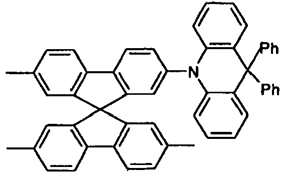 | 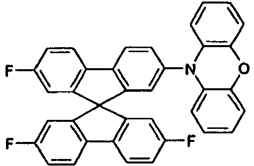 |
| (187) | (188) | (189) |
| 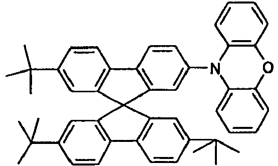 | 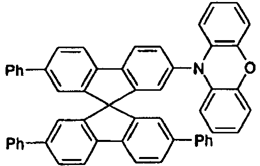 | 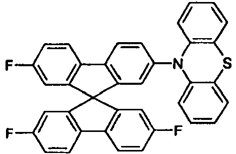 |
| (190) | (191) | (192) |
| 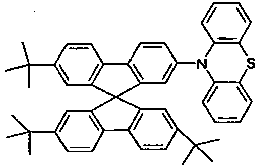 | 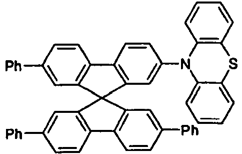 | 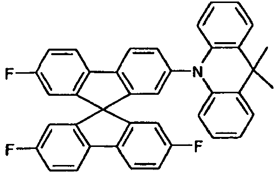 |
| (193) | (194) | (195) |
| 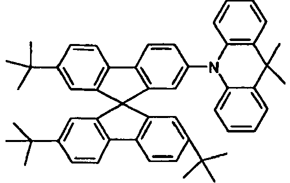 | 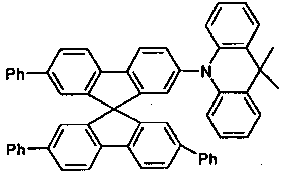 | 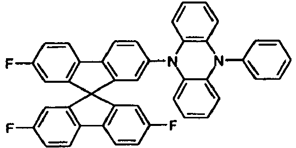 |
| (196) | (197) | (198) |
| 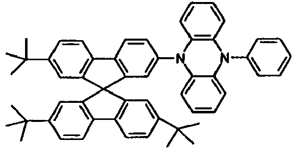 | 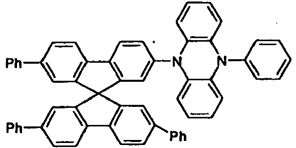 | 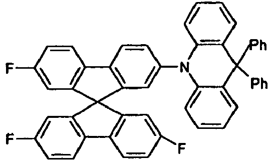 |
| (199) | (200) | (201) |
| 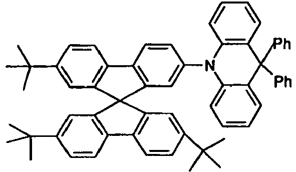 | | |
| (202) | | |

Die erfindungsgemäßen Verbindungen können nach dem Fachmann bekannten Syntheseschritten, wie z. B. Bromierung, Ullmann-Arylierung, Hartwig-Buchwald-Kupplung, etc., dargestellt werden. Insbesondere lassen sich die Verbindungen aus einem entsprechenden Halogen-substituierten Spirobifluoren durch Einführung der Aminogruppe synthetisieren, wie in Schema 1 dargestellt. Dabei ist es entweder möglich, zunächst ein primäres Amin mit einem Substituenten Ar¹ einzuführen und die Gruppe Ar² in einer weiteren Kupplungsreaktion einzuführen, wie in Schema 1 a) gezeigt. Ebenso ist es möglich, direkt in einem Schritt das sekundäre Amin Ar¹Ar²NH einzuführen, wie in Schema 1 b) gezeigt. Als Gruppe X am Spirobifluoren eignen sich reaktive Abgangsgruppen, wie beispielsweise Cl, Br, I, Triflat oder Tosylat. Als Kupplungsreaktionen eignen sich beispielsweise Kupplungsreaktionen nach Hartwig-Buchwald oder nach Ullmann. Die Reaktionsbedingungen, die für diese Kupplungsreaktionen verwendet werden können, sind dem Fachmann der organischen Synthese bekannt.

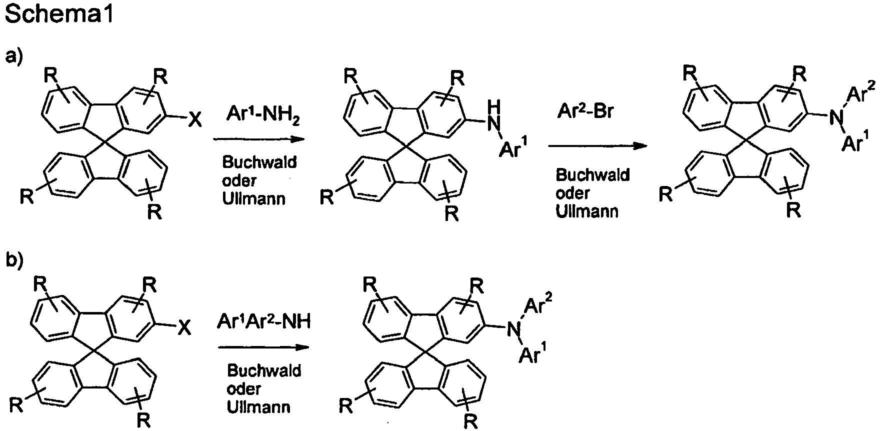

Für Verbindungen mit p = 1 oder 2 lässt sich die Gruppe Ar-NAr¹Ar² ebenfalls über eine metallkatalysierte Kupplungsreaktion einführen, beispielsweise über eine Suzuki-Kupplung oder eine Stille-Kupplung.

Ein weiterer Gegenstand der vorliegenden Erfindung ist daher ein Verfahren zur Herstellung einer Verbindung gemäß Formel (1) durch Kupplung eines Spirobifluorenderivats, welches in 2-Position mit einer reaktiven Abgangsgruppe substituiert ist, mit
a) einem primären Amin, gefolgt von der Kupplung mit einer weiteren aromatischen Gruppe, die mit einer reaktiven Abgangsgruppe substituiert ist, oder
b) mit sekundären Amin, oder
c) mit einem Triarylaminderivat.

Dabei ist die reaktive Abgangsgruppe bevorzugt ausgewählt aus Cl, Br, I, Triflat oder Tosylat oder für eine Suzuki-Kupplung auch eine Boronsäure bzw. ein Boronsäurederivat, insbesondere ein Boronsäureester.

Die Kupplungsreaktion ist bevorzugt ausgewählt aus Hartwig-Buchwald-Kupplungen, aus Ullmann-Kupplungen oder aus Suzuki-Kupplungen.

Die erfindungsgemäßen Verbindungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine erfindungsgemäße Verbindung. Dabei gelten die oben ausgeführten Bevorzugungen ebenso für die elektronischen Vorrichtungen.

Die elektronische Vorrichtung ist bevorzugt ausgewählt aus der Gruppe bestehend aus organischen Elektrolumineszenzvorrichtungen (organischen Leuchtdioden, OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (ODSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt aber organischen Elektrolumineszenzvorrichtungen (OLEDs), besonders bevorzugt phosphoreszierenden OLEDs.

Die organischen Elektrolumineszenzvorrichtungen und die lichtemittierenden elektrochemischen Zellen können für verschiedene Anwendungen eingesetzt werden, beispielsweise für einfarbige oder mehrfarbige Displays, für Beleuchtungsanwendungen oder für medizinische und/oder kosmetische Anwendungen, beispielsweise in der Phototherapie.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Dabei ist es möglich, dass alle emittierenden Schichten fluoreszierend sind oder dass alle emittierenden Schichten phosphoreszierend sind oder dass eine oder mehrere emittierende Schichten fluoreszierend und eine oder mehrere andere Schichten phosphoreszierend sind.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die bevorzugten Ausführungsformen als Lochtransportmaterial in einer Lochtransport- oder Lochinjektions- oder Exzitonenblockierschicht oder als Matrixmaterial für fluoreszierende oder phosphoreszierende Emitter, insbesondere für phosphoreszierende Emitter. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. die bevorzugten Ausführungsformen als Lochtransport- oder Lochinjektionsmaterial in einer Lochtransport- oder Lochinjektionsschicht eingesetzt. Dabei kann die emittierende Schicht fluoreszierend oder phosphoreszierend sein. Eine Lochinjektionsschicht im Sinne der vorliegenden Erfindung ist eine Schicht, die direkt an die Anode angrenzt. Eine Lochtransportschicht im Sinne der vorliegenden Erfindung ist eine Schicht, welche zwischen einer Lochinjektionsschicht und einer emittierenden Schicht vorliegt.

In nochmals einer weiteren bevorzugten Ausführungsform der Erfindung wird die Verbindung gemäß Formel (1) bzw. die bevorzugten Ausführungsformen in einer Exzitonenblockierschicht eingesetzt. Unter einer Exzitonenblockierschicht wird eine Schicht verstanden, die auf Anodenseite direkt an eine emittierende Schicht angrenzt.

Besonders bevorzugt ist der Einsatz der Verbindung gemäß Formel (1) bzw. der bevorzugten Ausführungsformen in einer Lochtransport- bzw. Exzitonenblockierschicht.

In einer weiteren bevorzugten Ausführungsform der Erfindung wird die Verbindung gemäß Formel (1) bzw. die bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. die bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand. Im Sinne dieser Anmeldung sollen alle lumineszierenden Komplexe mit Übergangsmetallen oder Lanthanoiden, insbesondere alle lumineszierenden Iridium-, Platin- und Kupferkomplexe als phosphoreszierende Verbindungen angesehen werden.

Die Mischung aus der Verbindung gemäß Formel (1) bzw. die bevorzugten Ausführungsformen und der emittierenden Verbindung enthält zwischen 99.9 und 1 Gew.-%, vorzugsweise zwischen 99 und 10 Gew.-%, besonders bevorzugt zwischen 97 und 60 Gew.-%, insbesondere zwischen 95 und 80 Gew.-% der Verbindung gemäß Formel (1) bzw. die bevorzugten Ausführungsformen bezogen auf die Gesamtmischung aus Emitter und Matrixmaterial. Entsprechend enthält die Mischung zwischen 0.1 und 99 Gew.-%, vorzugsweise zwischen 1 und 90 Gew.-%, besonders bevorzugt zwischen 3 und 40 Gew.-%, insbesondere zwischen 5 und 20 Gew.-% des Emitters bezogen auf die Gesamtmischung aus Emitter und Matrixmaterial. Die oben angegebenen Grenzen gelten insbesondere, wenn die Schicht aus Lösung aufgebracht wird. Wird die Schicht durch Vakuumverdampfung aufgebracht, gelten dieselben Zahlenwerte, wobei in diesem Fall der Prozenzanteil jeweils in Vol.-% angegeben ist.

Eine besonders bevorzugte Ausführungsform der vorliegenden Erfindung ist der Einsatz der Verbindung gemäß Formel (1) bzw. die bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. die bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazolderivate, z. B. gemäß EP 1617710, EP 1617711, EP 1731584, JP 2005/347160, bipolare Matrixmaterialien, z. B. gemäß 7/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 08/056746, Zinkkomplexe, z. B. gemäß EP 652273 oder WO 2009/062578, Fluorenderivate, z. B. gemäß WO 2009/124627, Diazasilol- bzw. Tetraazasilol-Derivate, z. B. gemäß WO 2010/054729, Diazaphosphol-Derivate, z. B. gemäß WO 2010/054730, oder verbrückte Carbazolderivate, z. B. gemäß US 2009/0136779, WO 2010/050778, WO 2011/042107 oder gemäß der nicht offen gelegten Anmeldung DE 102010005697.9. Weiterhin ist es möglich, einen elektronisch neutralen Co-Host zu verwenden, der weder lochtransportierende noch elektronentransportierende Eigenschaften aufweist, wie beispielsweise in WO 2010/108579 beschrieben.

Ebenso ist es möglich, zwei oder mehrere phosphoreszierende Emitter in der Mischung zu verwenden. Dabei wirkt der Emitter, welcher kürzerwellig emittiert, als Co-Host in der Mischung.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Beispiele der oben beschriebenen Emitter können den Anmeldungen WO 2000/70655, WO 2001/41512, WO 2002/02714, WO 2002/15645, EP 1191613, EP 1191612, EP 1191614, WO 2005/033244, WO 2005/019373, US 2005/0258742, WO 2009/146770, WO 2010/015307, WO 2010/031485, WO 2010/054731, WO 2010/054728, WO 2010/086089, WO 2010/099852 oder WO 2010/102709 entnommen werden. Weiterhin eignen sich beispielsweise die Komplexe gemäß den nicht offen gelegten Anmeldungen EP 10006208.2 und DE 102010027317.1. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Es ist weiterhin möglich, die Verbindung gemäß Formel (1) bzw. die bevorzugten Ausführungsformen sowohl in einer Lochtransportschicht bzw. Exzitonenblockierschicht als auch als Matrix in einer emittierenden Schicht zu verwenden.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. LITI (Light Induced Thermal Imaging, Thermotransferdruck), InkJet Druck (Tintenstrahldruck), Siebdruck, Flexodruck, Offsetdruck oder Nozzle Printing, hergestellt werden. Hierfür sind lösliche Verbindungen nötig, welche beispielsweise durch geeignete Substitution erhalten werden. Diese Verfahren eignen sich insbesondere auch für die erfindungsgemäßen Verbindungen, da diese allgemein eine sehr gute Löslichkeit in organischen Lösemitteln aufweisen.

Weiterhin sind Hybridverfahren möglich, bei denen beispielsweise eine oder mehrere Schichten aus Lösung aufgebracht werden und eine oder mehrere weitere Schichten aufgedampft werden. So kann beispielsweise die emittierende Schicht aus Lösung aufgebracht werden und die Elektronentransportschicht aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, Dispersion oder Miniemulsion, enthaltend mindestens eine Verbindung gemäß Formel (1) bzw. die oben ausgeführten bevorzugten Ausführungsformen und mindestens ein Lösemittel, insbesondere ein organisches Lösemittel. Wie solche Lösungen hergestellt werden können, ist dem Fachmann bekannt und beispielsweise in der WO 2002/072714, der WO 2003/019694 und der darin zitierten Literatur beschrieben.

Ein weiterer Gegenstand der vorliegenden Erfindung sind Mischungen enthaltend mindestens eine Verbindung gemäß Formel (1) bzw. die oben ausgeführten bevorzugten Ausführungsformen und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird. Die Mischung kann dann auch zusätzlich noch ein weiteres Material als zusätzliches Matrixmaterial enthalten.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochtransport- bzw. Lochinjektionsschicht in einer organischen Elektrolumineszenzvorrichtung. Dabei eignen sie sich insbesondere auch für die Verwendung in einer Schicht, die direkt an eine phosphoreszierende emittierende Schicht angrenzt, da die erfindungsgemäßen Verbindungen nicht die Lumineszenz löschen.
2. Die erfindungsgemäßen Verbindungen, eingesetzt als Matrixmaterial für fluoreszierende oder phosphoreszierende Emitter führen zu sehr hohen Effizienzen sowie zu langen Lebensdauern. Dies gilt insbesondere, wenn die Verbindungen als Matrixmaterial zusammen mit einem weiteren Matrixmaterial und einem phosphoreszierenden Emitter eingesetzt werden.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatz- und Betriebsspannungen.
4. Die erfindungsgemäßen Verbindungen weisen eine hohe thermische Stabilität auf und lassen sich unzersetzt und rückstandsfrei sublimieren.
5. Die erfindungsgemäßen Verbindungen weisen eine hohe Oxidationsstabilität auf, was sich insbesondere positiv auf die Handhabung dieser Verbindungen sowie auf die Lagerstabilität für Lösungen auswirkt.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR bezogen werden. Die Nummern in eckigen Klammern bei den literaturbekannten Edukten sind die entsprechenden CAS-Nummern.

### A1) Biphenyl-2-yl-(9,9'-Spirobi-9H-fluoren-2-yl)-amin

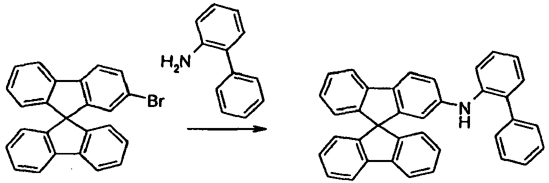

Eine Lösung von Biphenyl-2-ylamin (119.9 g, 709 mmol) und 2-Brom-9,9-spirobifluoren (280.3 g, 709 mmol) in entgastem Toluol (400 mL) wird mit 1,1'-Bis(diphenylphosphino)ferrocen (5.89 g, 10.6 mmol), Palladiumacetat (2.38 g, 10.6 mmol) und Natrium-*tert*-butanolat (88.6 g, 921 mmol) versetzt und 20 h unter Rückfluss erhitzt. Das Reaktionsgemisch wird auf Raumtemperatur abgekühlt, mit Toluol erweitert und über Celite filtriert. Das Filtrat wird mit Wasser erweitert, mit Toluol reextrahiert, die vereinigten organischen Phasen getrocknet und im Vakuum eingeengt. Der Rückstand wird über Kieselgel filtriert (Heptan/Dichlormethan) und aus Isopropanol kristallisiert. Das Produkt wird in Form eines hellgelben Feststoffs erhalten. Die Ausbeute beträgt 298 g (87 %).

Analog werden folgende Verbindungen erhalten:

| **Bsp.** | **Edukt 1** | **Edukt 2** | **Produkt** | **Ausbeute** |
|---|---|---|---|---|
| A2 | 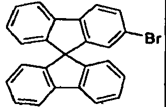 | 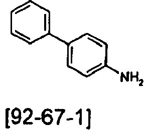 | 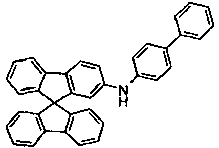 | 90% |
| A3 | 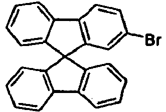 | 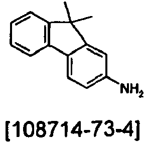 | 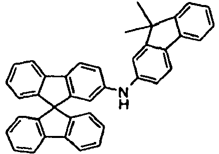 | 87 % |
| A4 | 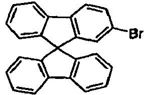 | 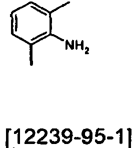 | 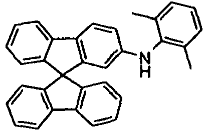 | 68 % |
| A5 | 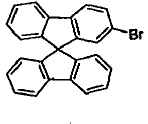 | 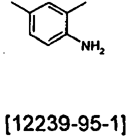 | 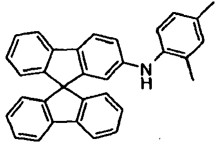 | 69% |
| A6 | 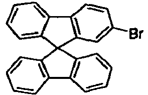 | 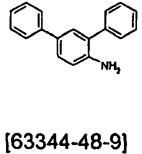 | 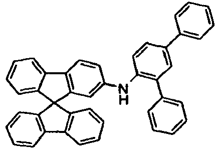 | 83 % |
| A7 | 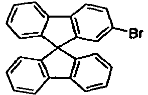 | 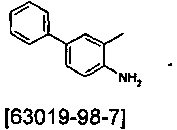 | 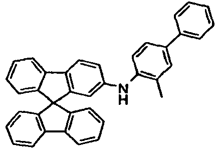 | 89 % |
| A8 | 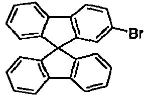 | 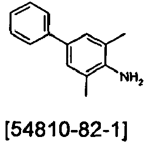 | 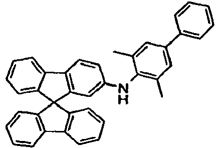 | 65% |
| A9 | 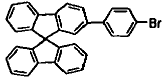 | 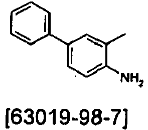 | 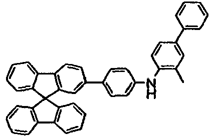 | 87% |
| A10 | 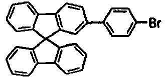 | 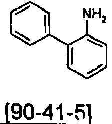 | 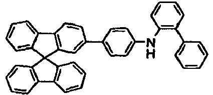 | 74% |
| A11 | 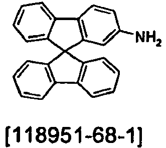 | 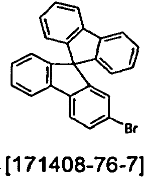 | 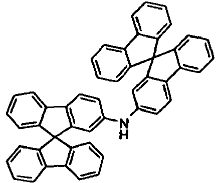 | 77% |

### B1) Biphenyl-4-yl-biphenyl-2-yl-(9,9-Spirobi[9H-fluoren-2-yl)-amin

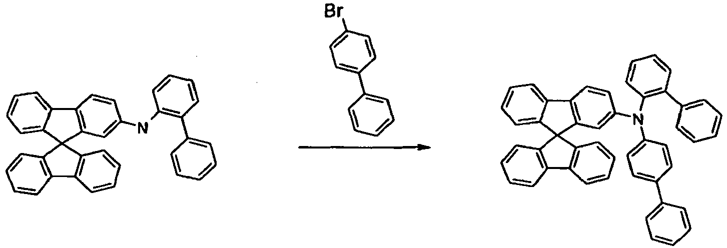

Eine Lösung von Biphenyl-2-yl-(9,9'-spirobi-9H-fluoren -2-yl)-amin (53.0 g, 110 mmol) und 4-Brom-biphenyl (32 g, 140 mmol) in entgastem Toluol (500 mL) wird mit Tri-*tert*-butylphosphin (4.4 mL einer 1.0 M Lösung in Toluol, 4.4 mmol), Palladiumacetat (248 mg, 1.1 mmol) und Natrium-*tert-*butanolat (16.0 g, 166 mmol) versetzt und 2 h unter Rückfluss erhitzt. Das Reaktionsgemisch wird auf Raumtemperatur abgekühlt, mit Toluol erweitert und über Celite filtriert. Das Filtrat wird im Vakuum eingeengt und der Rückstand aus Essigsäureethylester/Heptan kristallisiert. Das Rohprodukt wird soxhlettiert (Toluol) und durch zweimalige Zonensublimation im Vakuum (p = 3 x 10⁻⁴ mbar, T = 298 °C) gereinigt. Das Produkt wird in Form eines schwach gelben Feststoffs isoliert (60 g, 87% d. Th., Reinheit >99.99% laut HPLC).

Analog werden folgende Verbindungen erhalten:

| **Bsp.** | **Edukt 1** | **Edukt 2** | **Produkt** | **Ausbeute** |
|---|---|---|---|---|
| **B2** | 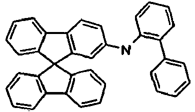 | 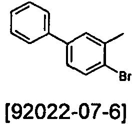 | 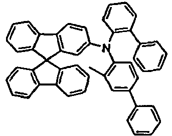 | 70% |
| **B3** | 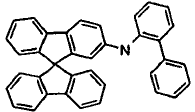 | 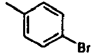 | 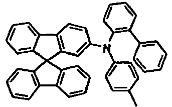 | 72% |
| **B4** | 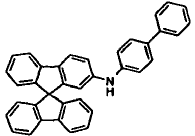 | 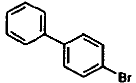 | 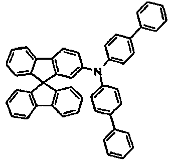 | 79% |
| **B5** | 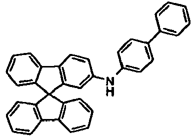 | 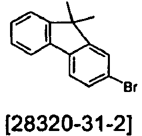 | 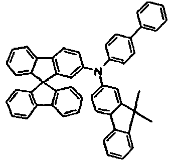 | 77% |
| **B6** | 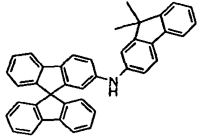 | 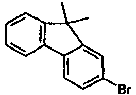 | 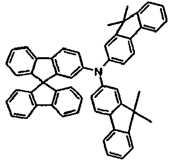 | 68 % |
| **B7** | 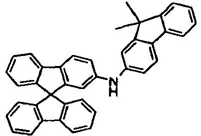 | 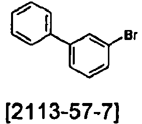 | 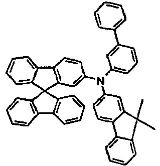 | 68 % |
| **B8** | 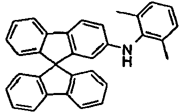 | 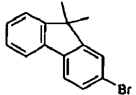 | 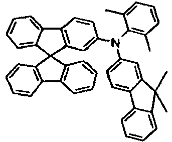 | 68% |
| **B9** | 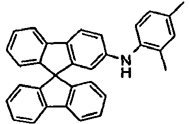 | 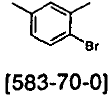 | 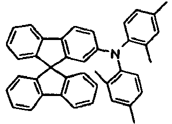 | 63% |
| **B10** | 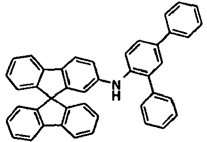 | 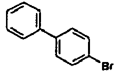 | 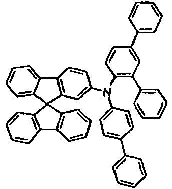 | 64% |
| **B11** | 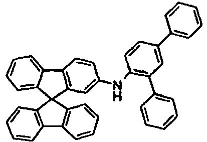 | 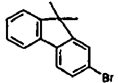 | 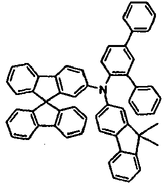 | 65 % |
| **B12** | 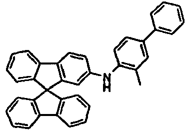 | 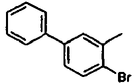 | 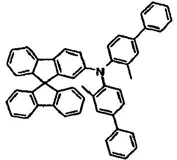 | 89 % |
| **B13** | 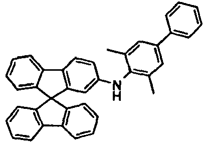 | 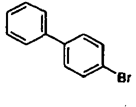 | 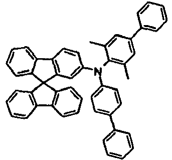 | 65% |
| **B14** | 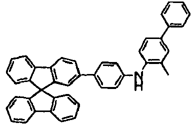 | 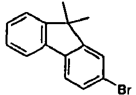 | 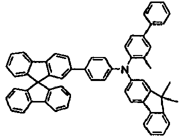 | 69% |
| **B15** | 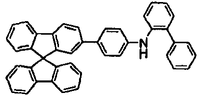 | 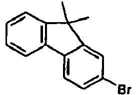 | 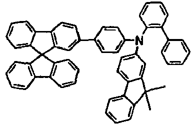 | 65% |
| **B16** | 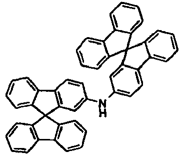 | 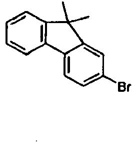 | 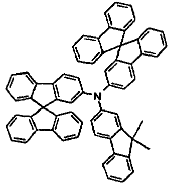 | 77% |
| **B17 Vergl.** | 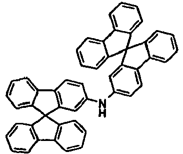 | 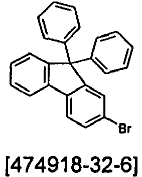 | 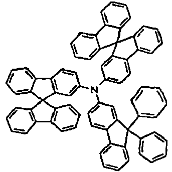 | 77% |
| **B18** | 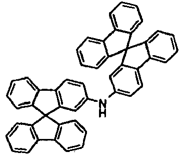 | 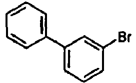 | 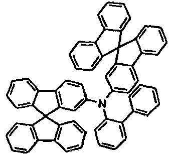 | 65% |
| **B19** | 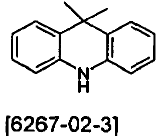 | 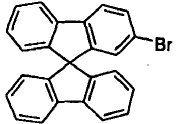 | 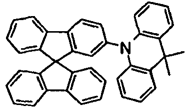 | 65% |
| **B20** | 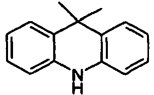 | 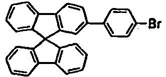 | 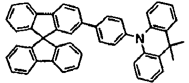 | 59% |
| **B21** | 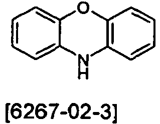 | 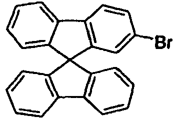 | 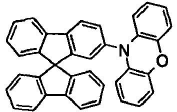 | 61% |
| **B22** | 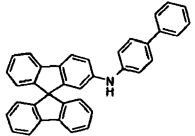 | 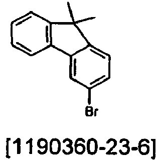 | 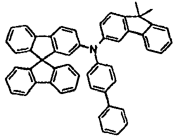 | 78 % |
| **B23** | 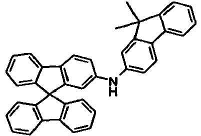 | 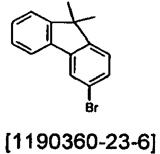 | 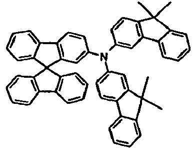 | 77% |
| **B24** | 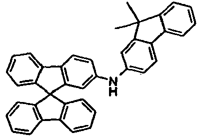 | 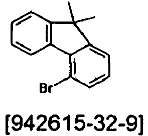 | 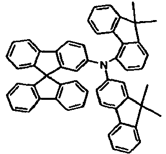 | 64% |
| **B25** | 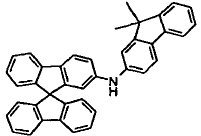 | 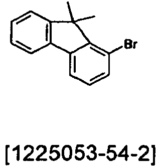 | 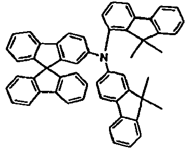 | 69% |
| **B26** | 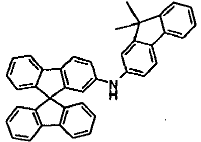 | 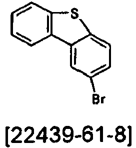 | 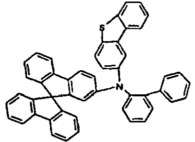 | 66% |
| **B27** | 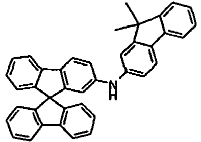 | 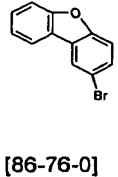 | 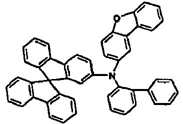 | 75% |

### Beispiel C: Synthese von Biphenyl-2-yl-(9,9-dimethyl-9H-fluoren-2-yl)-(9,9'-spirobifluoren-2-yl)-amin

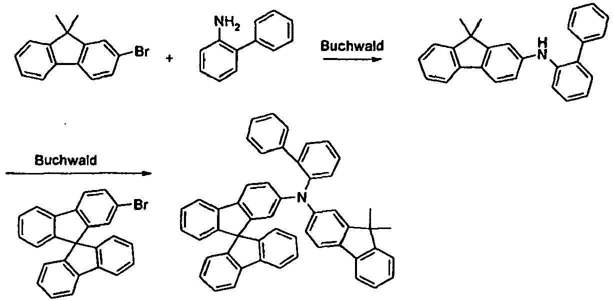

### a) Biphenyl-2-yl-(9,9-dimethyl-9H-fluoren-2-yl)-amin

Eine Lösung von Biphenyl-2-ylamin (31.0 g, 183 mmol) und 2-Brom-9,9-dimethyl-9H-fluoren (50.0 g, 183 mmol) in entgastem Toluol (400 mL) wird mit 1,1'-Bis(diphenylphosphino)ferrocen (1.5 g, 2.7 mmol), Palladiumacetat (616 mg, 2.7 mmol) und Natrium-*tert*-butanolat (22.9 g, 238 mmol) versetzt und 20 h unter Rückfluss erhitzt. Das Reaktionsgemisch wird auf Raumtemperatur abgekühlt, mit Toluol erweitert und über Celite filtriert. Das Filtrat wird mit Wasser erweitert, mit Toluol reextrahiert, die vereinigten organischen Phasen getrocknet und im Vakuum eingeengt. Der Rückstand wird über Kieselgel filtriert (Heptan/Dichlormethan) und aus Isopropanol kristallisiert. Biphenyl-2-yl-(9,9-dimethyl-9H-fluoren-2-yl)-amin wird in Form eines hellgelben Feststoffs erhalten (63.0 g, 95% d. Th.).

### b) Biphenyl-2-yl-(9,9-dimethyl-9H-fluoren-2-yl)-(9,9'-spirobifluoren-2-yl)-amin

Eine Lösung von Biphenyl-2-yl-(9,9-dimethyl-9*H*-fluoren-2-yl)-amin (40.0 g, 111 mmol) und 2-Brom-9,9'-spirobifluoren (56.9 g, 144 mmol) in entgastem Toluol (500 mL) wird mit Tri-*tert*-butylphosphin (4.4 mL einer 1.0 M Lösung in Toluol, 4.4 mmol), Palladiumacetat (248 mg, 1.1 mmol) und Natrium-*tert*-butanolat (16.0 g, 166 mmol) versetzt und 2 h unter Rückfluss erhitzt. Das Reaktionsgemisch wird auf Raumtemperatur abgekühlt, mit Toluol erweitert und über Celite filtriert. Das Filtrat wird im Vakuum eingeengt und der Rückstand aus Essigsäureethylester/Heptan kristallisiert. Das Rohprodukt wird soxhlettiert (Toluol) und durch zweimalige Zonensublimation im Vakuum (p = 3 x 10⁻⁴ mbar, T = 298 °C) gereinigt. Biphenyl-2-yl-(9,9-dimethyl-9*H*-fluoren-2-yl)-(9,9'-spirobifluoren-2-yl)-amin wird in Form eines schwach gelben Feststoffs isoliert (20.4 g, 27% d. Th., Reinheit >99.99% laut HPLC).

Analog können folgende Verbindungen erhalten werden:

| **Bsp.** | **Edukt 1** | | **Edukt 2** | **Produkt** | **Ausbeute** |
|---|---|---|---|---|---|
| C1 | 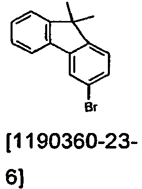 | 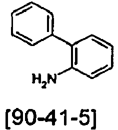 | 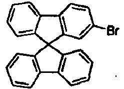 | 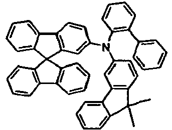 | 73% |
| C2 | 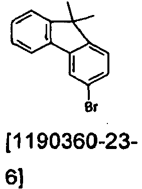 | 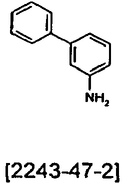 | 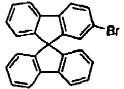 | 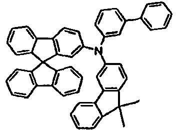 | 75% |
| C3 | 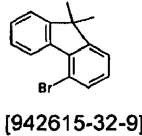 | 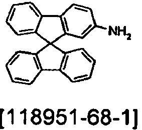 | 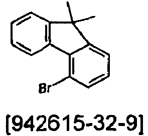 | 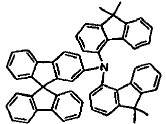 | 79% |
| C4 | 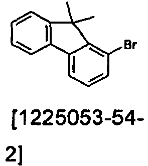 | 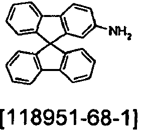 | 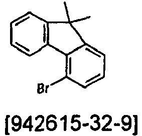 | 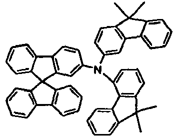 | 78% |
| **C5** | 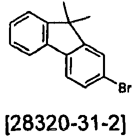 | 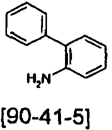 | 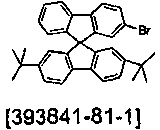 | 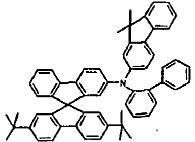 | 65% |
| **C6** | 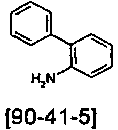 | 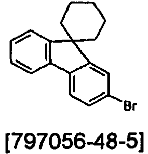 | 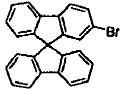 | 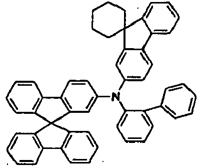 | 70% |
| **C7** | 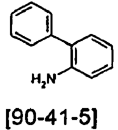 | 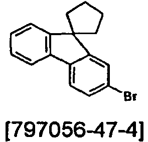 | 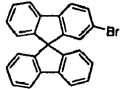 | 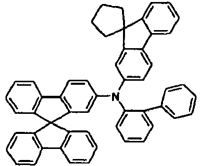 | 80% |

### Beispiel D: 2-(9,9'-Spirobi(9H-fluoren))-9,9-dimethyl-10-phenyl-9,10-dihydroacridin

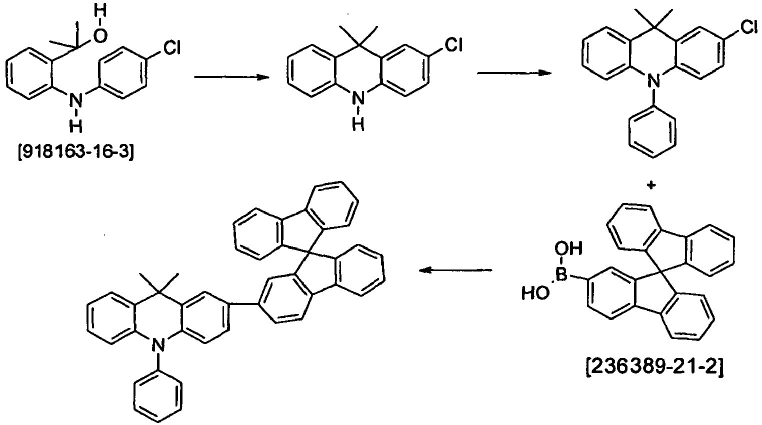

### a) Synthese von 2-Chlor-9,9-dimethyl-9,10-dihydroacridin

30.3 g (116 mmol) 2-[2-(4-Chlor-phenylamino)-phenyl]-propan-2-ol werden in 700 mL entgastem Toluol gelöst, mit einer Suspension aus 93 g Polyphosphorsäure und 61.7 g Methansulfonsäure versetzt, 1 h bei Raumtemperatur gerührt und 1 h auf 50 °C erhitzt. Der Ansatz wird abgekühlt, auf Eis gegeben und dreimal mit Essigsäureethylester extrahiert. Die vereinigten organischen Phasen werden mit gesättigter Natriumchloridlösung gewaschen, über Magnesiumsulfat getrocknet und eingeengt. Nach Filtration des Rohproduktes über Kieselgel mit Heptan/Essigsäureethylester (20:1) erhält man 25.1 g (89 %) 2-Chlor-9,9-dimethyl-9,10-dihydro-acridin als hellgelbe Kristalle.

### b) Synthese von 2-Chlor-9,9-dimethyl-10-phenyl-9,10-dihydroacridin

Eine entgaste Lösung von 16.6 mL (147 mmol) 4-lodbenzol und 30 g (123 mmol) 2-Chlor-9,9-dimethyl-9,10-dihydroacridin in 600 mL Toluol wird 1 h mit N₂ gesättigt. Danach wird die Lösung zuerst mit 2.09 mL (8.6 mmol) P(*t*Bu)₃, dann mit 1.38 g (6.1 mmol) Palladium(II)acetat versetzt und anschließend 17.7 g (185 mmol) NaO*t*Bu im festen Zustand zugegeben. Die Reaktionsmischung wird 1 h unter Rückfluss erhitzt. Nach Abkühlen auf Raumtemperatur werden vorsichtig 500 mL Wasser zugesetzt. Die wässrige Phase wird mit 3 x 50 mL Toluol gewaschen, über MgSO₄ getrocknet und das Lösungsmittel im Vakuum entfernt. Nach Filtration des Rohprodukts über Kieselgel mit Heptan/Essigsäureethylester (20:1) erhält man 32.2 g (81 %) 2-Chlor-9,9-dimethyl-10-phenyl-9,10-dihydroacridin als hellgelbe Kristalle.

### c) Synthese von 2-(9,9'-Spirobi(9H-fluoren))-9,9-dimethyl-10-phenyl-9,10-dihydroacridin

39.6 g (110 mmol) 9,9'-Spirobi[9H-fluoren]-2-yl-boronsäure, 35.2 g (110 mmol) 2-Chlor-9,9-dimethyl-10-phenyl-9,10-dihydroacridin und 9.7 g (92 mmol) Natriumcarbonat werden in 350 mL Toluol, 350 mL Dioxan und 500 mL Wasser suspendiert. Zu dieser Suspension werden 913 mg (3.0 mmol) Tri-o-tolylphosphin und 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus CH₂Cl₂/*iso-*Propanol umkristallisiert und abschließend im Hochvakuum sublimiert. Ausbeute: 52 g (100 mmol), 79 % d. Th., Reinheit nach HPLC 99.9 %.

### Beispiel E: Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1-E68, Sol1-3 (siehe Tabellen 1 und 2) werden die Daten verschiedener OLEDs vorgestellt. Glasplättchen, die mit strukturiertem ITO (Indium-Zinn-Oxid) der Dicke 150 nm beschichtet sind werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / optionale Lochinjektionsschicht (HIL) / Lochtransportschicht (HTL) / optionale Zwischenschicht 1 (IL1) / optionale Zwischenschicht 2 (IL2) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie Ket1:FTPh:TEG1 (60%:30%:10%) bedeutet hierbei, dass das Material Ket1 in einem Volumenanteil von 60%, FTPh in einem Anteil von 30% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) sowie die Lebensdauer bestimmt. Die Elektrolumineszenzspektrum werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich ist die externe Quanteneffizienz bei einer Betriebsleuchtdichte von 1000 cd/m². Als Lebensdauer LD wird die Zeit definiert, nach der die Leuchtdichte bei einem Betrieb mit konstantem Strom von der Startleuchtdichte L0 auf einen gewissen Anteil L1 abgesunken ist. Eine Angabe von L0 = 4000 cd/m² und L1 = 80% in Tabelle 2 bedeutet, dass die in Spalte LD angegebene Lebensdauer der Zeit entspricht, nach der die Anfangsleuchtdichte der entsprechenden OLED von 4000 cd/m² auf 3200 cd/m² abgesunken ist. Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/m² eine übliche Angabe.

Die Daten der verschiedenen OLEDs sind in Tabelle 2 zusammengefasst. Die Beispiele V1-V20 sind Vergleichsbeispiele gemäß dem Stand der Technik, die Beispiele E1-E68 und Sol1-3 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen deutliche Verbesserungen gegenüber dem Stand der Technik erzielt, teilweise in allen Parametern, in manchen Fällen ist aber nur eine Verbesserung von Effizienz oder Spannung oder Lebensdauer zu beobachten. Allerdings stellt bereits die Verbesserung eines der genannten Parameter einen signifikanten Fortschritt dar, weil verschiedene Anwendungen die Optimierung hinsichtlich unterschiedlicher Parameter erfordern.

### Verwendung von erfindungsgemäßen Verbindungen als Lochtransportmateriatien

Die OLEDs V1-V5 und V13-V20 sind Vergleichsbeispiele, welche die Lochtransportmaterialien BPA1, NPB, CbzA1 und SpA-tb gemäß dem Stand der Technik enthalten. Als erfindungsgemäße Materialien werden die Verbindungen B1-B8, B10-B21, B23-B25, C, C1-C2, C4-C7 sowie D eingesetzt (Beispiele E1, E3, E6-E12, E15-E20, E22, E24-E31, E33-E68).

Durch Einsatz erfindungsgemäßer Materialien erhält man vor allem signifikante Verbesserungen der Stromeffizienz sowie der Lebensdauer. Die Betriebsspannung bleibt im Vergleich zum Stand der Technik in etwa gleich, was aufgrund der verbesserten Stromeffizienz zu einer Verbesserung der Leistungseffizienz führt. So erhält man beispielsweise durch Einsatz der Verbindung B11 in einer OLED mit dem blau fluoreszierenden Dotanden D1 eine Steigerung der Leistungseffizienz um etwa 35% gegenüber NPB (Beispiele E17, V1). Die Lebensdauer lässt sich gegenüber NPB um fast 70% steigern, wenn die Verbindung C verwendet wird (Beispiele E30, V2).

Beim Einsatz in dickeren Schichten, die z.B. zur Optimierung der optischen Auskoppeleffizienz sowie zur Verbesserung der Produktionsausbeute eingesetzt werden können, zeigen die erfindungsgemäßen Materialien ebenfalls Vorteile: Während mit der Verbindung SpA-tb, die ein tert-Butyl substituiertes Spirobifluoren enthält, eine Erhöhung der Schichtdicke von 20 auf 70 nm zu einem Spannungsanstieg von 0.6 V führt, bleibt die Spannung bei Einsatz der Verbindung C fast unverändert (Beispiele V19, V20, E61, E62). Auch beim Einsatz der Verbindung C5, die nur ein substituiertes Spirobifluoren enthält, erhält man nur eine moderate Erhöhung der Spannung um 0.2 V. Das gleiche gilt für die Verbindung B16, die zwei unsubstituierte Spirobifluoren-Einheiten enthält (Beispiele E63-E66).

### Verwendung von erfindungsgemäßen Verbindungen als Matrixmaterialien in phosphoreszierenden OLEDs

Die erfindungsgemäßen Materialien lassen sich auch als Komponente in Mixed Matrix Systemen einsetzen. Bei einer Mixed Matrix wird eine erste Matrixkomponente mit einer zweiten Matrixkomponente und einem Dotanden gemischt, was gegenüber den Einzelmatrixmaterialien vor allem eine Verbesserung der Lebensdauer bietet. Hierbei ergibt sich gemäß dem Stand der Technik jedoch häufig eine Erhöhung der Betriebsspannung und eine Verringerung der Effizienz gegenüber Einzelmatrixmaterialien. Durch Einsatz der erfindungsgemäßen Verbindungen lassen sich hier Verbesserungen erzielen.

Gegenüber CBP erhält man durch Verwendung von B13 in Kombination mit ST1 z. B. eine Verbesserung der Spannung um 0.7 V, was sich in einer deutlichen Erhöhung der Leistungseffizienz um ca. 20% äußert (Beispiele E23, V7). Eine Verbesserung der Lebensdauer um etwa 50% ergibt sich durch Austausch von CBP gegen die Verbindung B2 in einer Mixed Matrix mit Ket1 als zweiter Komponente (Beispiele E5, V9). In Kombination mit DAP1 erhält man durch Einsatz der Verbindung B9 ebenfalls eine deutliche Verbesserung der Leistungseffizienz (Beispiele E14, V12**Fehlerl Verweisquelle konnte nicht gefunden werden.).** Dies zeigt, dass sich die erfindungsgemäßen Materialien gewinnbringend mit sehr unterschiedlichen Materialklassen in einer Mixed Matrix kombinieren lassen.

In Kombination mit dem roten Dotanden TER1 erhält man mit dem Material TSpA1 bereits gute Leistungsdaten (Beispiel V6). Verwendet man die Materialien B1 und B18 gemäß dem Stand der Technik, so lässt sich eine weitere Verbesserung erzielen (Beispiele E2, E32). Vor allem das Material B1, welches nur eine Spiroeinheit enthält, zeigt eine Verbesserung um etwa 30% in der Lebensdauer und 20% in der Leistungseffizienz gegenüber TSpA1 (Beispiele E2, V6).

### Verwendung von erfindungsgemäßen Verbindungen als lösungsprozessiertes HTM

Aus einer Formulierung von 10 mg/ml der Substanz C in Toluol werden 40 nm dicke Filme auf verschiedenen Substraten durch Aufschleudern hergestellt. Die Probe wird anschließend bei einer Temperatur von 180°C 10 min lang auf einer Heizplatte ausgeheizt. Anschließend wird die Probe in eine Vakuumverdampfungsanlage eingebracht und eine 30 nm dicke Emissionsschicht bestehend aus M2:D4(95%:5%) aufgedampft. Daran anschließend wird eine 20 nm dicke Elektronentransportschicht aus ST2:LiQ(50%:50%) und abschließend eine 100 nm dicke Kathode aus Aluminium aufgedampft.

**Beispiel Sol1:** Die beschriebene Schichtfolge wird auf folgendem Substrat aufgebracht: 50 nm dickes ITO auf Glas, auf welches eine 20 nm dicke PEDOT-Schicht aufgebracht ist. Die PEDOT Schicht wird wie weiter oben beschrieben aus Wasser aufgeschleudert.

**Beispiel Sol2:** Die beschriebene Schichtfolge wird auf folgendem Substrat aufgebracht: 50 nm dickes ITO auf Glas, auf welches eine 20 nm dicke HAT-CN Schicht aufgebracht ist. Die HAT-CN Schicht wird durch Verdampfen in einer Vakuumanlage aufgebracht.

**Beispiel Sol3:** Die Herstellung erfolgt wie bei Sol2, mit der Ausnahme dass anstatt ST2:LiQ das Material ETM2 als Elektronentransportschicht sowie eine 1.5 nm dicke LiF-Schicht als Elektroneninjektionsschicht verwendet wird.

Wie Tabelle 2 zu entnehmen ist, erhält man mit der aus Lösung prozessierten Substanz C gute bis sehr gute Daten, vor allem wenn das Material auf eine HAT-CN Schicht aufgebracht wird.

**Tabelle 1: Aufbau der OLEDs**

| Bsp. | HIL Dicke | HTL Dicke | IL1 Dicke | IL2 Dicke | EBL Dicke | EML Dicke | HBL Dicke | ETL Dicke | EIL Dicke |
|---|---|---|---|---|---|---|---|---|---|
| V1 | HATCN 5nm | SpA1 140nm | --- | --- | NPB 20nm | M1:D1 (95%:5%) 30nm | --- | Alq3 20nm | LiF 1nm |
| V2 | HATCN 5nm | SpA1 140nm | --- | --- | NPB 20nm | M1:D1 (95%:5%) 30nm | --- | ETM1:LiQ (50%:50%) 20nm | --- |
| V3 | HATCN 5nm | SpA1 110nm | --- | --- | NPB 20nm | M2:D2 (90%:10%) 30nm | --- | Alq₃ 20nm | LiF 1nm |
| V4 | HATCN 5nm | SpNPB 40nm | --- | --- | NPB 20nm | M2:D3 (98.5%:1.5%) 30nm | --- | ST2:LiQ (50%:50%) 20nm | --- |
| V5 | --- | SpA1 70nm | HATCN 5nm | --- | BPA1 90nm | Ket1:TEG1 (90%:10%) 30nm | --- | ST1:LiQ (50%:50%) 40nm | --- |
| V6 | --- | SpA1 20nm | --- | --- | NPB 20nm | Ket1:TSpA1:TER1 (65%:25%:10%) 30nm | Ket1 10nm | Alq₃ 20nm | LiF 1nm |
| V7 | --- | SpA1 70nm | HATCN 5nm | --- | BPA1 90nm | ST1:CBP:TEG1 (30%:60%:10%) 30nm | ST1 10nm | ST1:LiQ (50%:50%) 30nm | --- |
| V8 | --- | SpA1 70nm | HATCN 5nm | --- | BPA1 90nm | ST1:TCTA:TEG1 (30%:60%:10%) 30nm | --- | ST1:LiQ (50%:50%) 30nm | --- |
| V9 | HATCN 20nm | --- | --- | --- | BPA1 20nm | Ket1:FTPh:TEG1 (60%:30%:10%) 30nm | Ket1 10nm | ETM2 20nm | LiF 1nm |
| V10 | HATCN 20nm | --- | --- | --- | BPA1 20nm | Ket1:TCTA:TEG1 (60%:30%:10%) 30nm | Ket1 10nm | ETM2 20nm | LiF 1nm |
| V11 | HATCN 20nm | --- | --- | --- | BPA1 20nm | Ket1:CBP:TEG1 (60%:30%:10%) 30nm | Ket1 10nm | ETM2 20nm | LiF 1nm |
| V12 | --- | SpA1 70nm | HATCN 5nm | --- | BPA1 90nm | DAP1:CBP:TEG1 (30%:60%:10%) 30nm | --- | ST1:LiQ (50%:50%) 30nm | --- |
| V13 | --- | SpA1 70nm | HATCN 5nm | --- | BPA1 90nm | IC1:TEG1 (90%:10%) 30nm | ST1 10nm | ST1:LiQ (50%:50%) 30 nm | --- |
| V14 | HATCN 5nm | SpA1 140nm | HATCN 5nm | --- | BPA1 20nm | M2:D4 (95%:5%) 20nm | --- | ST1:LiQ (50%:50%) 30nm | LiQ 1nm |
| V15 | HATCN 5nm | SpA1 140nm | HATCN 5nm | --- | NPB 20nm | M2:D4 (95%:5%) 20nm | --- | ST1:LiQ (50%:50%) 30nm | LiQ 1nm |
| V16 | HATCN 5nm | TIFA1 140nm | HATCN 5nm | --- | CbzA1 20nm | M2:D4 (95%:5%) 20nm | --- | ST2:LiQ (50%:50%) 30nm | LiQ 1nm |
| V17 | HATCN 5nm | SpA1 140nm | HATCN 5nm | --- | CbzA1 20nm | IC1:TEG1 (90%:10%) 30nm | --- | ST2:LiQ (50%:50%) 40 nm | LiQ 1nm |
| V18 | HATCN 5nm | SpA1 140nm | HATCN 5nm | CbzA1 10nm | CbzA1 20nm | M2:D4 (95%:5%) 20nm | --- | ST2:LiQ (50%:50%) 30nm | LiQ 1nm |
| V19 | HATCN 5nm | SpA1 130nm | HATCN 5nm | --- | SpA-tb 20nm | IC1:TEG1 (90%:10%) 30nm | --- | ST2:LiQ (50%:50%) 40 nm | LiQ 1nm |
| V20 | HATCN 5nm | SpA1 80nm | HATCN 5nm | --- | SpA-tb 70nm | IC1:TEG1 (90%:10%) 30nm | --- | ST2:LiQ (50%:50%) 40 nm | LiQ 1nm |
| E1 | --- | SpA1 70nm | HATCN 5nm | --- | B1 90nm | IC1:TEG1 (90%:10%) 30nm | ST1 10nm | ST1:LiQ (50%:50%) 30 nm | --- |
| E2 | --- | SpA1 20nm | --- | --- | NPB 20nm | Ket1:B1:TER1 (65%:25%:10%) 30nm | Ket1 10nm | Alq₃ 20nm | LiF 1nm |
| E3 | HATCN 5nm | SpNPB 40nm | --- | --- | B2 20nm | M2:D3 (98.5%:1.5%) 30nm | --- | ST2:LiQ (50%:50%) 20nm | --- |
| E4 | --- | SpA1 70nm | HATCN 5nm | --- | BPA1 90nm | ST1:B2:TEG1 (30%:60%:10%) 30nm | ST1 10nm | ST1:LiQ (50%:50%) 30nm | --- |
| E5 | HATCN 20nm | --- | --- | --- | BPA1 20nm | Ket1:B2:TEG1 (60%:30%:10%) 30nm | Ket1 10nm | ETM2 20nm | LiF 1nm |
| E6 | HATCN 5nm | SpA1 140nm | --- | --- | B3 20nm | M1:D1 (95%:5%) 30nm | --- | ETM1:LiQ (50%:50%) 20nm | --- |
| E7 | HATCN 5nm | SpA1 110nm | --- | --- | B4 20nm | M2:D2 (90%:10%) 30nm | --- | Alq₃ 20nm | LiF 1nm |
| E8 | HATCN 5nm | SpA1 110nm | --- | --- | B5 20nm | M2:D2 (90%:10%) 30nm | --- | Alq₃ 20nm | LiF 1nm |
| E9 | HATCN 5nm | SpA1 110nm | --- | --- | B6 20nm | M2:D2 (90%:10%) 30nm | --- | Alq₃ 20nm | LiF 1nm |
| E10 | HATCN 5nm | SpA1 110nm | --- | --- | B7 20nm | M2:D2 (90%:10%) 30nm | --- | Alq₃ 20nm | LiF 1nm |
| E11 | HATCN 5nm | SpA1 140nm | --- | --- | B8 20nm | M1:D1 (95%:5%) 30nm | --- | ETM1:LiQ (50%:50%) 20nm | --- |
| E12 | --- | SpA1 70nm | HATCN 5nm | --- | B8 90nm | IC1:TEG1 (90%:10%) 30nm | ST1 10nm | ST1:LiQ (50%:50%) 30 nm | --- |
| E13 | --- | SpA1 70nm | HATCN 5nm | --- | BPA1 90nm | ST1:B9:TEG1 (30%:60%:10%) 30nm | ST1 10nm | ST1:LiQ (50%:50%) 30nm | --- |
| E14 | --- | SpA1 70nm | HATCN 5nm | --- | BPA1 90nm | DAP1:B9:TEG1 (30%:60%:10%) 30nm | --- | ST1:LiQ (50%:50%) 30nm | --- |
| E15 | HATCN 5nm | SpA1 140nm | --- | --- | B10 20nm | M1:D1 (95%:5%) 30nm | --- | ETM1:LiQ (50%:50%) 20nm | --- |
| E16 | --- | SpA1 70nm | HATCN 5nm | --- | B10 90nm | Ket1:TEG1 (90%:10%) 30nm | --- | ST1:LiQ (50%:50%) 40nm | --- |
| E17 | HATCN 5nm | SpA1 140nm | --- | --- | B11 20nm | M1:D1 (95%:5%) 30nm | --- | Alq3 20nm | LiF 1nm |
| E18 | HATCN 5nm | SpA1 110nm | --- | --- | B11 20nm | M2:D2 (90%:10%) 30nm | --- | Alq₃ 20nm | LiF 1nm |
| E19 | --- | SpA1 70nm | HATCN 5nm | --- | B11 90nm | IC1:TEG1 (90%:10%) 30nm | ST1 10nm | ST1:LiQ (50%:50%) 30 nm | --- |
| E20 | HATCN 5nm | SpNPB 40nm | --- | --- | B12 20nm | M2:D3 (98.5%:1.5%) 30nm | --- | ST2:LiQ (50%:50%) 20nm | --- |
| E21 | --- | SpA1 70nm | HATCN 5nm | --- | BPA1 90nm | ST1:B12:TEG1 (30%:60%:10%) 30nm | ST1 10nm | ST1:LiQ (50%:50%) 30nm | --- |
| E22 | HATCN 5nm | SpNPB 40nm | --- | --- | B13 20nm | M2:D3 (98.5%:1.5%) 30nm | --- | ST2:LiQ (50%:50%) 20nm | --- |
| E23 | --- | SpA1 70nm | HATCN 5nm | --- | BPA1 90nm | ST1:B13:TEG1 (30%:60%:10%) 30nm | ST1 10nm | ST1:LiQ (50%:50%) 30nm | --- |
| E24 | HATCN 5nm | SpA1 140nm | --- | --- | B14 20nm | M1:D1 (95%:5%) 30nm | --- | Alq3 20nm | LiF 1nm |
| E25 | HATCN 5nm | SpA1 110nm | --- | --- | B14 20nm | M2:D2 (90%:10%) 30nm | - | Alq₃ 20nm | LiF 1nm |
| E26 | HATCN 5nm | SpA1 140nm | --- | --- | B15 20nm | M1:D1 (95%:5%) 30nm | --- | Alq3 20nm | LiF 1nm |
| E27 | HATCN 5nm | SpA1 140nm | --- | --- | B15 20nm | M1:D1 (95%:5%) 30nm | --- | ETM1:LiQ (50%:50%) 20nm | --- |
| E28 | HATCN 5nm | SpA1 140nm | --- | --- | B16 20nm | M1:D1 (95%:5%) 30nm | --- | ETM1:LiQ (50%:50%) 20nm | --- |
| E29 | HATCN 5nm | SpA1 140nm | --- | --- | B17 20nm | M1:D1 (95%:5%) 30nm | --- | ETM1:LiQ (50%:50%) 20nm | --- |
| E30 | HATCN 5nm | SpA1 140nm | --- | --- | B18 20nm | M1:D1 (95%:5%) 30nm | --- | ETM1:LiQ (50%:50%) 20nm | --- |
| E31 | HATCN 5nm | SpNPB 40nm | --- | --- | B18 20nm | M2:D3 (98.5%:1.5%) 30nm | --- | ST2:LiQ (50%:50%) 20nm | --- |
| E32 | --- | SpA1 20nm | --- | --- | NPB 20nm | Ket1:B18:TER1 (65%:25%:10%) 30nm | Ket1 10nm | Alq3 20nm | LiF 1nm |
| E33 | --- | SpA1 70nm | HATCN 5nm | --- | B19 90nm | IC1:TEG1 (90%:10%) 30nm | ST1 10nm | ST1:LiQ (50%: 50%) 30 nm | --- |
| E34 | --- | SpA1 70nm | HATCN 5nm | --- | B20 90nm | IC1:TEG1 (90%:10%) 30nm | ST1 10nm | ST1:LiQ (50%:50%) 30 nm | --- |
| E35 | --- | SpA1 70nm | HATCN 5nm | --- | B21 90nm | IC1:TEG1 (90%:10%) 30nm | ST1 10nm | ST1:LiQ (50%:50%) 30 nm | --- |
| E36 | HATCN | SpA1 | --- | --- | C | M1:D1 (95%:5%) | --- | Alq3 | LiF |
| | 5nm | 140nm | | | 20nm | 30nm | | 20nm | 1nm |
| E37 | HATCN 5nm | SpA1 140nm | - | - | C 20nm | M1:D1 (95%:5%) 30nm | --- | ETM1:LiQ (50%:50%) 20nm | --- |
| E38 | HATCN 5nm | SpA1 110nm | --- | --- | C 20nm | M2:D2 (90%:10%) 30nm | --- | Alq₃ 20nm | LiF 1 nm |
| E39 | --- | SpA1 70nm | HATCN 5nm | --- | C 90nm | IC1:TEG1 (90%:10%) 30nm | ST1 10nm | ST1:LiQ (50%:50%) 30 nm | --- |
| E40 | HATCN 5nm | SpA1 140nm | HATCN 5nm | --- | C 20nm | M2:D4 (95%:5%) 20nm | --- | ST2:LiQ (50%:50%) 30nm | LiQ 1nm |
| E41 | HATCN 5nm | SpA1 140nm | HATCN 5nm | --- | C 20nm | M2:D4 (95%:5%) 20nm | --- | ST1:LiQ (50%:50%) 30nm | LiQ 1nm |
| E42 | HATCN 5nm | TIFA1 140nm | HATCN 5nm | --- | B1 20nm | M2:D4 (95%:5%) 20nm | --- | ST2:LiQ (50%:50%) 30nm | LiQ 1nm |
| E43 | HATCN 5nm | TIFA1 140nm | HATCN 5nm | --- | C 20nm | M2:D4 (95%:5%) 20nm | --- | ST2:LiQ (50%:50%) 30nm | LiQ 1nm |
| E44 | HATCN 5nm | SpA1 125nm | HATCN 5nm | C 10nm | B18 20nm | M2:D4 (95%:5%) 20nm | --- | ST2:LiQ (50%:50%) 30nm | LiQ 1nm |
| E45 | HATCN 5nm | SpA1 125nm | HATCN 5nm | C 10nm | D 20nm | M2:D4 (95%:5%) 20nm | --- | ST2:LiQ (50%:50%) 30nm | LiQ 1nm |
| E46 | HATCN 5nm | SpA1 125nm | HATCN 5nm | C 10nm | B19 20nm | M2:D4 (95%:5%) 20nm | --- | ST2:LiQ (50%:50%) 30nm | LiQ 1nm |
| E47 | HATCN 5nm | SpA1 140nm | HATCN 5nm | --- | C5 20nm | M2:D4 (95%:5%) 20nm | --- | ST2:LiQ (50%:50%) 30nm | LiQ 1nm |
| E48 | HATCN 5nm | SpA1 140nm | HATCN 5nm | CbzA1 10nm | C6 20nm | M2:D4 (95%:5%) 20nm | --- | ST2:LiQ (50%:50%) 30nm | LiQ 1nm |
| E49 | HATCN 5nm | SpA1 140nm | HATCN 5nm | --- | C6 20nm | IC1:TEG1 (90%:10%) 30nm | --- | ST2:LiQ (50%:50%) 40 nm | LiQ 1nm |
| E50 | HATCN 5nm | SpA1 140nm | HATCN 5nm | --- | C7 20nm | IC1:TEG1 (90%:10%) 30nm | --- | ST2:LiQ (50%:50%) 40 nm | LiQ 1nm |
| E51 | HATCN 5nm | SpA1 140nm | HATCN 5nm | --- | B22 20nm | IC1:TEG1 (90%:10%) 30nm | --- | ST2:LiQ (50%:50%) 40 nm | LiQ 1 nm |
| E52 | HATCN 5nm | TIFA1 140nm | HATCN 5nm | --- | C1 20nm | M2:D4 (95%:5%) 20nm | --- | ST2:LiQ (50%:50%) 30nm | LiQ 1nm |
| E53 | HATCN 5nm | SpA1 140nm | HATCN 5nm | --- | B23 20nm | IC1:TEG1 (90%:10%) 30nm | --- | ST2:LiQ (50%:50%) 40 nm | LiQ 1nm |
| E54 | --- | SpA1 70nm | HATCN 5nm | --- | B24 90nm | IC1:TEG1 (90%:10%) 30nm | ST1 10nm | ST1:LiQ (50%:50%) 30 nm | --- |
| E55 | HATCN 5nm | SpA1 140nm | HATCN 5nm | --- | B24 20nm | M2:D4 (95%:5%) 20nm | --- | ST2:LiQ (50%:50%) 30nm | LiQ 1nm |
| E56 | HATCN 5nm | SpA1 140nm | HATCN 5nm | --- | B25 20nm | M2:D4 (95%:5%) 20nm | --- | ST2:LiQ (50%:50%) 30nm | LiQ 1nm |
| E57 | ---- | SpA1 70nm | HATCN 5nm | --- | C2 90nm | IC1:TEG1 (90%:10%) 30nm | ST1 10nm | ST1:LiQ (50%:50%) 30 nm | --- |
| E58 | --- | SpA1 70nm | HATCN 5nm | --- | C4 90nm | IC1:TEG1 (90%:10%) 30nm | ST1 10nm | ST1:LiQ (50%:50%) 30 nm | --- |
| E59 | HATCN 5nm | SpA1 140nm | HATCN 5nm | CbzA1 10nm | B26 20nm | M2:D4 (95%:5%) 20nm | --- | ST2:LiQ (50%:50%) 30nm | LiQ 1nm |
| E60 | HATCN 5nm | SpA1 140nm | HATCN 5nm | CbzA1 10nm | B27 20nm | M2:D4 (95%:5%) 20nm | --- | ST2:LiQ (50%:50%) 30nm | LiQ 1nm |
| E61 | HATCN 5nm | SpA1 130nm | HATCN 5nm | --- | C 20nm | IC1:TEG1 (90%:10%) 30nm | --- | ST2:LiQ (50%:50%) 40 nm | LiQ 1nm |
| E62 | HATCN 5nm | SpA1 80nm | HATCN 5nm | --- | C 70nm | IC1:TEG1 (90%:10%) 30nm | --- | ST2:LiQ (50%:50%) 40 nm | LiQ 1nm |
| E63 | HATCN 5nm | SpA1 130nm | HATCN 5nm | --- | C5 20nm | IC1:TEG1 (90%:10%) 30nm | --- | ST2:LiQ (50%:50%) 40 nm | LiQ 1nm |
| E64 | HATCN 5nm | SpA1 80nm | HATCN 5nm | --- | C5 70nm | IC1:TEG1 (90%:10%) 30nm | --- | ST2:LiQ (50%:50%) 40 nm | LiQ 1nm |
| E65 | HATCN 5nm | SpA1 130nm | HATCN 5nm | --- | B16 20nm | IC1:TEG1 (90%:10%) 30nm | --- | ST2:LiQ (50%:50%) 40 nm | LiQ 1nm |
| E66 | HATCN 5nm | SpA1 80nm | HATCN 5nm | --- | B16 70nm | IC1:TEG1 (90%:10%) 30nm | --- | ST2:LiQ (50%:50%) 40 nm | LiQ 1nm |
| E67 | HATCN 5nm | SpA1 140nm | HATCN 5nm | --- | B18 20nm | M2:D4 (95%:5%) 20nm | --- | ST2:LiQ (50%:50%) 30nm | LiQ 1nm |
| E68 | --- | B6 70nm | HATCN 5nm | --- | BPA1 90nm | IC1:TEG1 (90%:10%) 30nm | ST1 10nm | ST1:LiQ (50%:50%) 30 nm | --- |

**Tabelle 2: Daten der OLEDs**

| Bsp. | U1000 (V) | SE1000 (cd/A) | LE1000 (lm/W) | EQE 1000 | CIE x/y bei 1000 cd/m² | L0 (cd/m²) | L1 % | LD (h) |
|---|---|---|---|---|---|---|---|---|
| V1 | 6.4 | 5.1 | 2.5 | 4.2% | 0.14/0.15 | 6000 | 50 | 150 |
| V2 | 4.7 | 8.1 | 5.4 | 6.3% | 0.14/0.16 | 6000 | 50 | 145 |
| V3 | 5.0 | 17 | 11 | 5.0% | 0.28/0.61 | 25000 | 50 | 480 |
| V4 | 4.3 | 9.8 | 7.1 | 7.6% | 0.141/0.160 | 6000 | 50 | 210 |
| V5 | 3.9 | 41 | 33 | 11.0% | 0.36/0.61 | 4000 | 80 | 315 |
| V6 | 4.3 | 7.7 | 5.6 | 10.8% | 0.68/0.32 | 4000 | 80 | 360 |
| V7 | 4.4 | 48 | 34 | 13.3% | 0.37/0.60 | 4000 | 80 | 450 |
| V8 | 4.2 | 43 | 32 | 12.0% | 0.35/0.60 | 4000 | 80 | 195 |
| V9 | 4.0 | 46 | 36 | 12.8% | 0.36/0.61 | 4000 | 80 | 370 |
| V10 | 3.9 | 42 | 34 | 11.6% | 0.35/0.60 | 4000 | 80 | 175 |
| V11 | 4.1 | 44 | 34 | 12.3% | 0.36/0.61 | 4000 | 80 | 280 |
| V12Fe hlerl Verwel squelle konnte nicht gefund en werde n. | 4.6 | 47 | 32 | 13.2% | 0.36/0.60 | 4000 | 80 | 490 |
| V13 | 3.5 | 53 | 48 | 14.8% | 0.36/0.60 | 4000 | 80 | 430 |
| V14 | 4.2 | 7.0 | 9.5 | 7.4% | 0.14/0.16 | 6000 | 50 | 250 |
| V15 | 4.2 | 8.2 | 6.3 | 6.5% | 0.14/0.16 | 6000 | 50 | 315 |
| V16 | 4.4 | 7.6 | 5.4 | 5.4% | 0.14/0.18 | 6000 | 65 | 240 |
| V17 | 3.5 | 59 | 53 | 16.4% | 0.37/0.60 | 10000 | 70 | 235 |
| V18 | 4.3 | 8.4 | 6.1 | 6.5% | 0.14/0.16 | 6000 | 50 | 320 |
| V19 | 3.5 | 59 | 53 | 16.3% | 0.37/0.60 | 10000 | 65 | 310 |
| V20 | 4.1 | 57 | 43 | 15.9% | 0.37/0.60 | 10000 | 65 | 210 |
| E1 | 3.7 | 59 | 50 | 16.4% | 0.36/0.60 | 4000 | 80 | 490 |
| E2 | 4.2 | 9.0 | 6.7 | 12.8% | 0.68/0.32 | 4000 | 80 | 460 |
| E3 | 4.4 | 10.4 | 7.4 | 8.1% | 0.14/0.16 | 6000 | 50 | 260 |
| E4 | 4.0 | 51 | 40 | 14.2% | 0.37/0.61 | 4000 | 80 | 620 |
| E5 | 3.7 | 50 | 43 | 14.0% | 0.37/0.61 | 4000 | 80 | 550 |
| E6 | 4.6 | 9.1 | 6.3 | 7.1% | 0.14/0.16 | 6000 | 50 | 130 |
| E7 | 5.1 | 19 | 12 | 5.4% | 0.28/0.61 | 25000 | 50 | 460 |
| E8 | 5.1 | 18 | 11 | 5.2% | 0.28/0.61 | 25000 | 50 | 520 |
| E9 | 4.8 | 20 | 13 | 5.8% | 0.28/0.61 | 25000 | 50 | 530 |
| E10 | 5.0 | 18 | 11 | 5.3% | 0.28/0.61 | 25000 | 50 | 430 |
| E11 | 4.7 | 8.7 | 5.8 | 6.8% | 0.14/0.15 | 6000 | 50 | 160 |
| E12 | 3.4 | 58 | 54 | 16.1% | 0.36/0.60 | 4000 | 80 | 420 |
| E13 | 4.1 | 50 | 38 | 13.9% | 0.37/0.61 | 4000 | 80 | 470 |
| E14 | 3.9 | 52 | 41 | 14.4% | 0.37/0.61 | 4000 | 80 | 460 |
| E15 | 4.8 | 9.2 | 6.1 | 7.2% | 0.14/0.15 | 6000 | 50 | 230 |
| E16 | 4.0 | 46 | 36 | 12.8% | 0.37/0.60 | 4000 | 80 | 470 |
| E17 | 6.1 | 6.7 | 3.4 | 5.5% | 0.14/0.15 | 6000 | 50 | 230 |
| E18 | 5.2 | 20 | 12 | 5.7% | 0.28/0.61 | 25000 | 50 | 620 |
| E19 | 3.5 | 56 | 50 | 15.6% | 0.36/0.60 | 4000 | 80 | 550 |
| E20 | 4.5 | 11 | 7.6 | 8.4% | 0.14/0.16 | 6000 | 50 | 290 |
| E21 | 3.8 | 47 | 40 | 13.1% | 0.36/0.62 | 4000 | 80 | 440 |
| E22 | 4.5 | 11 | 7.3 | 8.2% | 0.14/0.16 | 6000 | 50 | 270 |
| E23 | 3.7 | 49 | 41 | 13.6% | 0.36/0.62 | 4000 | 80 | 470 |
| E24 | 6.2 | 6.2 | 3.1 | 5.1% | 0.14/0.15 | 6000 | 50 | 175 |
| E25 | 5.0 | 19 | 12 | 5.7% | 0.28/0.61 | 25000 | 50 | 510 |
| E26 | 6.3 | 6.4 | 3.2 | 5.3% | 0.14/0.15 | 6000 | 50 | 190 |
| E27 | 4.8 | 9.5 | 6.2 | 7.4% | 0.14/0.15 | 6000 | 50 | 175 |
| E28 | 4.5 | 8.7 | 6.1 | 6.8% | 0.14/0.15 | 6000 | 50 | 180 |
| E29 | 4.6 | 7.9 | 5.4 | 6.2% | 0.14/0.16 | 6000 | 50 | 165 |
| E30 | 4.8 | 9.5 | 6.3 | 7.4% | 0.14/0.16 | 6000 | 50 | 245 |
| E31 | 4.4 | 11 | 7.5 | 8.2% | 0.14/0.160 | 6000 | 50 | 280 |
| E32 | 4.4 | 8.2 | 5.9 | 11.6% | 0.68/0.32 | 4000 | 80 | 420 |
| E33 | 3.6 | 57 | 50 | 15.9% | 0.36/0.60 | 4000 | 80 | 410 |
| E34 | 3.8 | 58 | 49 | 16.1% | 0.36/0.60 | 4000 | 80 | 380 |
| E35 | 3.4 | 59 | 54 | 16.4% | 0.37/0.60 | 4000 | 80 | 360 |
| E36 | 6.2 | 6.6 | 3.3 | 5.4% | 0.14/0.15 | 6000 | 50 | 215 |
| E37 | 4.7 | 9.5 | 6.3 | 7.4% | 0.14/0.16 | 6000 | 50 | 240 |
| E38 | 5.2 | 19 | 12 | 5.7% | 0.28/0.61 | 25000 | 50 | 610 |
| E39 | 3.6 | 57 | 50 | 15.8% | 0.36/0.60 | 4000 | 80 | 530 |
| E40 | 4.2 | 10 | 7.4 | 7.7% | 0.14/0.16 | 6000 | 50 | 390 |
| E41 | 3.9 | 10.3 | 8.3 | 8.3% | 0.14/0.16 | 6000 | 50 | 360 |
| E42 | 4.5 | 8.3 | 5.7 | 5.9% | 0.14/0.18 | 6000 | 65 | 280 |
| E43 | 4.3 | 9.0 | 6.6 | 6.4% | 0.14/0.18 | 6000 | 65 | 295 |
| E44 | 4.3 | 9.0 | 6.3 | 7.3% | 0.14/0.15 | 6000 | 65 | 185 |
| E45 | 4.5 | 9.5 | 6.7 | 7.5% | 0.14/0.16 | 6000 | 65 | 190 |
| E46 | 4.7 | 10.5 | 6.9 | 8.5% | 0.14/0.16 | 6000 | 65 | 165 |
| E47 | 4.2 | 9.7 | 7.2 | 7.5% | 0.14/0.16 | 6000 | 50 | 395 |
| E48 | 4.3 | 10.1 | 7.7 | 8.4% | 0.14/0.16 | 6000 | 50 | 300 |
| E49 | 3.3 | 62 | 58 | 17.1% | 0.37/0.60 | 10000 | 70 | 230 |
| E50 | 3.4 | 61 | 57 | 17.3% | 0.37/0.60 | 10000 | 70 | 240 |
| E51 | 3.5 | 64 | 58 | 17.7% | 0.37/0.60 | 10000 | 70 | 225 |
| E52 | 4.2 | 9.9 | 7.4 | 7.8% | 0.14/0.16 | 6000 | 50 | 340 |
| E53 | 3.6 | 60 | 53 | 16.5% | 0.37/0.60 | 10000 | 70 | 215 |
| E54 | 3.5 | 56 | 49 | 15.4% | 0.36/0.60 | 4000 | 80 | 495 |
| E55 | 4.3 | 8.7 | 6.5 | 6.8% | 0.14/0.16 | 6000 | 50 | 350 |
| E56 | 4.2 | 9.5 | 7.0 | 7.3% | 0.14/0.16 | 6000 | 50 | 355 |
| E57 | 3.5 | 56 | 50 | 15.4% | 0.36/0.60 | 4000 | 80 | 460 |
| E58 | 3.6 | 59 | 52 | 16.3% | 0.36/0.60 | 4000 | 80 | 490 |
| E59 | 4.2 | 9.9 | 7.4 | 7.7% | 0.14/0.60 | 6000 | 50 | 240 |
| E60 | 4.2 | 9.5 | 7.1 | 7.4% | 0.14/0.60 | 6000 | 50 | 290 |
| E61 | 3.4 | 61 | 56 | 16.8% | 0.37/0.60 | 10000 | 65 | 380 |
| E62 | 3.5 | 60 | 54 | 16.5% | 0.37/0.60 | 10000 | 65 | 370 |
| E63 | 3.5 | 61 | 55 | 16.9% | 0.37/0.60 | 10000 | 65 | 305 |
| E64 | 3.7 | 59 | 50 | 16.3% | 0.37/0.60 | 10000 | 65 | 270 |
| E65 | 3.4 | 56 | 51 | 15.4% | 0.37/0.60 | 10000 | 65 | 340 |
| E66 | 3.6 | 55 | 47 | 15.3% | 0.37/0.60 | 10000 | 65 | 345 |
| E67 | 4.2 | 8.8 | 6.6 | 7.1% | 0.14/0.16 | 6000 | 50 | 315 |
| E68 | 3.4 | 55 | 50 | 15.1% | 0.36/0.60 | 4000 | 80 | 465 |
| Sol1 | 4.8 | 8.4 | 5.5 | 6.9% | 0.14/0.15 | 6000 | 50 | 155 |
| Sol2 | 4.7 | 6.8 | 4.6 | 5.5% | 0.14/0.15 | 6000 | 50 | 260 |
| Sol3 | 4.7 | 6.4 | 4.3 | 5.4% | 0.14/0.14 | 6000 | 65 | 190 |

**Tabelle 3: Strukturformeln der Materialien für die OLEDs**

| | |
|---|---|
| 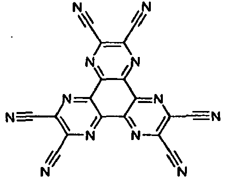 | 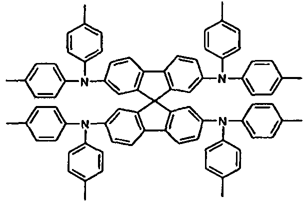 |
| HATCN | SpA1 |
| 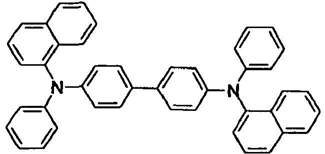 | 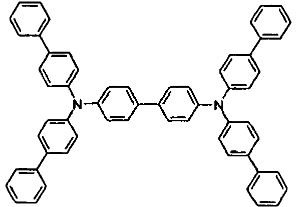 |
| NPB (Stand der Technik) | BPA1 (Stand der Technik) |
| 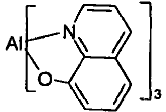 | 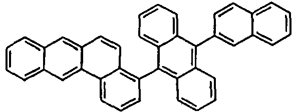 |
| Alq₃ | M1 |
| 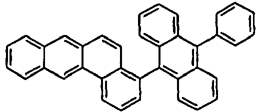 | 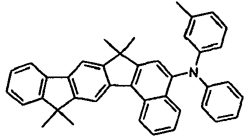 |
| M2 | D1 |
| 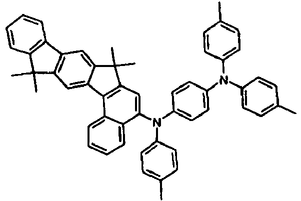 | 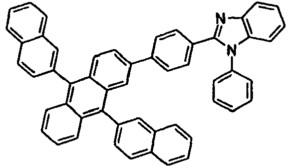 |
| D2 | ETM1 |
| 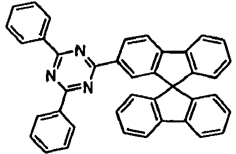 | 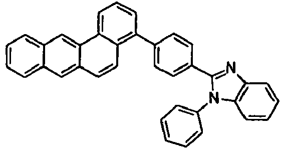 |
| ST1 | ETM2 |
| 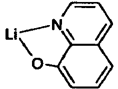 | 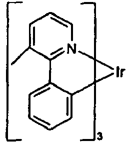 |
| LiQ | TEG1 |
| 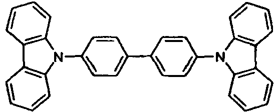 | 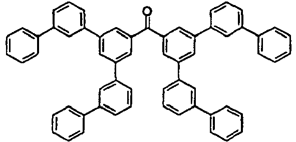 |
| CBP (Stand der Technik) | Ket1 |
| 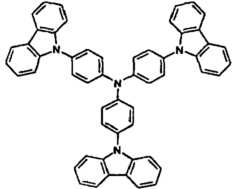 | 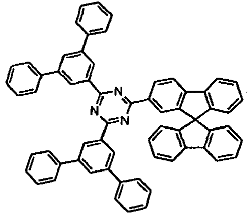 |
| TCTA (Stand der Technik) | ST2 |
| 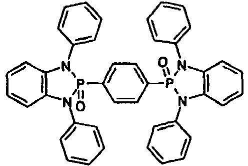 | 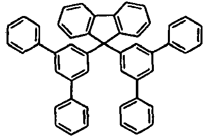 |
| DAP1 | FTPh (Stand der Technik) |
| 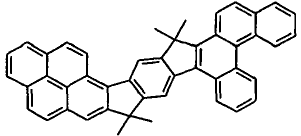 | 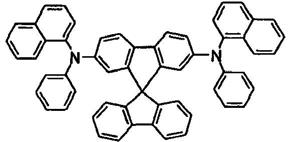 |
| D3 | SpNBP |
| 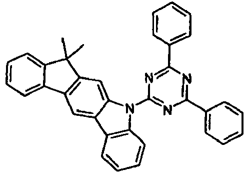 | 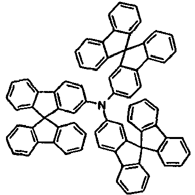 |
| IC1 | TSpA1 (Stand der Technik) |
| 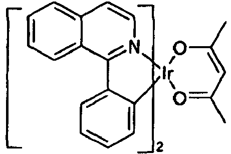 | 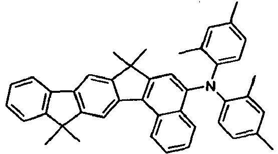 |
| TER1 | D4 |
| 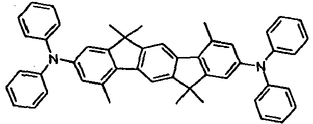 | 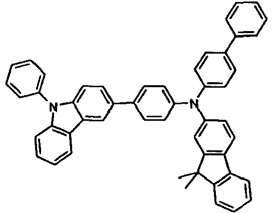 |
| TIFA1 | CbzA1 (Stand der Technik) |
| 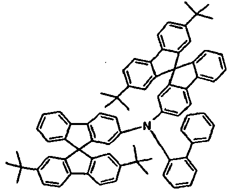 | |
| SpA-tb (Stand der Technik) | |

## Patentansprüche

1. Verbindung gemäß Formel (1), 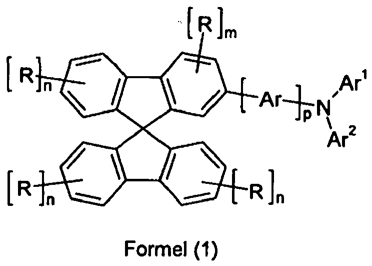 wobei für die verwendeten Symbole und Indizes gilt:
Ar ist gleich oder verschieden bei jedem Auftreten ein aromatisches Ringsystem, ausgewählt aus der Gruppe bestehend aus Benzol, Naphthalin, Phenanthren, Fluoren, Spirobifluoren, Dibenzofuran und Dibenzothiophen, welches jeweils mit einem oder mehreren Resten R¹ substituiert sein kann; dabei kann auch Ar mit Ar¹ und/oder mit Ar² durch eine Gruppe E verbunden sein;
Ar¹, Ar² ist gleich oder verschieden bei jedem Auftreten ein aromatisches oder heteroaromatisches Ringsystem mit 6 bis 60 C-Atomen, ausgewählt aus der Gruppe bestehend aus Benzol, Naphthalin, Phenanthren, Fluoren, Dibenzofuran und Dibenzothiophen, das jeweils auch durch einen oder mehrere Reste R¹ substituiert sein kann, oder unsubstituiertem Spirobifluoren oder einer Kombination aus zwei, drei, vier oder fünf dieser Gruppen, die jeweils gleich oder verschieden sein können; dabei können Ar¹ und Ar² miteinander und/oder Ar¹ mit Ar und/oder Ar² mit Ar durch eine Gruppe E verbunden sein;
E ist gleich oder verschieden bei jedem Auftreten ausgewählt aus der Gruppe bestehend aus C(R¹)₂, O, S und NR¹;
R, R¹ ist bei jedem Auftreten gleich oder verschieden ausgewählt aus der Gruppe bestehend aus H, D, F, Cl, Br, I, CN, Si(R²)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils mit einem oder mehreren Resten R² substituiert sein kann, wobei jeweils eine oder mehrere nicht-benachbarte CH₂-Gruppen durch Si(R²)₂, C=NR², P(=O)(R²), SO, SO₂, NR², O, S oder CONR² ersetzt sein können und wobei ein oder mehrere H-Atome durch D, F, Cl, Br oder I ersetzt sein können, einem aromatischen oder heteroaromatischen Ringsystem mit 6 bis 60 C-Atomen, ausgewählt aus der Gruppe bestehend aus Benzol, Naphthalin, Phenanthren, Fluoren, Spirobifluoren, Dibenzofuran und Dibenzothiophen, das jeweils mit einem oder mehreren Resten R² substituiert sein kann, oder einer Kombination aus zwei, drei, vier oder fünf dieser Gruppen, die jeweils gleich oder verschieden sein können, einer Aryloxygruppe mit 5 bis 60 aromatischen Ringatomen, die mit einem oder mehreren Resten R² substituiert sein kann, oder einer Aralkylgruppe mit 5 bis 60 aromatischen Ringatomen, die jeweils mit einem oder mehreren Resten R² substituiert sein kann, wobei optional zwei oder mehr benachbarte Substituenten R bzw. zwei oder mehr benachbarte Substituenten R¹ ein mono- oder polycyclisches, aliphatisches Ringsystem bilden können, das mit einem oder mehreren Resten R² substituiert sein kann;
R² ist bei jedem Auftreten gleich oder verschieden ausgewählt aus der Gruppe bestehend aus H, D, F, Cl, Br, I, Si(R³)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils mit einem oder mehreren Resten R³ substituiert sein kann, wobei eine oder mehrere nicht-benachbarte CH₂-Gruppen durch Si(R³)₂, C=NR³, P(=O)(R³), SO, SO₂, NR³, O, S oder CONR³ ersetzt sein können und wobei ein oder mehrere H-Atome durch D, F, Cl, Br oder I ersetzt sein können, einem aromatischen Ringsystem mit 6 bis 60 C-Atomen, ausgewählt aus der Gruppe bestehend aus Benzol, Naphthalin, Phenanthren, Fluoren, Spirobifluoren oder einer Kombination aus zwei, drei, vier oder fünf dieser Gruppen, die jeweils gleich oder verschieden sein können, das jeweils mit einem oder mehreren Resten R³ substituiert sein kann, einer Aryloxygruppe mit 5 bis 60 aromatischen Ringatomen, die mit einem oder mehreren Resten R³ substituiert sein kann, oder einer Aralkylgruppe mit 5 bis 60 aromatischen Ringatomen, die mit einem oder mehreren Resten R³ substituiert sein kann, wobei optional zwei oder mehr benachbarte Substituenten R² ein mono- oder polycyclisches, aliphatisches Ringsystem bilden können, das mit einem oder mehreren Resten R³ substituiert sein kann;
R³ ist ausgewählt aus der Gruppe bestehend aus H, D, F, einem aliphatischem Kohlenwasserstoffrest mit 1 bis 20 C-Atomen, einem aromatischem Ringsystem mit 6 bis 30 C-Atomen, in dem ein oder mehrere H-Atome durch D oder F ersetzt sein können, wobei zwei oder mehr benachbarte Substituenten R³ miteinander ein mono- oder polycyclisches, aliphatisches, Ringsystem bilden können;
m ist 0, 1, 2 oder 3;
n ist bei jedem Auftreten gleich oder verschieden 0, 1, 2, 3 oder 4;
p ist 0, 1 oder 2;
dabei sind die folgenden Verbindungen von der Erfindung ausgenommen:

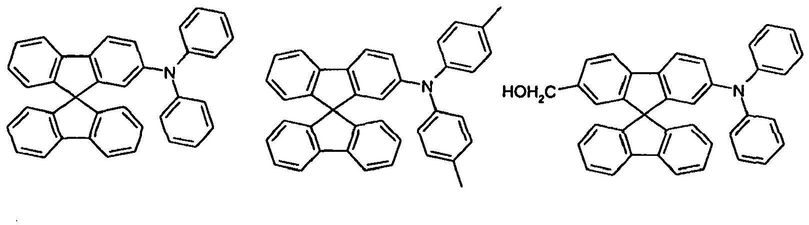

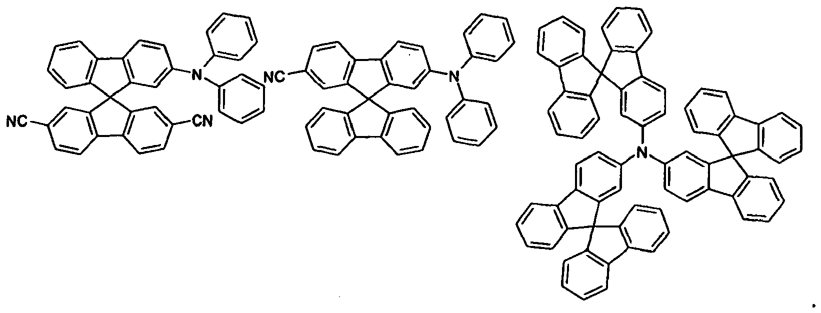

2. Verbindung nach Anspruch 1 gemäß Formel (2), Formel (3a), (3b), (4a) oder Formel (4b), 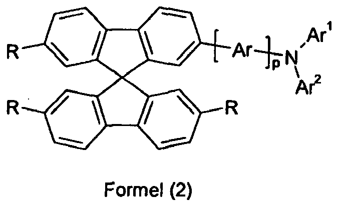 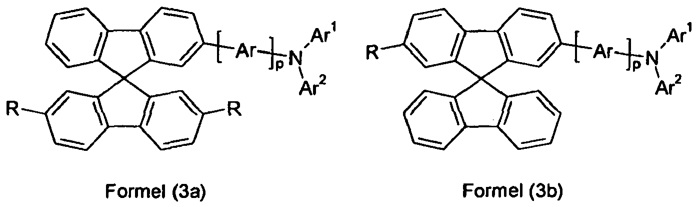 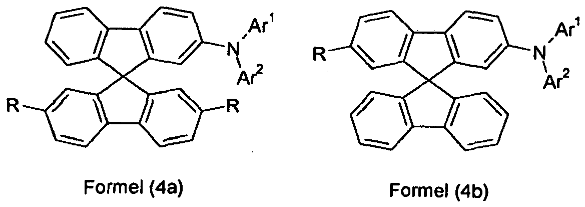 wobei die verwendeten Symbole und Indizes die in Anspruch 1 genannten Bedeutungen aufweisen.

3. Verbindung nach Anspruch 1 oder 2, **dadurch gekennzeichnet, dass** die Gruppen Ar¹ und Ar² gleich oder verschieden bei jedem Auftreten ausgewählt aus den Gruppen der Formeln (5) bis (28), 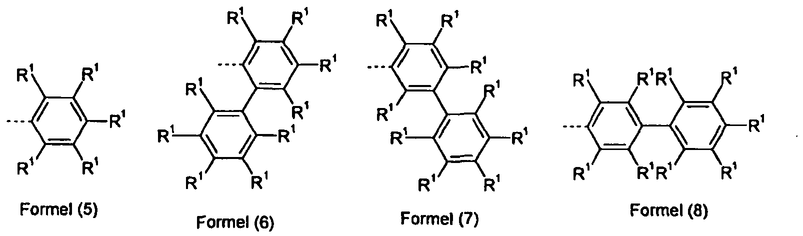 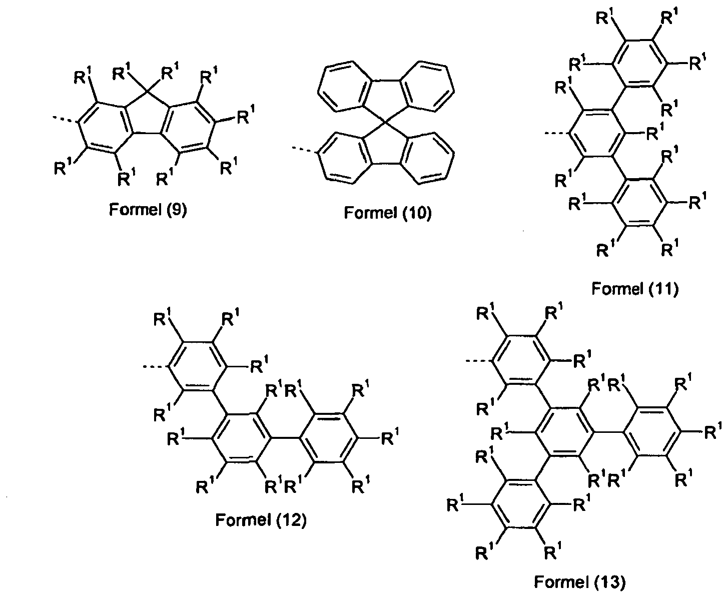 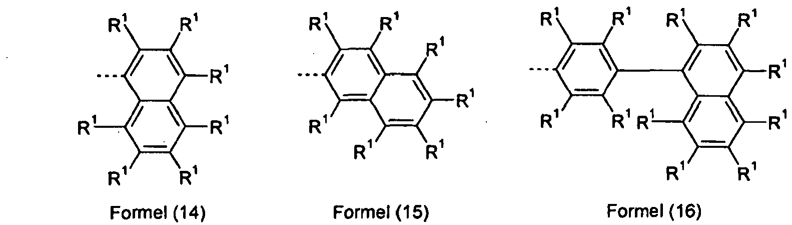 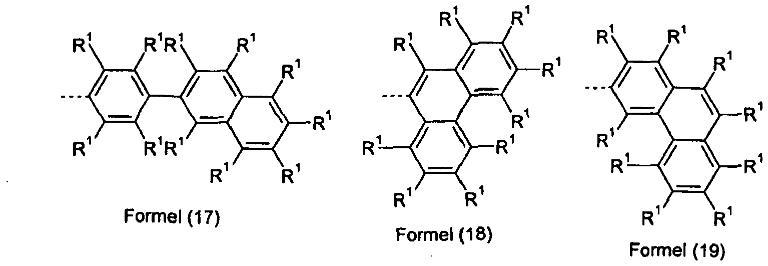 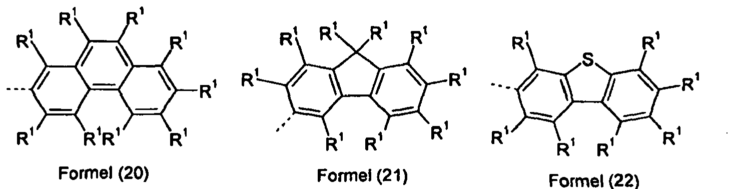 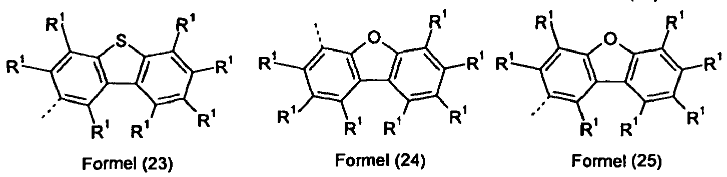 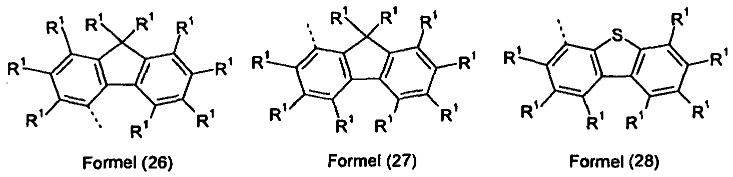 wobei die verwendeten Symbole die in Anspruch 1 genannten Bedeutungen haben und die gestrichelte Bindung die Position der Bindung der Gruppe an den Stickstoff andeutet.

4. Verbindung nach einem oder mehreren der Ansprüche 1 bis 3, **dadurch gekennzeichnet, dass** die Gruppen Ar¹ und Ar² gleich oder verschieden bei jedem Auftreten ausgewählt sind aus den Gruppen der Formeln (5a) bis (28a), 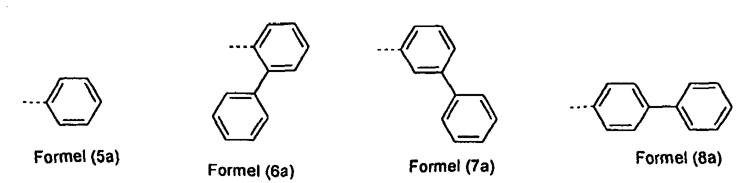 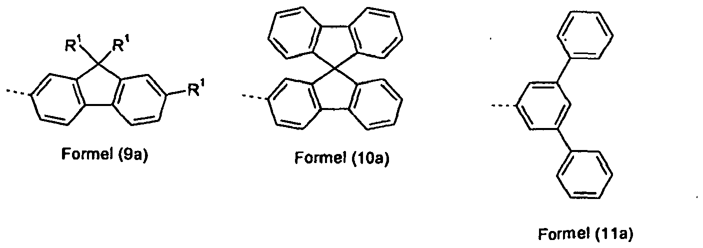 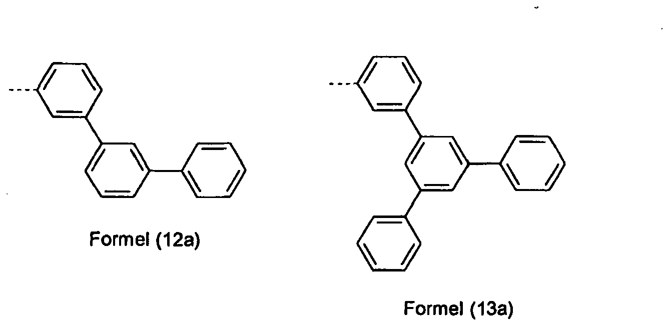 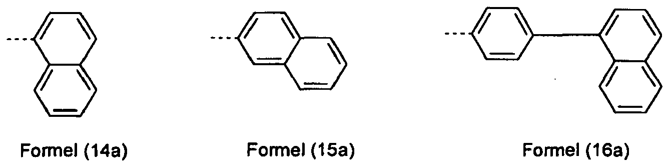 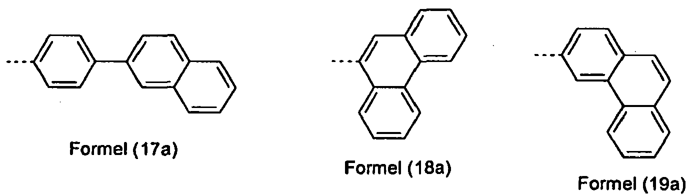 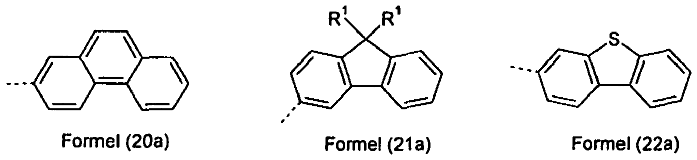 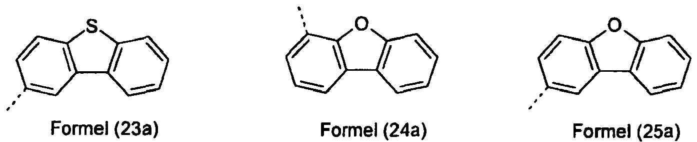 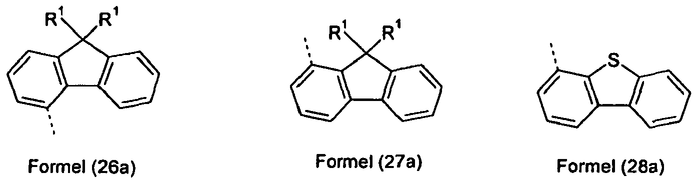 wobei die verwendeten Symbole die in Anspruch 1 genannten Bedeutungen haben und die gestrichelte Bindung die Position der Bindung der Gruppe an den Stickstoff andeutet.

5. Verbindung nach Anspruch 3 oder Anspruch 4, **dadurch gekennzeichnet, dass** Ar¹ eine Gruppe der Formel (6), (7), (8), (9) oder (21) und bevorzugt eine Gruppe der Formel (6a), (7a), (8a), (9a) oder (21a) ist.

6. Verbindung nach einem oder mehreren der Ansprüche 1 bis 5, **dadurch gekennzeichnet, dass** die Gruppen Ar¹ und Ar² unterschiedlich voneinander sind.

7. Verbindung nach einem oder mehreren der Ansprüche 1 bis 6, **dadurch gekennzeichnet, dass** die Gruppe -NAr¹Ar² die Struktur gemäß einer der Formeln (29), (30), (31) oder (32) aufweist oder dass die Gruppe -Ar-NAr¹Ar² die Struktur gemäß einer der Formeln (33), (34), (35) oder (36) aufweist, 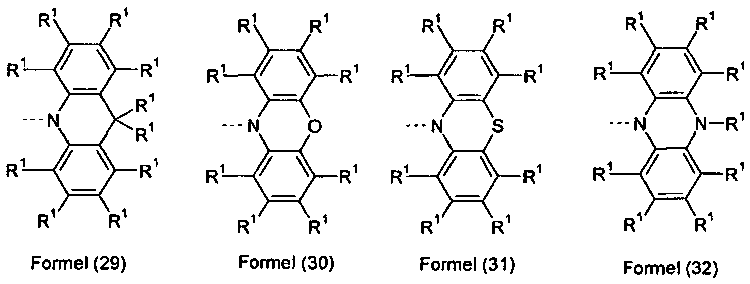 wobei die verwendeten Symbole die in Anspruch 1 genannten Bedeutungen aufweisen und die gestrichelte Bindung die Bindung an das Spirobifluoren bzw. an Ar andeutet; 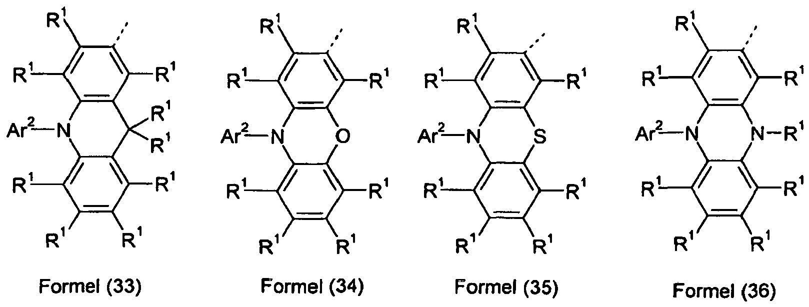 wobei die verwendeten Symbole die in Anspruch 1 genannten Bedeutungen aufweisen und die gestrichelte Bindung die Bindung an das Spirobifluoren andeutet.

8. Verbindung nach einem oder mehreren der Ansprüche 1 bis 7, **dadurch gekennzeichnet, dass** die Gruppe -(Ar)ₚ-für eine Gruppe gemäß einer der Formeln (37) bis (50) steht, 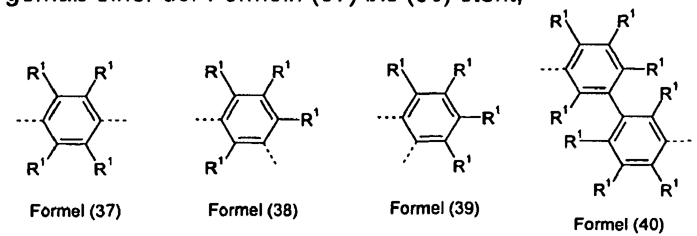 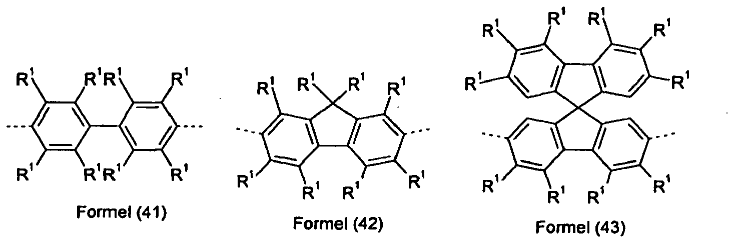 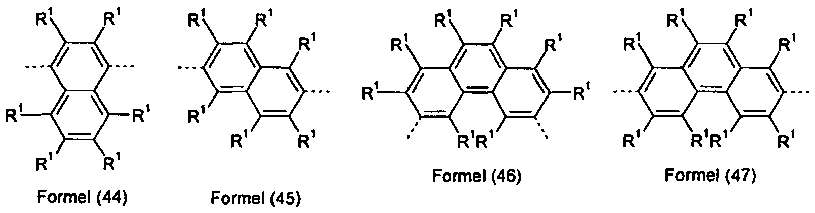 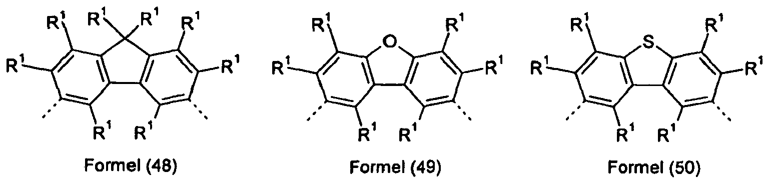 wobei die verwendeten Symbole die in Anspruch 1 genannten Bedeutungen aufweisen und eine gestrichelte Bindung die Bindung an das Spirobifluoren andeutet und die andere gestrichelte Bindung die Bindung an das Stickstoffatom andeutet.

9. Verbindung nach einem oder mehreren der Ansprüche 1 bis 8, für die gilt:
Ar ist gleich oder verschieden bei jedem Auftreten ein aromatisches Ringsystem, wobei für p = 1 oder 2 -(Ar)p- ausgewählt ist aus den Gruppen der Formel (37) bis (50); dabei kann Ar auch mit Ar¹ und/oder Ar² durch eine Gruppe E verbunden sein;
Ar¹, Ar² ist gleich oder verschieden bei jedem Auftreten ein aromatisches Ringsystem, ausgewählt aus den Gruppen der Formel (5) bis (25);
oder -NAr¹Ar² steht für eine Gruppe gemäß einer der Formeln (29) bis (33);
oder -Ar-NAr¹Ar² steht für eine Gruppe gemäß einer der Formeln (34) bis (37);
E ist bei jedem Auftreten gleich oder verschieden C(R¹)₂, N(R¹), O oder S;
R ist bei jedem Auftreten gleich oder verschieden ausgewählt aus der Gruppe bestehend aus H, D, F, Si(R²)₃, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, einem aromatischen Ringsystem mit 6 bis 60 C-Atomen, ausgewählt aus der Gruppe bestehend aus Benzol, Naphthalin, Phenanthren, Fluoren, Spirobifluoren oder einer Kombination aus zwei oder drei dieser Gruppen, die jeweils gleich oder verschieden sein können, das jeweils mit einem oder mehreren Resten R² substituiert sein kann, wobei optional zwei oder mehr benachbarte Substituenten R ein mono-oder polycyclisches, aliphatisches Ringsystem bilden können, das mit einem oder mehreren Resten R² substituiert sein kann;
R¹ ist, wenn der Rest R¹ and Ar¹ oder Ar² bindet, gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atome;
oder R¹, welcher in Formel (29) bzw. (33) an die Kohlenstoffbrücke bindet, ist gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R² substituiert sein kann; dabei können die beiden Reste R¹ auch miteinander ein aliphatisches oder aromatisches Ringsystem bilden;
oder R¹, welcher in Formel (32) bzw. (36) an die Stickstoffbrücke bindet, ist ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R² substituiert sein kann;
R² ist bei jedem Auftreten gleich oder verschieden ausgewählt aus der Gruppe bestehend aus H, D, einer geradkettigen Alkylgruppe mit 1 bis 5 C-Atomen oder einer verzweigten oder cyclischen Alkylgruppe mit 3 bis 5 C-Atomen, oder einem aromatischen Ringsystem mit 6 bis 18 C-Atomen;
R³ ist ausgewählt aus der Gruppe bestehend aus H, D, F, einem aliphatischem Kohlenwasserstoffrest mit 1 bis 10 C-Atomen, einem aromatischem Ringsystem mit 6 bis 24 C-Atomen, in dem ein oder mehrere H-Atome durch D oder F ersetzt sein können, wobei zwei oder mehr benachbarte Substituenten R³ miteinander ein mono- oder polycyclisches, aliphatisches, aromatisches oder heteroaromatisches Ringsystem bilden können;
m ist 0, 1 oder 2;
n ist bei jedem Auftreten gleich oder verschieden O, 1 oder 2;
p ist 0, 1 oder 2.

10. Verfahren zur Herstellung einer Verbindung nach einem oder mehreren der Ansprüche 1 bis 9 durch Kupplung eines Spirobifluorenderivats, welches in 2-Position mit einer reaktiven Abgangsgruppe substituiert ist, mit
a) einem primären Amin, gefolgt von der Kupplung mit einer weiteren aromatischen Gruppe, die mit einer reaktiven Abgangsgruppe substituiert ist, oder
b) mit sekundären Amin, oder
c) mit einem Triarylaminderivat.

11. Formulierung, insbesondere eine Lösung, Dispersion oder Mini-emulsion, enthaltend mindestens eine Verbindung nach einem oder mehreren der Ansprüche 1 bis 9 und mindestens ein Lösemittel, insbesondere ein organisches Lösemittel, oder eine Mischung, enthaltend mindestens eine Verbindung nach einem oder mehreren der Ansprüche 1 bis 9 und mindestens eine weitere Verbindung.

12. Verwendung einer Verbindung nach einem oder mehreren der Ansprüche 1 bis 9 oder einer Formulierung nach Anspruch 11 in einer elektronischen Vorrichtung, insbesondere in einer organischen Elektrolumineszenzvorrichtung.

13. Elektronische Vorrichtung, insbesondere ausgewählt aus der Gruppe bestehend aus organischen Elektrolumineszenzvorrichtungen (organischen Leuchtdioden, OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (ODSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", enthaltend mindestens eine Verbindung nach einem oder mehreren der Ansprüche 1 bis 9 oder eine Mischung nach Anspruch 11.

14. Organische Elektrolumineszenzvorrichtung nach Anspruch 13, **dadurch gekennzeichnet, dass** die Verbindung nach einem oder mehreren der Ansprüche 1 bis 9 oder die Mischung nach Anspruch 11 als Lochtransportmaterial in einer Lochtransport- oder Lochinjektions- oder Exzitonenblockierschicht oder als Matrixmaterial für fluoreszierende oder phosphoreszierende Emitter eingesetzt wird.

## Claims

1. Compound of the formula (1), 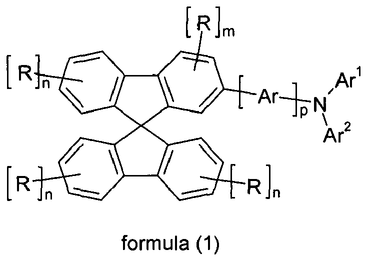 where the following applies to the symbols and indices used:
Ar is, identically or differently on each occurrence, an aromatic ring system selected from the group consisting of benzene, naphthalene, phenanthrene, fluorene, spirobifluorene, dibenzofuran and dibenzothiophene, which may in each case be substituted by one or more radicals R¹; Ar may also be connected to Ar¹ and/or to Ar² here by a group E;
Ar¹, Ar² are, identically or differently on each occurrence, an aromatic or heteroaromatic ring system having 6 to 60 C atoms selected from the group consisting of benzene, naphthalene, phenanthrene, fluorene, dibenzofuran and dibenzothiophene, which may in each case also be substituted by one or more radicals R¹, or unsubstituted spirobifluorene or a combination of two, three, four or five of these groups, which may in each case be identical or different; Ar¹ and Ar² here may be connected to one another and/or Ar¹ may be connected to Ar and/or Ar² may be connected to Ar by a group E;
E is, identically or differently on each occurrence, selected from the group consisting of C(R')₂, O, S and NR¹;
R, R¹ are on each occurrence, identically or differently, selected from the group consisting of H, D, F, Cl, Br, I, CN, Si(R²)₃, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R², where in each case one or more non-adjacent CH₂ groups may be replaced by Si(R²)₂, C=NR², P(=O)(R²), SO, SO₂, NR², O, S or CONR² and where one or more H atoms may be replaced by D, F, Cl, Br or I, an aromatic or heteroaromatic ring system having 6 to 60 C atoms selected from the group consisting of benzene, naphthalene, phenanthrene, fluorene, spirobifluorene, dibenzofuran and dibenzothiophene, which may in each case may be substituted by one or more radicals R², or a combination of two, three, four or five of these groups, which may in each case be identical or different, an aryloxy group having 5 to 60 aromatic ring atoms, which may be substituted by one or more radicals R², or an aralkyl group having 5 to 60 aromatic ring atoms, which may in each case be substituted by one or more radicals R², where two or more adjacent substituents R or two or more adjacent substituents R¹ may optionally form a mono- or polycyclic, aliphatic ring system, which may be substituted by one or more radicals R²;
R² is on each occurrence, identically or differently, selected from the group consisting of H, D, F, CI, Br, I, Si(R³)₃, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R³, where one or more non-adjacent CH₂ groups may be replaced by Si(R³)₂, C=NR³, P(=O)(R³), SO, SO₂, NR³, O, S or CONR³ and where one or more H atoms may be replaced by D, F, Cl, Br or I, an aromatic ring system having 6 to 60 C atoms selected from the group consisting of benzene, naphthalene, phenanthrene, fluorene, spirobifluorene or a combination of two, three, four or five of these groups, which may in each case be identical or different, which ring system may in each case be substituted by one or more radicals R³, an aryloxy group having 5 to 60 aromatic ring atoms, which may be substituted by one or more radicals R³, or an aralkyl group having 5 to 60 aromatic ring atoms, which may be substituted by one or more radicals R³, where two or more adjacent substituents R² may optionally form a mono- or polycyclic, aliphatic ring system, which may be substituted by one or more radicals R³;
R³ is selected from the group consisting of H, D, F, an aliphatic hydrocarbon radical having 1 to 20 C atoms, an aromatic ring system having 6 to 30 C atoms, in which one or more H atoms may be replaced by D or F, where two or more adjacent substituents R³ may form a mono- or polycyclic, aliphatic ring system with one another;
m is 0, 1, 2 or 3;
n is on each occurrence, identically or differently, 0, 1, 2, 3 or 4;
p is 0, 1 or 2;
the following compounds are excluded from the invention:

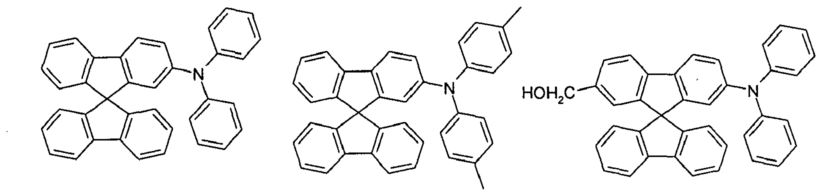

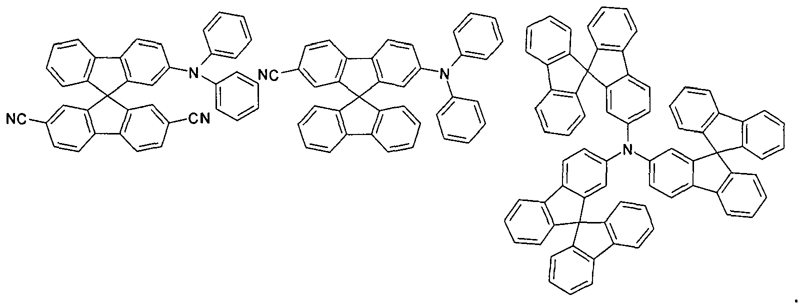

2. Compound according to Claim 1 of the formula (2), formula (3a), (3b), (4a) or formula (4b), 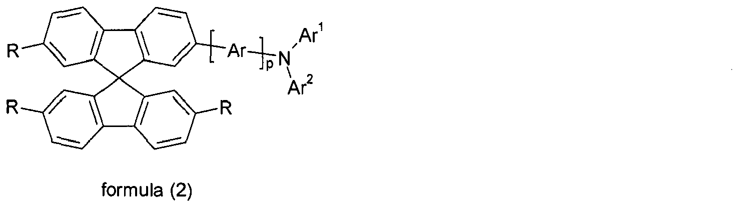 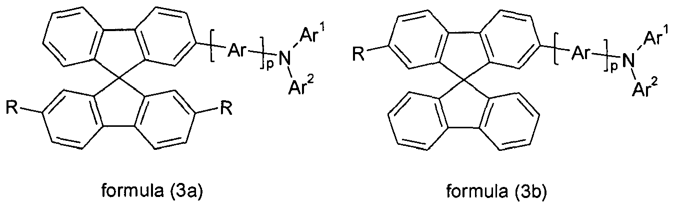 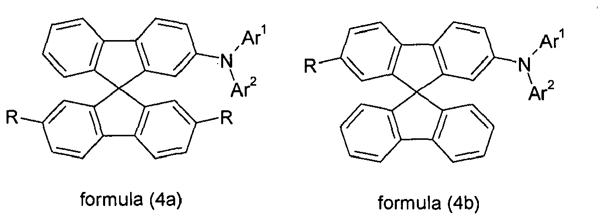 where the symbols and indices used have the meanings given in Claim 1.

3. Compound according to Claim 1 or 2, **characterised in that** the groups Ar¹ and Ar² are selected, identically or differently on each occurrence, from the groups of the formulae (5) to (28), 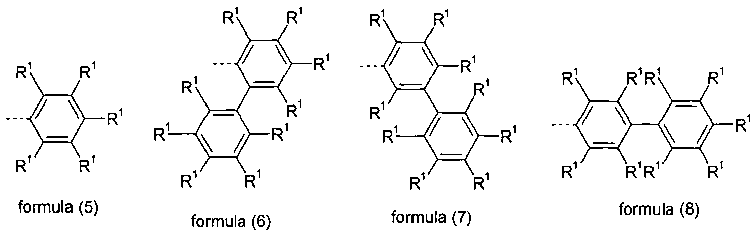 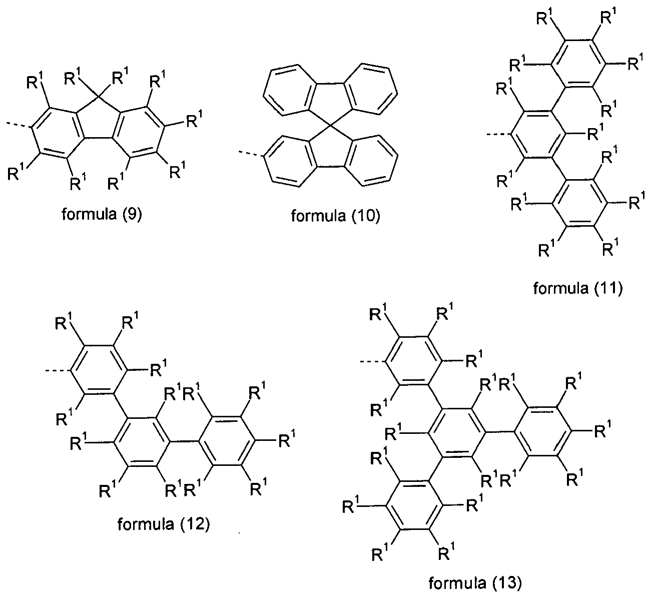 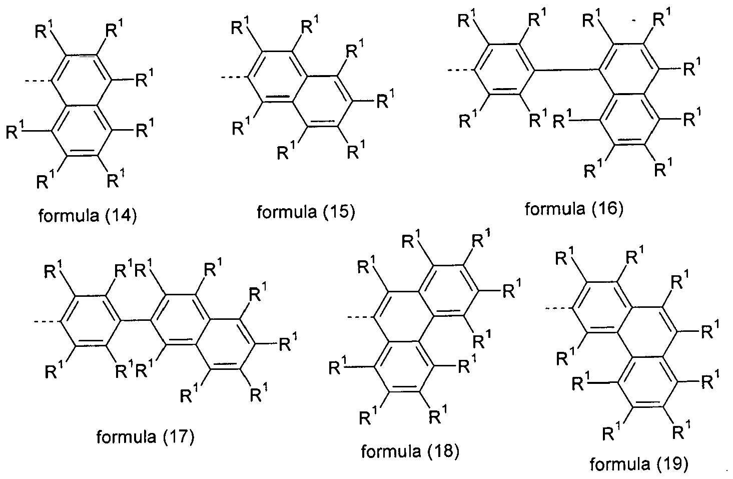 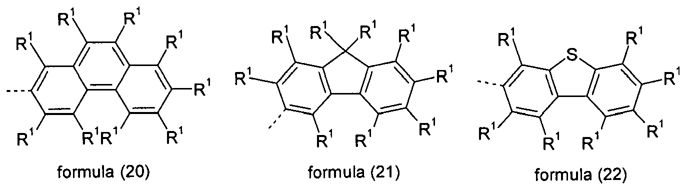 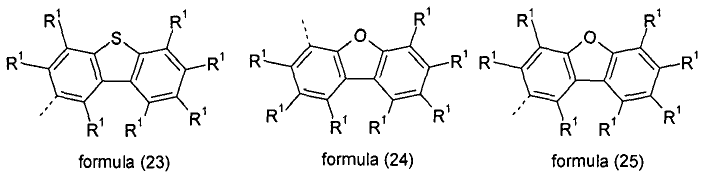 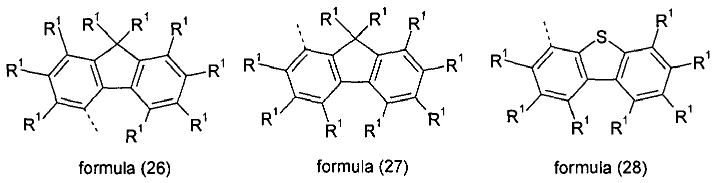 where the symbols used have the meanings given in Claim 1, and the dashed bond indicates the position of the bond from the group to the nitrogen.

4. Compound according to one or more of Claims 1 to 3, **characterised in that** the groups Ar¹ and Ar² are selected, identically or differently on each occurrence, from the groups of the formulae (5a) to (28a), 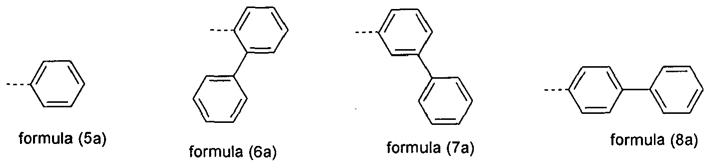 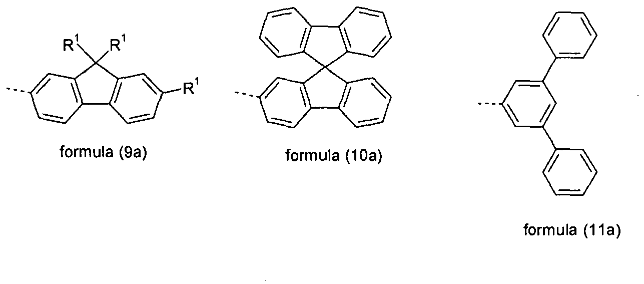 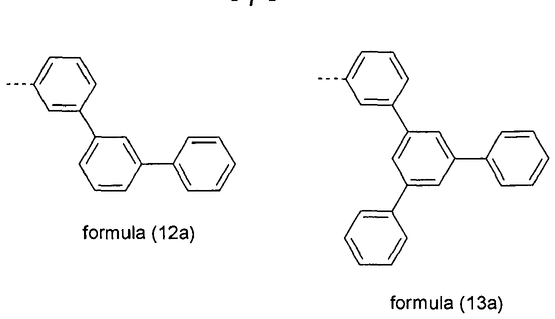 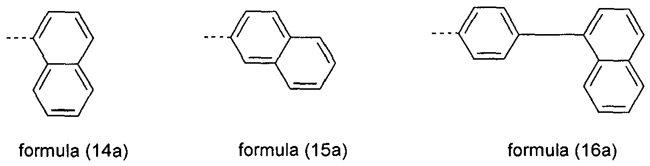 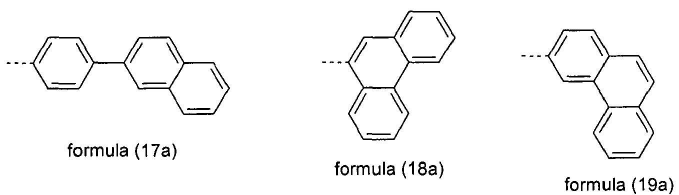 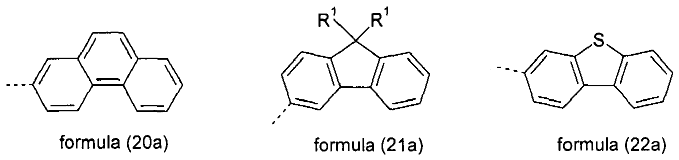 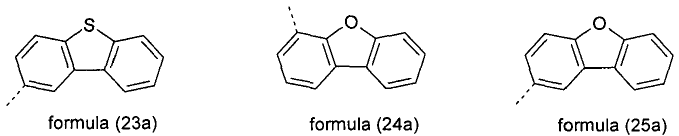 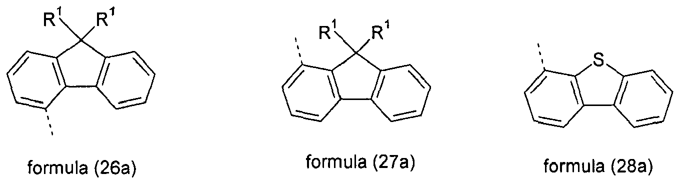 where the symbols used have the meanings given ion Claim 1, and the dashed bond indicates the position of the bond from the group to the nitrogen.

5. Compound according to Claim 3 or Claim 4, **characterised in that** Ar¹ is a group of the formula (6), (7), (8), (9) or (21) and preferably a group of the formula (6a), (7a), (8a), (9a) or (21 a).

6. Compound according to one or more of Claims 1 to 5, **characterised in that** the groups Ar¹ and Ar² are different from one another.

7. Compound according to one or more of Claims 1 to 6, **characterised in that** the group -NAr¹Ar² has the structure of one of the formulae (29), (30), (31) or (32) or **in that** the group -Ar-NAr¹Ar² has the structure of one of the formulae (33), (34), (35) or (36), 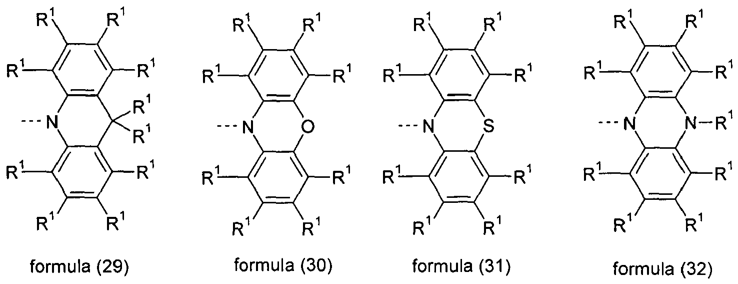 where the symbols used have the meanings given in Claim 1, and the dashed bond indicates the bond to the spirobifluorene or to Ar; 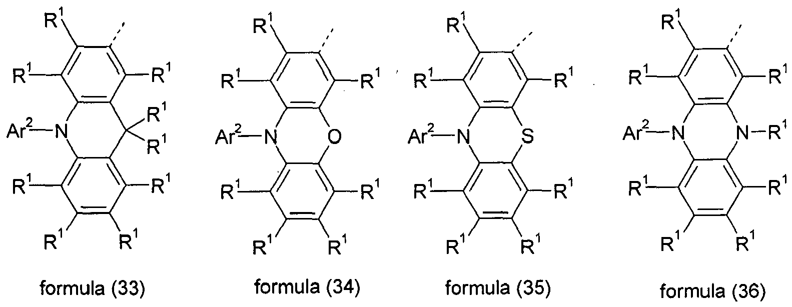 where the symbols used have the meanings given in Claim 1, and the dashed bond indicates the bond to the spirobifluorene.

8. Compound according to one or more of Claims 1 to 7, **characterised in that** the group -(Ar)p- stands for a group of one of the formulae (37) to (50), 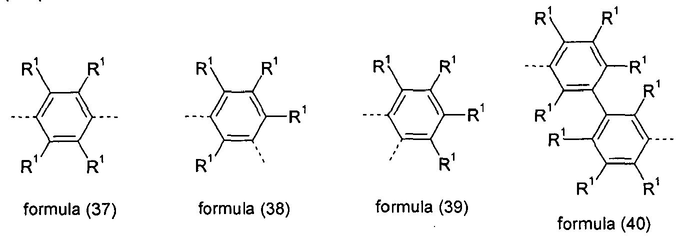 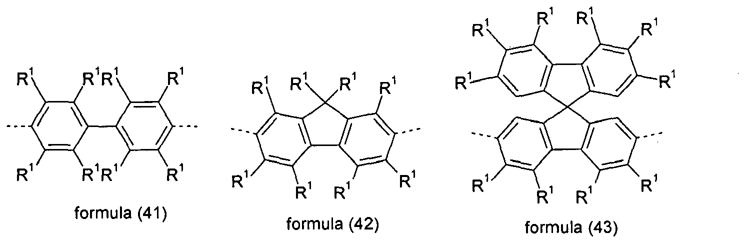 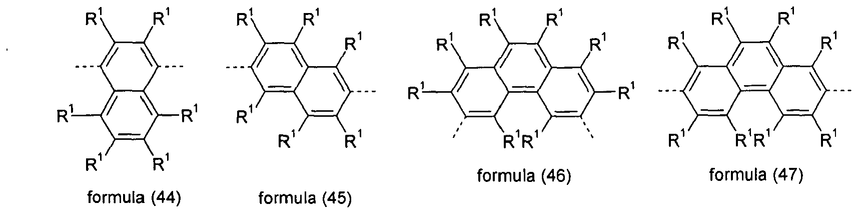 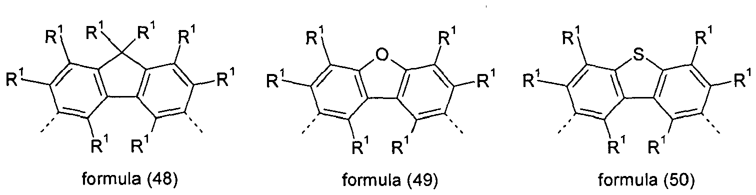 where the symbols used have the meanings given in Claim 1, and one dashed bond indicates the bond to the spirobifluorene and the other dashed bond indicates the bond to the nitrogen atom.

9. Compound according to one or more of Claims 1 to 8, for which:
Ar is, identically or differently on each occurrence, an aromatic ring system, where, for p = 1 or 2, -(Ar)p- is selected from the groups of
the formulae (37) to (50); Ar here may also be connected to Ar¹ and/or Ar² by a group E;
Ar¹, Ar² are, identically or differently on each occurrence, an aromatic ring system selected from the groups of the formulae (5) to (25); or -NAr¹Ar² stands for a group of one of the formulae (29) to (33); or -Ar-NAr¹Ar² stands for a group of one of the formulae (34) to (37);
E is on each occurrence, identically or differently, C(R¹)₂, N(R¹), O or S;
R is selected on each occurrence, identically or differently, from the group consisting of H, D, F, Si(R²)₃, a straight-chain alkyl or alkoxy group having 1 to 10 C atoms or a branched or cyclic alkyl or alkoxy group having 3 to 10 C atoms, each of which may be substituted by one or more radicals R², where one or more non-adjacent CH₂ groups may be replaced by O and where one or more H atoms may be replaced by D or F, an aromatic ring system having 6 to 60 C atoms selected from the group consisting of benzene, naphthalene, phenanthrene, fluorene, spirobifluorene or a combination of two or three of these groups, which may in each case be identical or different, which ring system may in each case be substituted by one or more radicals R², where two or more adjacent substituents R may optionally form a mono- or polycyclic, aliphatic ring system, which may be substituted by one or more radicals R²;
R¹ is, if the radical R¹ is bonded to Ar¹ or Ar², selected, identically or differently on each occurrence, from the group consisting of H, D, a straight-chain alkyl group having 1 to 10 C atoms or a branched or cyclic alkyl group having 3 to 10 C atoms;
or R¹ which is bonded to the carbon bridge in formula (29) or (33) is selected, identically or differently on each occurrence, from the group consisting of a straight-chain alkyl group having 1 to 10 C atoms, a branched or cyclic alkyl group having 3 to 10 C atoms or an aromatic ring system having 6 to 30 C atoms, which may be substituted by one or more radicals R²; the two radicals R¹ here may also form an aliphatic or aromatic ring system with one another;
or R¹ which is bonded to the nitrogen bridge in formula (32) or (36) is selected from the group consisting of a straight-chain alkyl group having 1 to 10 C atoms, a branched or cyclic alkyl group having 3 to 10 C atoms or an aromatic ring system having 6 to 30 C atoms, which may be substituted by one or more radicals R²;
R² is selected on each occurrence, identically or differently, from the group consisting of H, D, a straight-chain alkyl group having 1 to 5 C atoms or a branched or cyclic alkyl group having 3 to 5 C atoms or an aromatic ring system having 6 to 18 C atoms;
R³ is selected from the group consisting of H, D, F, an aliphatic hydrocarbon radical having 1 to 10 C atoms, an aromatic ring system having 6 to 24 C atoms, in which one or more H atoms may be replaced by D or F, where two or more adjacent substituents R³ may form a mono- or polycyclic, aliphatic, aromatic or heteroaromatic ring system with one another;
m is 0, 1 or 2;
n is on each occurrence, identically or differently, 0, 1 or 2;
p is 0, 1 or 2.

10. Process for the preparation of a compound according to one or more of Claims 1 to 9 by coupling a spirobifluorene derivative which is substituted in the 2-position by a reactive leaving group to
a) a primary amine, followed by coupling to a further aromatic group which is substituted by a reactive leaving group, or
b) to a secondary amine, or
c) to a triarylamine derivative.

11. Formulation, in particular a solution, dispersion or mini-emulsion, comprising at least one compound according to one or more of Claims 1 to 9 and at least one solvent, in particular an organic solvent, or a mixture comprising at least one compound according to one or more of Claims 1 to 9 and at least one further compound.

12. Use of a compound according to one or more of Claims 1 to 9 or a formulation according to Claim 11 in an electronic device, in particular in an organic electroluminescent device.

13. Electronic device, in particular selected from the group consisting of organic electroluminescent devices (organic light-emitting diodes, OLEDs), organic integrated circuits (O-ICs), organic field-effect transistors (O-FETs), organic thin-film transistors (O-TFTs), organic light-emitting transistors (O-LETs), organic solar cells (O-SCs), organic dye-sensitised solar cells (ODSSCs), organic optical detectors, organic photoreceptors, organic field-quench devices (O-FQDs), light-emitting electrochemical cells (LECs), organic laser diodes (O-lasers) and organic plasmon emitting devices, comprising at least one compound according to one or more of Claims 1 to 9 or a mixture according to Claim 11.

14. Organic electroluminescent device according to Claim 13, **characterised in that** the compound according to one or more of Claims 1 to 9 or the mixture according to Claim 11 is employed as hole-transport material in a hole-transport or hole-injection or exciton-blocking layer or as matrix material for fluorescent or phosphorescent emitters.

## Revendications

1. Composé de la formule (1) : 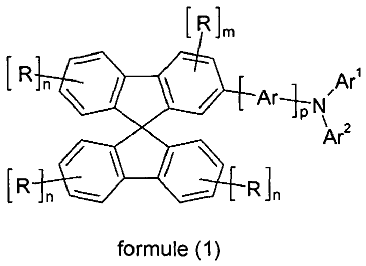 où ce qui suit s'applique aux symboles et indices utilisés :
Ar est, de manière identique ou différente pour chaque occurrence, un système de cycle aromatique choisi parmi le groupe constitué par benzène, naphtalène, phénanthrène, fluorène, spirobifluorène, dibenzofurane et dibenzothiophène, lequel peut dans chaque cas être substitué par un ou plusieurs radical/radicaux R¹ ; Ar peut également être connecté à Ar¹ et/ou à Ar² ici par un groupe E ;
Ar¹, Ar² sont, de manière identique ou différente pour chaque occurrence, un système de cycle aromatique ou hétéroaromatique comportant de 6 à 60 atomes de C choisi parmi le groupe constitué par benzène, naphtalène, phénanthrène, fluorène, dibenzofurane et dibenzothiophène, lequel peut dans chaque cas également être substitué par un ou plusieurs radical/radicaux R¹, ou spirobifluorène non substitué ou une combinaison de deux, trois, quatre ou cinq de ces groupes, lesquels peuvent dans chaque cas être identiques ou différents ; Ar¹ et Ar² ici peuvent être connectés l'un à l'autre et/ou Ar¹ peut être connecté à Ar et/ou Ar² peut être connecté à Ar par un groupe E ;
E est, de manière identique ou différente pour chaque occurrence, choisi parmi le groupe constitué par C(R¹)₂, O, S et NR¹ ;
R, R¹ sont, pour chaque occurrence, de manière identique ou différente, choisis parmi le groupe constitué par H, D, F, Cl, Br, I, CN, Si(R²)₃, un groupe alkyle, alcoxy ou thioalkyle en chaîne droite comportant de 1 à 40 atome(s) de C ou un groupe alkyle, alcoxy ou thioalkyle ramifié ou cyclique comportant de 3 à 40 atomes de C, dont chacun peut être substitué par un ou plusieurs radical/ radicaux R², où dans chaque cas un ou plusieurs groupe(s) CH₂ non adjacents peut/peuvent être remplacé(s) par Si(R²)₂, C=NR², P(=O)(R²), SO, SO₂, NR², O, S ou CONR² et où un ou plusieurs atome(s) de H peut/peuvent être remplacé(s) par D, F, Cl, Br ou I, un système de cycle aromatique ou hétéroaromatique comportant de 6 à 60 atomes de C choisi parmi le groupe constitué par benzène, naphtalène, phénanthrène, fluorène, spirobifluorène, dibenzofurane et dibenzothiophène, lequel peut dans chaque cas être substitué par un ou plusieurs radical/radicaux R², ou une combinaison de deux, trois, quatre ou cinq de ces groupes, lesquels peuvent dans chaque cas être identiques ou différents, un groupe aryloxy comportant de 5 à 60 atomes de cycle aromatique, lequel peut être substitué par un ou plusieurs radical/radicaux R², ou un groupe aralkyle comportant de 5 à 60 atomes de cycle aromatique, lequel peut dans chaque cas être substitué par un ou plusieurs radical/radicaux R², où deux substituants R adjacents ou plus ou deux substituants R¹ adjacents ou plus peuvent en option former un système de cycle aliphatique monocyclique ou polycyclique, lequel peut être substitué par un ou plusieurs radical/ radicaux R² ;
R² est pour chaque occurrence, de manière identique ou différente, choisi parmi le groupe constitué par H, D, F, Cl, Br, I, Si(R³)₃, un groupe alkyle, alcoxy ou thioalkyle en chaîne droite comportant de 1 à 40 atome(s) de C ou un groupe alkyle, alcoxy ou thioalkyle ramifié ou cyclique comportant de 3 à 40 atomes de C, dont chacun peut être substitué par un ou plusieurs radical/radicaux R³, où un ou plusieurs groupe(s) CH₂ non adjacents peut/peuvent être remplacé(s) par Si(R³)₂, C=NR³, P(=O)(R³), SO, SO₂, NR³, O, S ou CONR³ et où un ou plusieurs atome(s) de H peut/peuvent être remplacé(s) par D, F, Cl, Br ou I, un système de cycle aromatique comportant de 6 à 60 atomes de C choisi parmi le groupe constitué par benzène, naphtalène, phénanthrène, fluorène, spirobifluorène ou une combinaison de deux, trois, quatre ou cinq de ces groupes, lesquels peuvent dans chaque cas être identiques ou différents, lequel système de cycle peut dans chaque cas être substitué par un ou plusieurs radical/radicaux R³, un groupe aryloxy comportant de 5 à 60 atomes de cycle aromatique, lequel peut être substitué par un ou plusieurs radical/radicaux R³, ou un groupe aralkyle comportant de 5 à 60 atomes de cycle aromatique, lequel peut être substitué par un ou plusieurs radical/radicaux R³, où deux substituants R² adjacents ou plus peuvent en option former un système de cycle aliphatique monocyclique ou polycyclique, lequel peut être substitué par un ou plusieurs radical/ radicaux R³ ;
R³ est choisi parmi le groupe constitué par H, D, F, un radical hydrocarbone aliphatique comportant de 1 à 20 atome(s) de C, un système de cycle aromatique comportant de 6 à 30 atomes de C, où un ou plusieurs atome(s) de H peut/peuvent être remplacé(s) par D ou F, où deux substituants R³ adjacents ou plus peuvent former un système de cycle aliphatique monocyclique ou polycyclique l'un avec l'autre ou les uns avec les autres ;
m est 0, 1, 2 ou 3 ;
n est pour chaque occurrence, de manière identique ou différente, 0, 1, 2, 3 ou 4 ;
p est 0, 1 ou 2 ;
les composés qui suivent sont exclus de l'invention :

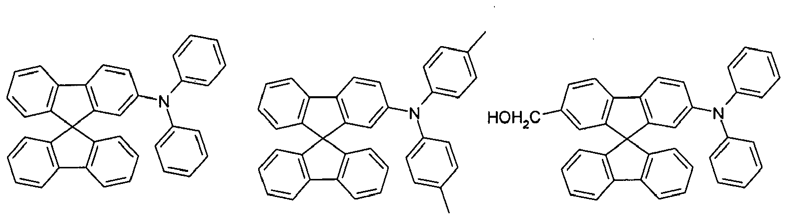

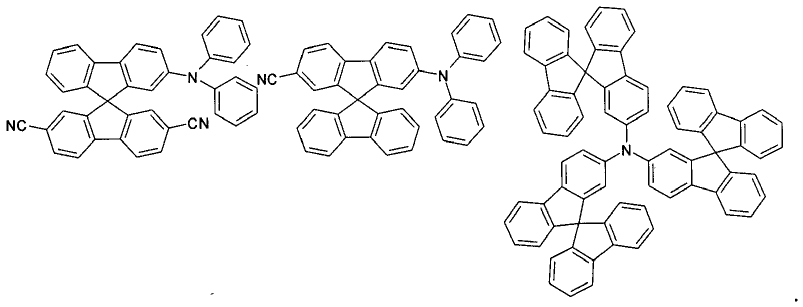

2. Composé selon la revendication 1 de la formule (2), de la formule (3a), (3b), (4a) ou de la formule (4b) : 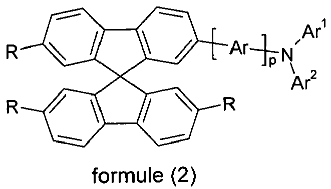 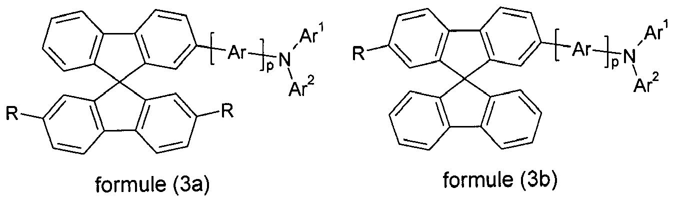 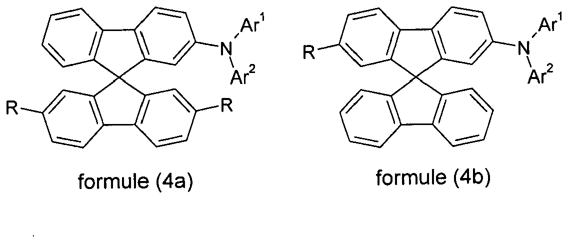 dans lesquelles les symboles et indices utilisés présentent les significations données selon la revendication 1.

3. Composé selon la revendication 1 ou 2, **caractérisé en ce que** les groupes Ar¹ et Ar² sont choisis, de manière identique ou différente pour chaque occurrence, parmi les groupes des formules (5) à (28) : 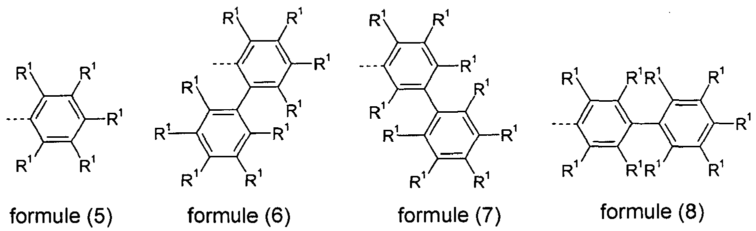 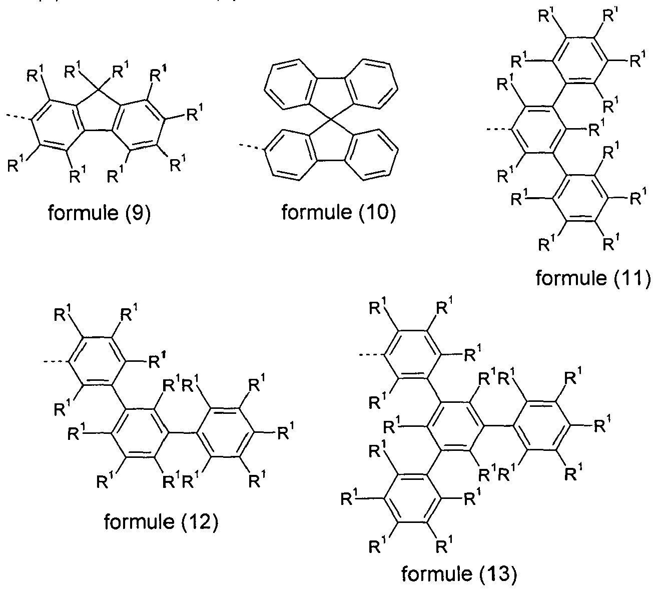 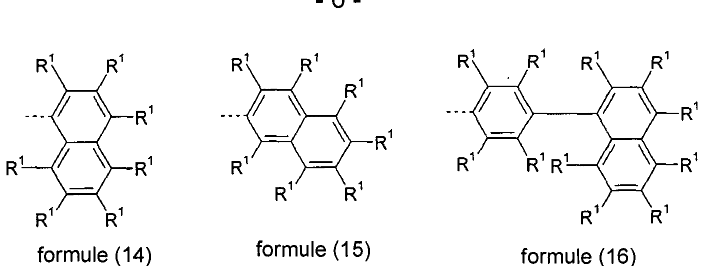 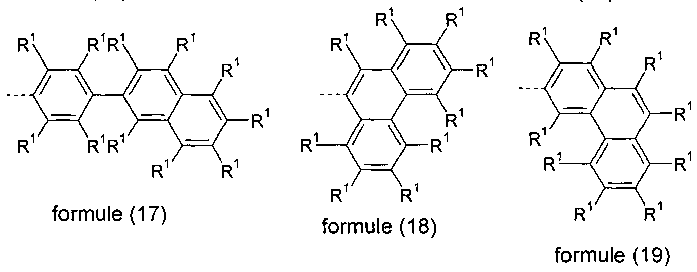 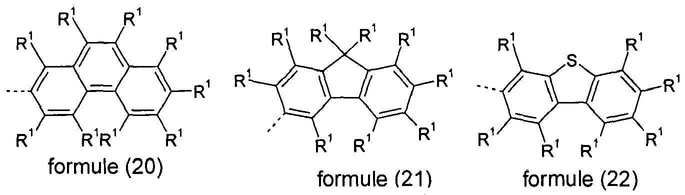 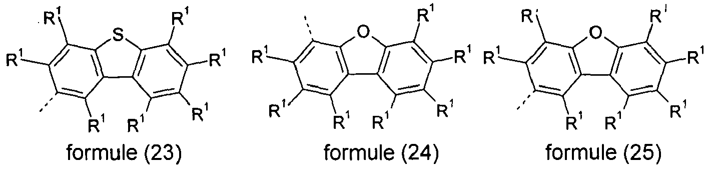 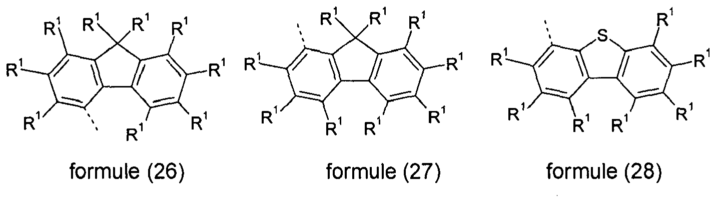 dans lesquelles les symboles utilisés présentent les significations données selon la revendication 1, et la liaison en pointillés représente la position de la liaison depuis le groupe jusqu'à l'azote.

4. Composé selon une ou plusieurs des revendications 1 à 3, **caractérisé en ce que** les groupes Ar¹ et Ar² sont choisis, de manière identique ou différente pour chaque occurrence, parmi les groupes des formules (5a) à (28a) : 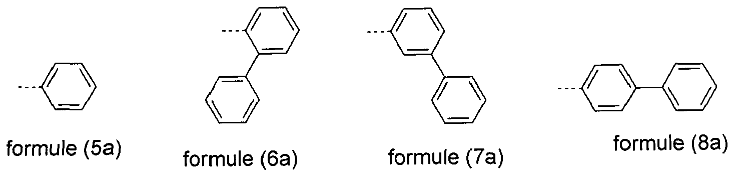 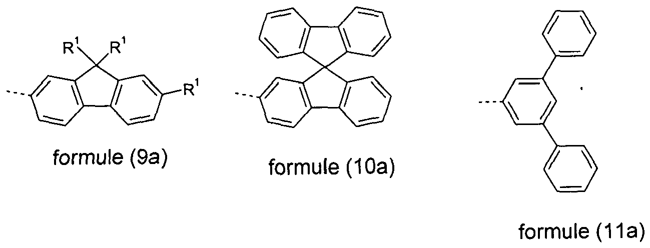 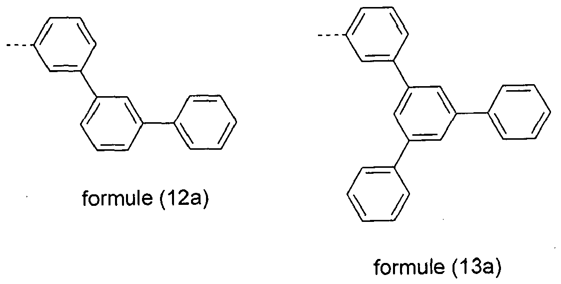 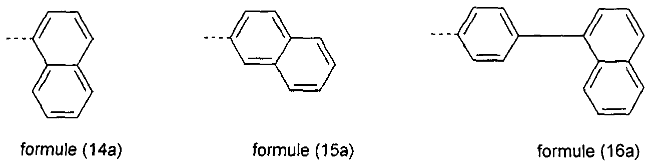 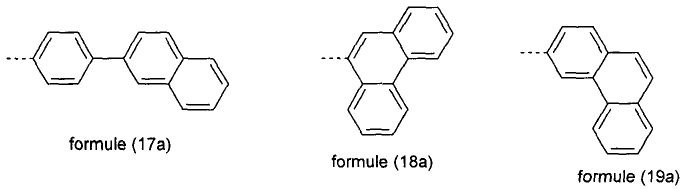 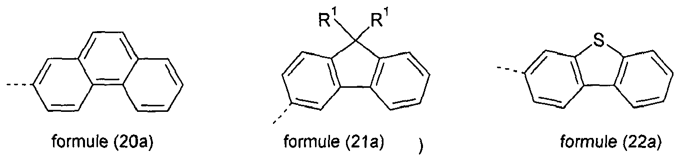 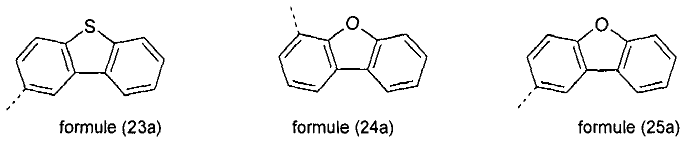 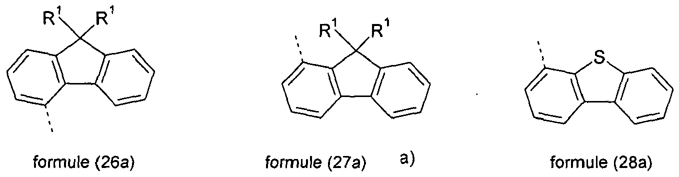 dans lesquelles les symboles utilisés présentent les significations données selon la revendication 1, et la liaison en pointillés représente la position de la liaison depuis le groupe jusqu'à l'azote.

5. Composé selon la revendication 3 ou la revendication 4, **caractérisé en ce que** Ar¹ est un groupe de la formule (6), (7), (8), (9) ou (21) et de façon préférable, un groupe de la formule (6a), (7a), (8a), (9a) ou (21a).

6. Composé selon une ou plusieurs des revendications 1 à 5, **caractérisé en ce que** les groupes Ar¹ et Ar² sont différents l'un de l'autre.

7. Composé selon une ou plusieurs des revendications 1 à 6, **caractérisé en ce que** le groupe -NAr¹Ar² présente la structure de l'une des formules (29), (30), (31) et (32) ou **en ce que** le groupe -Ar-NAr¹Ar² présente la structure de l'une des formules (33), (34), (35) et (36) : 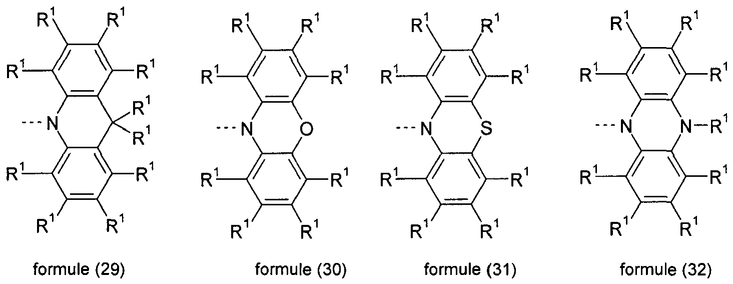 dans lesquelles les symboles utilisés présentent les significations données selon la revendication 1, et la liaison en pointillés représente la liaison sur le spirobifluorène ou sur Ar ; 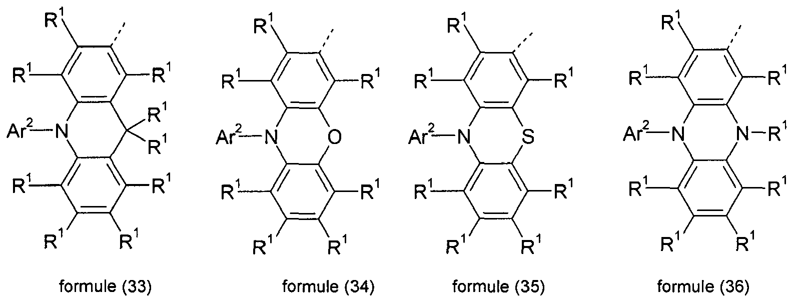 dans lesquelles les symboles utilisés présentent les significations données selon la revendication 1, et la liaison en pointillés représente la liaison sur le spirobifluorène.

8. Composé selon une ou plusieurs des revendications 1 à 7, **caractérisé en ce que** le groupe -(Ar)p- représente un groupe pris parmi l'une des formules (37) à (50) : 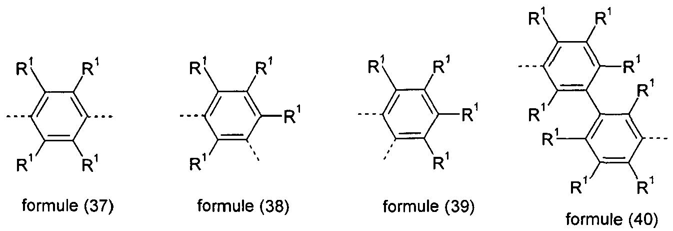 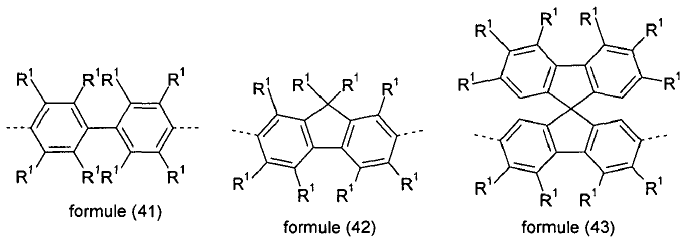 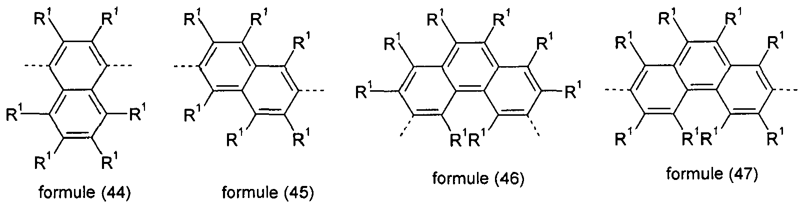 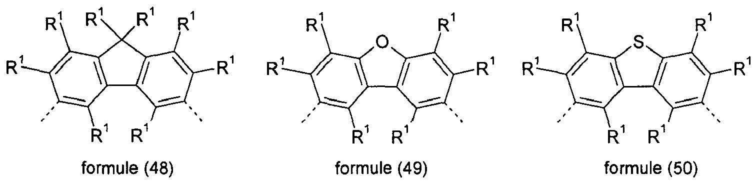 dans lesquelles les symboles utilisés présentent les significations données selon la revendication 1, et une première liaison en pointillés représente la liaison sur le spirobifluorène et l'autre liaison en pointillés représente la liaison sur l'atome d'azote.

9. Composé selon une ou plusieurs des revendications 1 à 8, pour lequel :
Ar est, de manière identique ou différente pour chaque occurrence, un système de cycle aromatique, où, pour p = 1 ou 2, -(Ar)p- est choisi parmi les groupes des formules (37) à (50) ; Ar ici peut également être connecté à Ar¹ et/ou à Ar² par un groupe E ;
Ar¹, Ar² sont, de manière identique ou différente pour chaque occurrence, un système de cycle aromatique choisi parmi les groupes des formules (5) à (25) ;
ou-NAr¹Ar² représente un groupe de l'une des formules (29) à (33) ;
ou -Ar-NAr¹Ar² représente un groupe de l'une des formules (34) à (37) ;
E est pour chaque occurrence, de manière identique ou différente, C(R¹)₂, N(R¹), O ou S ;
R est choisi pour chaque occurrence, de manière identique ou différente, parmi le groupe constitué par H, D, F, Si(R²)₃, un groupe alkyle ou alcoxy en chaîne droite comportant de 1 à 10 atome(s) de C ou un groupe aryle ou alcoxy ramifié ou cyclique comportant de 3 à 10 atomes de C, dont chacun peut être substitué par un ou plusieurs radical/radicaux R², où un ou plusieurs groupe(s) CH₂ non adjacents peut/peuvent être remplacé(s) par O et où un ou plusieurs atome(s) de H peut/peuvent être remplacé(s) par D ou F, un système de cycle aromatique comportant de 6 à 60 atomes de C choisi parmi le groupe constitué par benzène, naphtalène, phénanthrène, fluorène, spirobifluorène ou une combinaison de deux ou trois de ces groupes, lesquels peuvent dans chaque cas être identiques ou différents, lequel système de cycle peut dans chaque cas être substitué par un ou plusieurs radical/radicaux R², où deux substituants R adjacents ou plus peuvent en option former un système de cycle aliphatique monocyclique ou polycyclique, lequel peut être substitué par un ou plusieurs radical/ radicaux R² ;
R¹ est, si le radical R¹ est lié à Ar¹ ou Ar², choisi, de manière identique ou différente pour chaque occurrence, parmi le groupe constitué par H, D, un groupe alkyle en chaîne droite comportant de 1 à 10 atome(s) de C ou un groupe alkyle ramifié ou cyclique comportant de 3 à 10 atomes de C ;
ou R¹ qui est lié au pont de carbone dans la formule (29) ou (33) est choisi, de manière identique ou différente pour chaque occurrence, parmi le groupe constitué par un groupe alkyle en chaîne droite comportant de 1 à 10 atome(s) de C, un groupe alkyle ramifié ou cyclique comportant de 3 à 10 atomes de C ou un système de cycle aromatique comportant de 6 à 30 atomes de C, lequel peut être substitué par un ou plusieurs radical/radicaux R² ; les deux radicaux R¹ ici peuvent également former un système de cycle aliphatique ou aromatique l'un avec l'autre ;
ou R¹ qui est lié au pont d'azote dans la formule (32) ou (36) est choisi parmi le groupe constitué par un groupe alkyle en chaîne droite comportant de 1 à 10 atome(s) de C, un groupe alkyle ramifié ou cyclique comportant de 3 à 10 atomes de C ou un système de cycle aromatique comportant de 6 à 30 atomes de C, lequel peut être substitué par un ou plusieurs radical/radicaux R² ;
R² est choisi pour chaque occurrence, de manière identique ou différente, parmi le groupe constitué par H, D, un groupe alkyle en chaîne droite comportant de 1 à 5 atome(s) de C ou un groupe alkyle ramifié ou cyclique comportant de 3 à 5 atomes de C ou un système de cycle aromatique comportant de 6 à 18 atomes de C ;
R³ est choisi parmi le groupe constitué par H, D, F, un radical hydrocarbone aliphatique comportant de 1 à 10 atome(s) de C, un système de cycle aromatique comportant de 6 à 24 atomes de C, où un ou plusieurs atome(s) de H peut/peuvent être remplacé(s) par D ou F, où deux substituants R³ adjacents ou plus peuvent former un système de cycle aliphatique, aromatique ou hétéroaromatique monocyclique ou polycyclique l'un avec l'autre ou les uns avec les autres ;
m est 0, 1 ou 2 ;
n est pour chaque occurrence, de manière identique ou différente, 0, 1 ou 2 ;
p est 0, 1 ou 2.

10. Procédé pour la préparation d'un composé selon une ou plusieurs des revendications 1 à 9 en couplant un dérivé de spirobifluorène qui est substitué à la position 2 par un groupe partant réactif à
a) un amine primaire, ce couplage étant suivi par un couplage à un autre groupe aromatique qui est substitué par un groupe partant réactif, ou à
b) un amine secondaire, ou à
c) un dérivé de triarylamine.

11. Formulation, en particulier solution, dispersion ou mini-émulsion, comprenant au moins un composé selon une ou plusieurs des revendications 1 à 9 et au moins un solvant, en particulier un solvant organique, ou un mélange comprenant au moins un composé selon une ou plusieurs des revendications 1 à 9 et au moins un autre composé.

12. Utilisation d'un composé selon une ou plusieurs des revendications 1 à 9 ou d'une formulation selon la revendication 11 dans un dispositif électronique, en particulier dans un dispositif électroluminescent organique.

13. Dispositif électronique, en particulier choisi parmi le groupe constitué par des dispositifs électroluminescents organiques (diodes émettrices de lumière organiques, OLED), circuits intégrés organiques (O-IC), transistors à effet de champ organiques (O-FET), transistors à film mince organiques (O-TFT), transistors émetteurs de lumière organiques (O-LET), cellules solaires organiques (O-SC), cellules solaires sensibilisées par colorant organiques (ODSSC), détecteurs optiques organiques, photorécepteurs organiques, dispositifs à extinction de champ organiques (O-FQD), cellules électrochimiques émettrices de lumière (LEC), diodes laser organiques (O-laser) et dispositifs émetteurs de plasmon organiques, comprenant au moins un composé selon une ou plusieurs des revendications 1 à 9 ou un mélange selon la revendication 11.

14. Dispositif électroluminescent organique selon la revendication 13, **caractérisé en ce que** le composé selon une ou plusieurs des revendications 1 à 9 ou le mélange selon la revendication 11 est utilisé en tant que matériau de transport de trous dans une couche de transport de trous ou d'injection de trous ou de blocage d'excitons ou en tant que matériau de matrice pour des émetteurs fluorescents ou phosphorescents.
